(19) Europäisches Patentamt
European Patent Office
Office européen des brevets

(11) Publication number: **0 306 300 B1**

(12) # EUROPEAN PATENT SPECIFICATION

(45) Date of publication of patent specification: **03.05.95**

(51) Int. Cl.[6]: **C07D 487/04**, C07D 471/04, C07F 7/22, A61K 31/435, A61K 31/415, //(C07D487/04, 235:00,209:00),(C07D471/04, 235:00,221:00)

(21) Application number: **88308077.2**

(22) Date of filing: **01.09.88**

The file contains technical information submitted after the application was filed and not included in this specification

(54) **Pyrrolo(1,2-a)imidazole and imidazo(1,2-a)pyridine derivatives and their use as 5-lipoxygenase pathway inhibitors.**

(30) Priority: **02.09.87 US 92258**

(43) Date of publication of application:
**08.03.89 Bulletin 89/10**

(45) Publication of the grant of the patent:
**03.05.95 Bulletin 95/18**

(84) Designated Contracting States:
**AT BE CH DE ES FR GB GR IT LI LU NL SE**

(56) References cited:
**EP-A- 0 231 622**
**US-A- 4 175 127**
**US-A- 4 186 205**

(73) Proprietor: **SMITHKLINE BEECHAM CORPORATION**
**One Franklin Plaza**
**P.O. Box 7929**
**Philadelphia**
**Pennsylvania 19103 (US)**

(72) Inventor: **Adams, Jerry Leroy**
**611 Forest Road**
**Wayne**
**Pennsylvania 19087 (US)**
Inventor: **Nabil, Hanna**
**507 Kent Place**
**Berwyn**
**Pennsylvania 19312 (US)**
Inventor: **Razgaitis, Kazys**
**7 Arthur Road**
**Rosemont**
**Pennsylvania 19010 (US)**
Inventor: **Bender, Paul Elliot**
**504 Lilac Lane**
**Cherry Hill**
**New Jersey 08003 (US)**
Inventor: **Newton, John Frederick, Jr.**
**871 Frank Road**
**West Chester**
**Pennsylvania 19380 (US)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

Inventor: **Gleason, John Gerald**
**8 Heron Hill Drive**
**Downingtown**
**Pennsylvania 19335 (US)**
Inventor: **Perchonock, Carl David**
**5212 Augusta Street**
**Bethesda**
**Maryland 20816 (US)**

(74) Representative: **Giddings, Peter John, Dr. et al**
**SmithKline Beecham plc**
**Corporate Intellectual Property**
**SB House**
**Great West Road**
**Brentford, Middlesex TW8 9BD (GB)**

## Description

This invention relates to novel compounds, pharmaceutical compositions and methods of inhibiting the 5-lipoxygenase pathway of arachidonic acid metabolism in an animal in need thereof which comprises administering to such animal an effective, 5-lipoxygenase pathway inhibiting amount of a 2(3)-(pyridyl)-3(2)-(substituted phenyl)-6,7-dihydro-[5H]-pyrrolo-[1,2-a]imidazole a 2(3)-(pyridyl)-3(2)-(substituted phenyl)-5,6,7,8-tetrahydro-imidazo-[1,2-a]pyridine or a pharmaceutically acceptable salt thereof.

Davidson et al., U. S. Patent 4,507,481, issued March 26, 1985, disclose compounds of the formula:

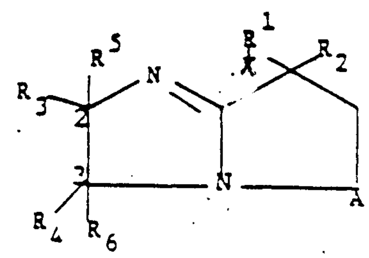

wherein:

X is O or S(O)n;

n is O, 1 or 2;

$R^1$ is H, lower alkyl, phenyl, benzyl or benzyl substituted with lower alkylamino, lower alkylamino, nitro, halo, hydroxy or lower alkoxy-;

$R_2$ is H or $XR^1$;

A is $CH_2$ or $CH_2CH_3$;

$R_3$ and $R_4$ are independently selected from A, lower alkyl, aryl, aryl substituted with lower alkyl, amino, lower alkylamino, nitro, lower alkoxy, hydroxy or halogen; provided that at least one of $R_3$ and $R_4$ is aryl or substituted aryl; and

$R_5$ and $R_6$ are each H or join to form a double bond at the 2,3-position.

Davidson et al. also disclose that such comounds are immunostimulants or immunosuppresants based on (a) their inhibiting or stimulating activity in a chemotaxis assay which measures the ability of a drug substance to influence the movement of murine macrophages responding to complement; (b) their immunosuppressing or activating activity in the Kennedy plaque assay in which an animal's humoral immune system is depressed artificially with 6-mercapto-purine. Neither the chemotaxis assay nor the Kennedy plaque assay is of any known utility for detecting or suggesting compounds which are inhibitors of the 5-lipoxygenase pathway. Davidson et al. also disclose that such compounds have antiinflammatory activity as determined by the carrageenan-induced paw edema assay in rats. As stated above, such assay has no known utility in detecting or suggesting compounds which are inhibitors of the 5-lipoxygenase pathway. Davidson et al. also disclose that such compounds have antiviral activity in mice with hepatitis; but such activity is of no known utility in detecting or suggesting compounds which are inhibitors of the 5-lipoxygenase pathway.

SUMMARY OF THE INVENTION

This invention relates to a compound of the formula (I)

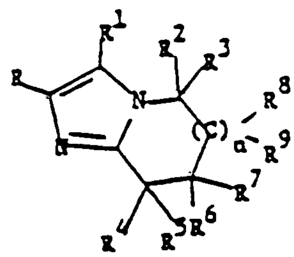

FORMULA (I)

wherein

1) One of R or $R^1$ must be alkyl substituted pyridyl and the other is selected from:

monosubstituted phenyl wherein said substituent is selected from H, halo, hydroxy $C_{1-3}$ alkoxy, $C_{1-3}$ alkylthio, $C_{1-4}$ alkyl,$C_{1-3}$ alkylsulfinyl, $C_{1-3}$ alkylsulfonyl, $C_{1-3}$ alkylamino, $C_{1-3}$ dialkylamino, $CF_3$, N-($C_{1-3}$ alkyl)-N-($C_{1-3}$alkanamido), N-pyrrolidino, N-piperidino, prop-2-ene-1-oxy or 2,2,2-trihaloethoxy;

2) $R^1$ is 4-pyridyl and R selected from:

monosubstituted phenyl wherein said substituent is selected from $C_{1-3}$ alkylthio, $C_{1-3}$ alkylsulfinyl, $C_{1-3}$ alkylsulfonyl, or branched or unbranched $C_{2-5}$ alkenylthio or $C_{2-5}$ alkenylsulfinyl; or

3) One of $R^1$ or R is pyridyl or alkylsubstituted pyridyl and the other is selected from monosubstituted or disubstituted phenyl wherein said substituent is selected from thiol [HS-], acylthio [AC(O)S-], dithioacyl [AC(S)S-], dialkylthiocarbamyl [$A_2$NC(O)S-], dialkyldithiocarbamyl [$A_2$NC(S)S-], alkylcarbonylalkylthio [AC(O)$CH_2$S-], carbalkoxyalkylthio [AOC(O)$CH_2$S-], or acyloxyalkylthio [AC(O)O$CH_2$S-] wherein the $CH_2$ is optionally substituted with $C_{1-4}$alkyl, and A is $C_{1-9}$alkyl;

and $R^2$, $R^3$, $R^4$, $R^5$, $R^6$, $R^7$, $R^8$ and $R^9$ are H, or one or two of $R^2$, $R^3$, $R^3$, $R^4$, $R^5$, $R^6$, $R^7$, $R^8$ and $R^9$ are independently selected from H or $C_{1-2}$ alkyl; and n is 0 or 1; or a pharmaceutically acceptable salt thereof.

The term N-($C_{1-3}$ alkyl)-N-($C_{1-3}$ alkanamido) is used herein at all occurrances to mean one of the following:

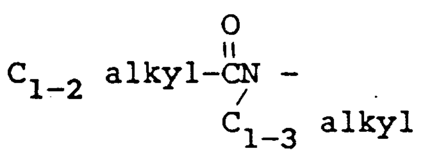

or

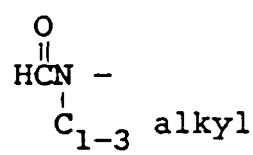

This invention also relates to a pharmaceutical composition comprising a pharmaceutically acceptable carrier or diluent and an effective, non-toxic 5-lipoxygenase pathway inhibiting amount of a compound of the formula (I) as defined above,

or a pharmaceutically acceptable salt thereof.

This invention also relates to intermediate compounds used in the preparation of a compound of Formula (I) having the following structural formula (J):

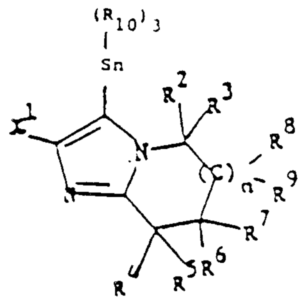

FORMULA (J)

wherein

n is 0 or 1;

$R^2$, $R^3$, $R^4$, $R^5$, $R^6$, $R^7$, $R^8$ and $R^9$ are H, or one or two of $R^2$, $R^3$, $R^4$, $R^5$, $R^6$, $R^7$, $R^8$ and $R^9$ are independently selected from H or $C_{1-2}$ alkyl;

$R_{10}$ is $C_{1-4}$ alkyl;

and $X^1$ is selected from

(a) monosubstituted phenyl wherein said substituent is selected from H, fluoro, chloro, $C_{1-3}$ alkoxy, $C_{1-4}$ alkyl, $C_{1-3}$ dialklamino, $CF_3$, $C_{1-3}$ alkylamino, N-pyrrolidino, N-piperidino, prop-2-ene-1-oxy or 2,2,2-trihaloethoxy; or

(b) pyridyl or alkyl substituted pyridyl; or a pharmaceutically acceptable salt thereof.

DETAILED DESCRIPTION OF THE INVENTION

This invention relates to compounds of Formula (I) as described above, pharmaceutical compositions comprising a pharmaceutically acceptable carrier or diluent and a compound of Formula (I), methods of treating 5-lipoxygenase pathway mediated diseases comprising administration of a compound of Formula (I) or a pharmaceutical composition containing a compound of Formula (I), and compounds of Formula (J) as described above.

All of the compounds of Formula (I) are useful in inhibiting the 5-lipoxygenase pathway of arachidonic acid metabolism in an animal in need thereof.

The compounds of Formula (I) can be prepared according to the following synthetic route:

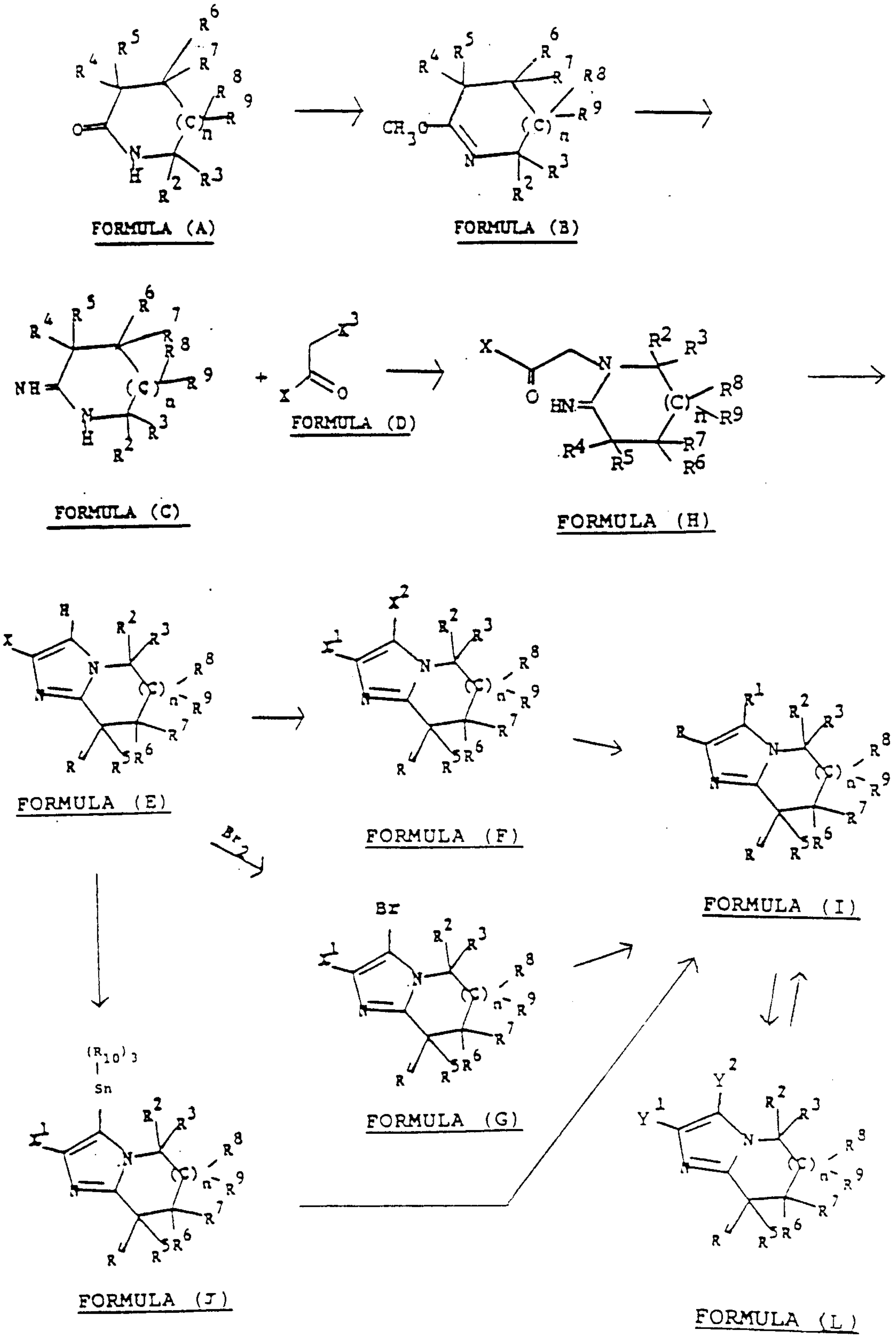


FORMULA (A)

FORMULA (B)

FORMULA (C)

FORMULA (D)

FORMULA (H)

FORMULA (E)

FORMULA (F)

FORMULA (I)

FORMULA (G)

FORMULA (J)

FORMULA (L)

All the compounds of Formula (E), Formula (F), Formula (G) and Formula (H) are useful as intermediates in the preparation of compounds of Formula (I). All of the necessary compounds of Formula (A), Formula (B), Formula (C) and Formula (D) can be obtained from commercial sources or are preparable by

conventional techniques such as those set out herein.

The compounds of Formula (E) have the following structure

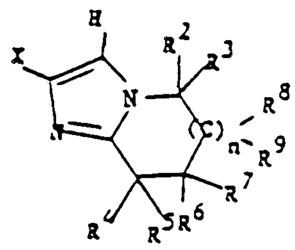

FORMULA (E)

wherein

n is 0 or 1;

$R^2$, $R^3$, $R^4$, $R^5$, $R^6$, $R^7$, $R^8$ and $R^9$ are H, or one or two of $R^2$, $R^3$, $R^4$, $R^5$, $R^6$, $R^7$, $R^8$ and $R^9$ are independently selected from H or $C_{1-2}$ alkyl;

X is selected from:

(a) pyridyl;

(b) monosubstituted phenyl, wherein said substituent is selected from halo, $C_{1-3}$ alkoxy, hydroxy, $C_{1-3}$ alkylthio, $C_{1-4}$ alkyl, $C_{1-3}$ alkylamino, $C_{1-3}$ dialkylamino, $CF_3$, N-($C_{1-3}$ alkanamido), N-($C_{1-3}$ alkyl)-N-($C_{1-3}$ alkanamido), N-pyrrolidino, N-piperidino, prop-2-ene-1-oxy or 2,2,2-trihaloethoxy;

or a pharmaceutically acceptable salt thereof.

A further intermediate compound used in the preparation of a compound of Formula (I) is a compound of the formula:

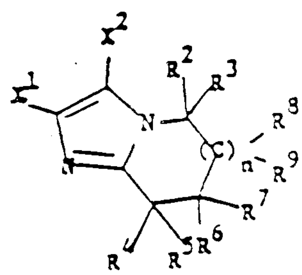

FORMULA (F)

wherein:

n is 0 or 1,

$R^2$, $R^3$, $R^4$, $R^5$, $R^6$, $R^7$, $R^8$, $R^9$ are all H, or one or two of $R^2$, $R^3$, $R^4$, $R^5$, $R^6$, $R^7$, $R^8$ and $R^9$, are independently selected from H or $C_{1-2}$ alkyl;

$X^2$ is 4-(1,4-dihydro)pyridyl substituted with N-($C_{1-8}$ alkanoyl), N-($C_{1-8}$ alkoxycarbonyl),N-(benzoyl), N-(phenoxycarbonyl), N-(phenylacetyl), or N-(benzyloxycarbonyl);

$X^1$ is selected from

(a) monosubstituted phenyl wherein said substituent is selected from H, halo, $C_{1-3}$ alkoxy, hydroxy, $C_{1-3}$ alkylthio, $C_{1-4}$ alkyl, N-($C_{1-3}$ alkyl)-N-($C_{1-3}$ alkanamido), $C_{1-3}$ dialkylamino, $CF_3$, N-pyrrolidino, N-piperidino, prop-2-ene-1-oxy or 2,2,2-trihaloethoxy;

or a pharmaceutically acceptable salt thereof.

A further intermediate compound used in the preparation of a compound of Formula (I) is a compound of the formula:

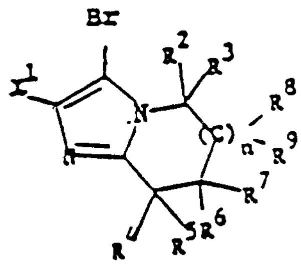

FORMULA (G)

wherein:

n is 0 or 1,

$R^2$, $R^3$, $R^4$, $R^5$, $R^6$, $R^7$, $R^8$ and $R^9$ are all H, or one or two of $R^2$, $R^3$, $R^4$, $R^5$, $R^6$, $R^7$, $R^8$, and $R^9$ are independently selected from H or $C_{1-2}$ alkyl; and

$X^1$ is selected from

(a) monosubstituted phenyl wherein said substituent is selected from H, fluoro, chloro, $C_{1-3}$ alkoxy, $C_{1-4}$ alkyl, $C_{1-3}$ dialkylamino, $CF_3$, $C_{1-3}$ alkylamino, N-pyrrolidino, N-piperidino, prop-2-ene-1-oxy or 2,2,2-trihaloethoxy;

or a pharmaceutically acceptable salt thereof.

A further intermediate compound used in the preparation of a compound of Formula (I) is a compound of the formula:

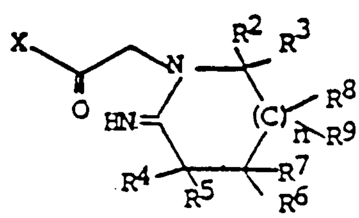

FORMULA (H)

wherein:

n is 0 or 1;

$R^2$, $R^3$, $R^4$, $R^5$, $R^6$, $R^7$, $R^8$, and $R^9$ are H, or one or two of $R^2$, $R^3$, $R^4$, $R^5$, $R^6$, $R^7$, $R^8$ and $R^9$ are independently selected from H or $C_{1-2}$ alkyl;

X is selected from:

(a) pyridyl;

(b) monosubstituted phenyl wherein said substituent is selected from H, halo, $C_{1-3}$ alkoxy, $C_{1-3}$ alkylthio, $C_{1-4}$ alkyl, N-($C_{1-3}$ alkanamido), $C_{1-3}$ dialkylamino, $CF_3$, N-pyrrolidino or N-piperidino;

or a pharmaceutically acceptable salt thereof.

A further intermediate compound used in the preparation of a compound of Formula (I) is a compound of the formula:

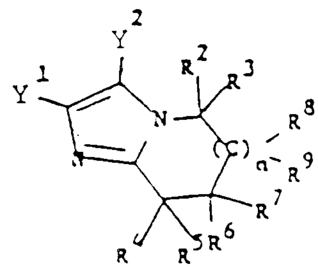

FORMULA (L)

wherein:

n is 0 or 1,

$R^2$, $R^3$, $R^4$, $R^5$, $R^6$, $R^7$, $R^8$, $R^9$ are all H, or one or two of $R^2$, $R^3$, $R^4$, $R^5$, $R^6$, $R^7$, $R^8$ and $R^9$, are independently selected from H or $C_{1-2}$ alkyl;

one of $Y^1$ or $Y^2$ is independently selected from 4-[1,2-dihydro-2-($C_{1-4}$-alkyl]pyridyl substituted with N-($C_{1-8}$ alkanoyl), N-($C_{1-8}$ alkoxycarbonyl), N-(benzoyl), N-(phenoxycarbonyl), N-(phenylacetyl), or N-(benzyloxycarbonyl);

and the other is selected from

(a) monosubstituted phenyl wherein said substituent is selected from H, halo, $C_{1-3}$ alkoxy, $C_{1-3}$ alkylthio, $C_{1-4}$ alkyl, N-($C_{1-3}$ alkyl)-N-($C_{1-3}$ alkanamido), $C_{1-3}$ dialkylamino, $CF_3$, N-pyrrolidino, N-piperidino, prop-2-ene-1-oxy or 2,2,2-trihaloethoxy;

or a pharmaceutically acceptable salt thereof.

Compounds of Formula (B), wherein n, $R^2$, $R^3$, $R^4$, $R^5$, $R^6$, $R^7$, $R^8$ and $R^9$ are as defined above, can be prepared by 0-alkylation of the corresponding 2-piperidone or 2-pyrrolidone of Formula (A), wherein n, $R^2$, $R^3$, $R^4$, $R^5$, $R^6$, $R^7$, $R^8$ and $R^9$ are as defined above, with an alkylating agent, such as dimethylsulfate, according to the method of Wick et al., Helv. Chim Acta, 54, 513 (1971). The necessary compounds of Formula (A) are commercially available or are prepared by known techniques. Compounds of Formula (C) wherein n, $R^2$, $R^3$, $R^4$, $R^5$, $R^6$, $R^7$, $R^8$, and $R^9$ are as defined above, can be prepared by treatment of the corresponding compound of Formula (B) with ammonia or an ammonium salt, such as ammonium chloride, in absolute ethanol according to the method of Etienne et al., Compt. Rend., 259, 2660 (1964). Compounds of Formula (C) wherein n is 1 or 2 and $R^2$, $R^3$, $R^4$, $R^5$, $R^6$, $R^7$, $R^8$ and $R^9$ are H are preferably prepared as described by the method of Moriconia and Cevasco, J. Org. Chem., 33, 2109 (1968) as their hydrohalide salts and liberated to the bases with concentrated aqueous NaOH or preferably with one molar equivalent of sodium methoxide in an alcoholic solvent. Compounds of Formula (D), wherein $X^3$ is Br and X is as defined above, are commercially available or are prepared by treatment of the correspondingly substituted acetophenone in $CH_2Cl_2$, $CHCl_3$, acetic acid or 48% hydrobromic acid with one equivalent of bromine [See, Langley, Org. Syn. Coll., 1, 127 (1944); Cowper et al., Org. Syn. Coll., 2, 480 (1943); and Lorenzin, et al., J. Org. Chem., 32, 4008 (1967)], or alternatively, by reaction in chloroform-ethyl acetate with a suspension of copper (II) bromide by the method of King and Ostrum, J. Org. Chem., 29, 3459 (1964).

The necessary acetophenones are commercially available or preparable by known techniques. Alternatively the Formula (D) compounds, wherein $X^3$ is chloro and X is (a) 4 monosubstituted phenyl where the substituent is selected from H, halo, $C_{1-4}$ alkyl, $C_{1-3}$ alkylthio, $C_{1-3}$ alkoxy, can be prepared by acylating the corresponding monosubstituted benzene by Friedel Crafts reaction with 2-chloroacetylchloride and $AlCl_3$, by the method of Joshi et al., J. Heterocyclic Chem., 16, 1141 (1979).

Compounds of Formula (E) serve as intermediates in the preparation of the compounds of Formula (I). Preferably, compounds of Formula (E) are prepared from their corresponding compound of Formula (H). Compounds of Formula (H) serve as intermediates in the preparation of compounds of Formula (E). Compounds of Formula (H) are prepared by treatment of a solution of a substituted Formula (D) compound, such as a 2-haloacetophenone, or a 2-bromoacetyl-2, 3 or 4-pyridine, which are described by Taurins et al., J. Heterocyclic Chem., 7, 1137 (1970), in a neutral, preferably nonpolar solvent with one molar equivalent of the corresponding Formula (C) compound, maintaining the temperature at or below 25 °C. The resulting Formula (H) hydrohalide salts are converted to Formula (E) compounds by refluxing in water. Alternatively, compounds of Formula (E) are prepared by treatment of a solution of the 2-iminopyrrolidine or 2-iminopiperidine with a substituted 2-bromoacetophenone of Formula (D), either in a polar organic solvent, such as DMF or ethanol, or in a nonpolar chlorinated hydrocarbon, followed by removing all or most of the solvent and refluxing the residue in aqueous solution. Compounds of Formula (E) wherein X is a pyridyl optionally substituted by a $C_{1-4}$ alkyl group are prepared by treatment of a mixture of a bromoacetylpyridine and 2-iminopyrrolidine or their hydrohalide salts in a polar aprotic solvent, such as dimethylformamide, with 2 to 5 equivalents of a base, such as a metal carbonate salt.

Compounds of Formula (I) where R is phenyl or substituted phenyl, and $R^1$ is 4-pyridyl are preferably prepared in two steps by a modification of the method of Lantos et al., European Patent Application No. 203,787 published March 12, 1986. In the first step, the corresponding compound of Formula (E) is treated, preferably at 20-25 °C, with pyridine and an acyl halide, an aroylhalide, an arylalkyl haloformate ester, or an alkyl haloformate ester, such as acetyl bromide, benzoylchloride, benzyl chloroformate, or preferably ethyl chloroformate, in a solvent in which the reactants are soluble and inert to form the compound of Formula (F). Alternatively the acyl pyridinium salt can be preformed and added to the solution of the Formula (E) compound. Compounds of Formula (F) serve as intermediates in the preparation of the compounds of Formula (I). In the second step, the Formula (F) compound, a 1,4-dihydropyridine product, is deacylated and aromatized with sulfur in refluxing decalin, tetralin, p-cymene or xylene, or preferably with potassium tert.-butoxide in tert.-butanol with $O_2$ gas at reflux for 15 minutes to the afford the corresponding compound of Formula (I).

Compounds of Formula (I) wherein R or $R^1$ is alkyl substituted pyridyl can be prepared by a similar process from compounds of Formula (L). The Formula (L) compound is deacylated and aromatized with sulfur in decalin, tetralin, p-cymene or xylene or with potassium tert.-butoxide in tert.-butanol with oxygen gas at reflux for 15 minutes to afford the corresponding Formula (I) compound. Compounds of Formula (L) are prepared by treatment of the Formula (I) compound with an acyl halide, aroylhalide, arylalkyl haloformate ester or an alkyl haloformate ester and a $C_{1-4}$ alkyl Grignard reagent using the process of Comins, D.L., and Abdullah, A.H., J. Org. Chem., Vol. 47, p. 4315 (1982).

The same Formula (E) compounds used to prepare the 4-pyridyl Formula (I) compounds are employed to prepare the 2-pyridyl and 3-pyridyl Formula (I) compounds. Treatment of the Formula (E) compounds with bromine by the procedure of Kano, Yakugaku Zasshi, 92, 51 (1972), results in 3-bromination to afford the 3-bromo-2-(substituted phenyl)-6,7-dihydro-(5H)-pyrrolo(1,2-a)imidazoles and 3-bromo-2-(substituted phenyl)-5,6,7,8-tetrahydro-imidazo(1,2-a)pyridines compounds of Formula (G). The compounds of Formula (G) serve as intermediates in the preparation of compounds of Formula (I). Formula (E) or Formula (G) compounds are treated with n-butyl lithium (n-BuLi) in tetrahydrofuran to afford their 3-lithio derivatives by metallation halogen-metal interchange. Transmetallation of the 3-lithio compounds with $MgBr_2$ or $ZnCl_2$ to the corresponding magnesium or zinc compounds, according to the method of Negishi et al., J. Org. Chem., 42, 1821, (1977), provides good coupling to 2- or 3-bromopyridine in the presence of $PdCl_2$(1,4-bis-(diphenylphosphino)butane) catalyst, a bidentate Pd (II) catalyst, using the method of Kumada et al., Tetrahedron Letters, 22, 5319 (1981). Alternatively the Formula (G) compounds may be coupled to the 2 or 3-metalated pyridine employing this bidentate Pd (II) catalyst, or the corresponding Ni(II) $Cl_2$ (1,2-bis-(diphenylphosphino) ethane catalyst [see, Pridgen, J. Org. Chem., 47, 4319 (1982)]. By either of these routes, Formula (I) compounds are obtained where $R^1$ is 2-pyridyl or 3-pyridyl.

The compounds of Formula (I) can also be prepared from Formula (E) by preparation of the trialkyltin derivative of Formula (E), designated as Formula (J). The compound of Formula (J) is prepared by treatment of the 3-lithio derivative of Formula (E) with trialkyltin chloride. The Formula (J) compound is reacted with a mixture of an aryl or heteroaryl halide, preferably iodide, or triflate, and tetrakis-(triphenylphosphine)palladium in a mixture of THF (tetrahydrofuran) and HMPA (hexamethylphosphoramide) to yield a compound of Formula (I). The compounds of Formula (I) wherein either or both of R and $R^1$ are 2-pyridyl, 3-pyridyl or wherein R is 2-pyridyl, 3-pyridyl, or 4-pyridyl are preferably made by this route. Alternatively, compounds of Formula (I) may be prepared by the analogous reaction of an aryl or heteroaryl trialkyltin compound with a mixture of a Formula (G) compound and tetrakis(triphenylphosphine)palladium under similar conditions.

Regioisomers of Formula (I) compounds where $R^1$ is substituted phenyl, or 2,3 or 4-pyridyl and R is 2, 3, and 4-pyridyl are obtained from compounds of Formula (E) where X is 2,3, or 4-pyridyl. Compounds of Formula (E) where X is 2, 3 or 4-pyridyl are prepared by treatment of a 2, 3, or 4-bromoacetylpyridine hydrobromide salt of Formula (D), wherein R is 2, 3 or 4-pyridyl [prepared as described by Taurins et al., J. Het Chem., 7, 1137 (1970)] with 2-3 equivalents of the 2-iminopyrrolidine or 2-iminopiperidine by the procedure used to prepare the other compounds of Formula (E) described above. 3-Bromination, by the procedure of Kano cited above, affords the corresponding Formula (G) compounds. Metallation of the Formula (E) compounds with n-BuLi or halogen-metal interchange of the Formula (G) compounds with n-BuLi, followed by transmetallation with $MgBr_2$ and coupling to the substituted halobenzene, preferably iodobenzene, or 2,3, or 4-halopyridine, preferably where halo is iodo, in the presence of the bidentate phosphine-palladium or nickel complex as described above affords the desired regioisomers of Formula (I) and the bis(pyridyl) compounds of Formula (I). Alternatively the metallated pyridine or substituted benzene may be coupled to the Formula (G) compounds employing the catalysts as described above.

Alternately the compounds of Formula (I) and (E) wherein R or $R^1$ is a mono substituted phenyl having at least one fluoro substituent can be converted to the corresponding Formula (I) or (E) compounds having an alkylthio substituted phenyl group. The fluoro substituted phenyl compound of Formula (I) or (E) is treated with 1.2 equivalents of the sodium salt of the alkylmercaptan in an aprotic polar solvent, preferably dimethylformamide.

Compounds of Formula (I) where R or $R^1$ is a mono-substituted phenyl having at least one $C_{1-3}$ alkylsulfinyl, $C_{1-3}$alkylsulfonyl, acyloxyalkylsulfinyl, or $C_{1-3}$alkenylsulfinyl substituent are prepared by treatment of one or more equivalents of the corresponding compound of Formula (I) where R or $R^1$ are $C_{1-3}$ alkylthiophenyl, $C_{1-3}$alkylsulfinylphenyl, acyloxyalkylthiophenyl or alkenylthiophenyl with one or more equivalents of an oxidizing agent (such as 3-chloroperbenzoic acid in an inert solvent or sodium periodate in a polar solvent such as aqueous methanol containing a mineral acid such as hydrochloric acid) per mercapto function, in an inert solvent. Compounds of Formula (I) wherein R or $R^1$ are $C_{1-3}$ alkylsulfonyl substituted phenyl are prepared by treatment of one equivalent of the corresponding $C_{1-3}$ sulfinyl Formula (I) compound with 2/3 equivalent of $KMnO_4$ per sulfinyl function in aqueous acid solution by the method of Chatterway et al., J. Chem. Soc. 1352 (1930), or alternatively with one equivalent of a peracid.

Acetophenones substituted with a mono-substituted phenyl having at least one N-($C_{1-3}$alkanamido) or N-($C_{1-3}$ alkyl)-N-($C_{1-3}$ alkanamido), and in some cases the Formula (E), and Formula (I) compounds, are prepared by acylation of the corresponding amino and N-($C_{1-3}$ alkylamino) compounds with the alkanoic acid anhydride or chloride in pyridine. Another alternative preparation of the N-($C_{1-3}$ alkyl)-N-($C_{1-3}$ alkanamido) phenyl substituted Formula (E) and Formula (I) compounds is the alkylation of the corresponding N-($C_{1-3}$ alkanamido) substituted compounds with sodium hydride and a $C_{1-3}$ alkyl bromide or iodide in dimethyl formamide.

Formula (E) and Formula (I) compounds containing a mono-substituted phenyl having at least one amino substituent are prepared either by hydrolysis of the corresponding N-($C_{1-3}$ alkanamido) compounds in refluxing 6 N mineral acid or by catalytic reduction of the corresponding nitro compounds.

Formula (E), Formula (G), and Formula (I) compounds containing a mono-substituted phenyl having at least one N-($C_{1-3}$ alkylamino) substituent are preferably prepared by acid catalyzed hydrolysis of the corresponding N-($C_{1-3}$ alkyl)-N-($C_{1-3}$ alkanamido) compounds of Formula (E), Formula (G) and Formula (I), respectively, prepared as described above for the aminophenyl substituted compounds, or alternatively, either by (a) reduction of the corresponding N-($C_{1-3}$ alkanamido) compounds wish borane or borane dimethylsulfide complex in THF by the method of Brown, "Organic Synthesis via Boranes", John Wiley and Sons, (1975), or (b) by cleavage of the corresponding N,N-(di $C_{1-3}$ alkylamino)phenyl substituted Formula (E) and Formula (I) compounds with cyanogen bromide in the Von Braun reaction [see, Hageman Org. Reactions, Vol. 7, 198 (1953)].

Formula (E) and Formula (I) compounds containing a mono-substituted phenyl having at least one N,N-(di $C_{1-3}$ alkylamino) substituent are alternatively prepared either by reduction of the corresponding N-($C_{1-3}$ alkyl)-N-($C_{1-3}$ alkanamido) compounds of Formula (E) and Formula (I) with borane as described above for the N-($C_{1-3}$ alkylamino) substituted compounds, or by displacement of the bromide by a N,N-dialkylamine in the corresponding 4-bromo-3-nitrophenyl Formula (E) and Formula (I) compounds by heating at 140 °C with the N,N-dialkylamine and potassium carbonate in an inert solvent.

Formula (E) and Formula (I) compounds containing a mono-substituted phenyl having at least one N-pyrrolidino and N-piperidino substituent are alternatively prepared by cyclodialkylation of the corresponding aminophenyl compounds with dibromobutane or dibromopentane and anhydrous potassium carbonate in an inert solvent such as dimethylformamide.

Compounds of Formula (E) where X is mono-substituted phenyl having at least one 2,2,2-trihaloethoxy or prop-2-ene-1-oxy substituent are prepared by alkylation of the appropriate phenols of Formula (E) with trifluoromethylsulfonic acid 2,2,2-trifluoroethyl ester or allyl bromide respectively as described by Bender et al, J. Med. Chem., 28, 1169 (1985), for preparation of compounds No. 23 and 33 described therein. Appropriately substituted mono hydroxy phenyl compounds or disubstitued phenyl compounds wherein one substituent is hydroxy of Formula (E) and Formula (I) are obtained by treatment of their respective correspondingly substituted methoxy derivatives with HBr in acetic acid, or preferably with $BBr_3$ in $CH_2Cl_2$ by the method described by Bender et al., J. Med. Chem., 28, 1169 (1985), for the preparation of compound No. 14 described therein.

Compounds of Formula (I) where R is $C_{1-3}$alkoxy mono-substituted phenyl are prepared by alkylation of the appropriately substituted hydroxyphenyl compounds with the corresponding $C_{1-3}$ alkylhalide in the presence of a strong base such as sodium hydride in an aprotic organic solvent such as dimethylformamide.

Compounds of Formula (I) wherein R or $R^1$ is phenyl substituted with an acyloxyalkylthio group wherein the alkyl is optionally substituted with $C_{1-4}$alkyl are prepared by treating a compound of Formula (I) wherein $R^1$ is phenyl substituted with at least one alkylsulfinyl group with an alkanoic acid anhydride. Hydrolysis of the resulting acyloxyalkylthio compounds yields compounds of Formula (I) wherein one of $R^1$ or R is phenyl substituted with a sulfhydryl function. The sulfhydryl substituted compounds can be treated with an alkanoic acid anhydride or a dithioalkanoic acid anhydride in pyridine to prepare compounds of Formula (I) wherein one of $R^1$ or R is phenyl substituted with one or more acylthio or dithioacyl groups.

Compounds of Formula (I) wherein $R^1$ or R is phenyl substituted with an alkenylthio group wherein one carbon atom separates the sulfur from the carbon bearing the double bond can be prepared by alkylating a compound of Formula (I) wherein one of $R^1$ or R is phenyl substituted with at least one sulfhydryl group with an appropriately substituted alkenylhalide such as allylbromide.

Compounds of Formula (I) wherein $R^1$ or R is phenyl substituted with an alkylcarbonylalkylthio or carbalkoxyalkylthio group are prepared by treatment of the corresponding sulfhydryl substituted compounds with an alkylcarbonylalkylhalide, such as bromoacetone, or with a carbalkoxyalkylhalide, such as ethylbromoacetate.

Compounds of Formula (I) wherein R or $R^1$ is phenyl substituted with an alkenylthio group wherein the sulfur is attached to the carbon bearing the double bond are prepared from the corresponding compounds wherein the phenyl is substituted with a mercapto group. The mercapto substituted compound is converted to a metal salt in a polar solvent with a strong base such as a metal hydride, a metal alkoxide or lithiumdiethylamide. The metal mercaptide salt is treated with trialkylsilylmethylchloride to afford an intermediate compound of Formula (I) wherein R or $R^1$ is phenyl substituted with at least one trialkylsilylmethylsulfide group. This intermediate in an aprotic solvent such as tetrahydrofuran is treated at reduced temperature with a lithiating reagent such as lithium diethylamide followed by treatment with an appropriate aliphatic aldehyde or ketone to prepare the compounds of Formula (I) wherein R or $R^1$ is phenyl substituted with one or more alkenylthio groups.

Pharmaceutically acceptable salts and their preparation are well known to those skilled in pharmaceuticals. Pharmaceutically acceptable salts of the compounds of Formula (I) which are useful in the present invention include, but are not limited to, maleate, fumarate, lactate, oxalate, methanesulfonate, ethane-sulfonate, benzenesulfonate, tartrate, citrate, hydrochloride, hydrobromide, sulfate and phosphate salts. Preferred pharmaceutically acceptable salts of the compounds of Formula (I) include hydrochloride and hydrobromide salts, as such salts can be prepared by known techniques such as the method of Bender et al., U.S. Patent 4,175,127, the disclosure of which is hereby incorporated by reference.

It has now been discovered that the compounds of Formula (I) are useful for treating disease states mediated by the 5-lipoxygenase pathway of arachidonic acid metabolism in an animal, including mammals, in need thereof. The discovery that the compounds of Formula (I) are inhibitors of the 5-lipoxygenase pathway is based on the effects of the compounds of Formula (I) on tissue inflammation in vivo and on the production of 5-lipoxygenase products by inflammatory cells in vitro in assays,some of which are described hereinafter. In summary, such assays reveal that the compounds of Formula (I) display anti-inflammatory activity in arachidonic acid-induced inflammation in the mouse ear model. The cyclooxygenase inhibitor, indomethacin, did not reduce inflammation in these assays. The 5-lipoxygenase pathway inhibitory action of the compounds of Formula (I) was confirmed by showing that they impaired the production of 5-lipoxygenase products such as leukotriene $B_4$ (di-HETE) and 5-HETE production by RBL-1 cells.

The pathophysiological role of arachidonic acid metabolites has been the focus of recent intensive studies. In addition to the well-described phlogistic activity (i.e. general inflammatory activity) of prostaglandins, the more recent description of similar activity for eicosanoids has broadened the interest in these products as mediators of inflammation [See, O'Flaherty, Lab. Invest., 47 314-329 (1982)]. The reported discovery of potent chemotactic and algesic activity for $LTB_4$ [see, Smith, Gen. Pharmacol., 12, 211-216 (1981) and Levine et al., Science, 225, 743-745 (1984)], together with known $LTC_4$ and $LTD_4$-mediated increase in capillary permeability [see, Simmons et al., Biochem. Pharmacol., 32, 1353-1359 (1983), Veno et al., Prostaglandins, 21, 637-647 (1981), and Camp et al., Br. J. Pharmacol., 80, 497-502 (1983)], has led to their consideration as targets for pharmacological intervention in both the fluid and cellular phases of inflammatory diseases.

The pharmacology of several inflammatory model systems has attested to the effectiveness of corticosteroids in reducing the cellular infiltration. These results, and the observation that corticosteroids

inhibit the generation of both cyclooxygenase and lipoxygenase products, suggest that such dual inhibitors may effectively reduce both the fluid and cellular phases of the inflammatory response since selective cyclooxygenase inhibitors do not reliably inhibit cell influx into inflammatory sites [See, Vinegar et al., Fed. Proc., 35, 2447-2456 (1976), Higgs et al., Brit. Bull., 39, 265-270 (1983), and Higgs et al., Prostaglandins, Leukotrienes and Medicine, 13, 89-92 (1984)]. The observations outlined above cogently argue that a dual inhibitor of arachidonic acid metabolism would be a more effective antiinflammatory agent than an inhibitor of cyclooxygenase only. Under optimal conditions, it is likely that an agent with preferential lipoxygenase inhibitory activity would not share the ulcerogenic liability of cyclooxygenase inhibitors or the toxicity of corticosteroids.

Recent clinical data also support the enthusiasm for inhibitors of the 5-lipoxygenase pathway in a variety of inflammatory diseases in which granulocyte and/or monocyte infiltration is prominent. The reported demonstration of elevated levels of $LTB_4$ in rheumatoid arthritic joint fluid [See, Davidson et al., Ann. Rheum. Dis., 42, 677-679 (1983)] also suggests a contributing role for arachidonic acid metabolites in rheumatoid arthritis. The recently reported preliminary observation of efficacy, including remission, reported with sulfasalazine treatment of rheumatoid arthritic patients [See Neumann et al., Brit. Med. J., 287, 1099-1102 (1983)] illustrates the utility of inhibitors of the 5-lipoxygenase pathway in rheumatoid arthritis.

Sulfasalazine, which is used for treatment of ulcerative colitis, has been reported to inhibit $LTB_4$ and 5-HETE production in vitro [See, Stenson et al., J. Clin. Invest., 69, 494-497 (1982)]. This observation, coupled with the fact that it has been reported that inflamed gastrointestinal mucosa from inflammatory bowel disease patients showed increased production of $LTB_4$ [See, Sharon et al., Gastroenterol., 84, 1306 (1983)], suggests that sulfasalazine can be effective by virtue of inhibition of production of chemotactic eicosanoids (such as the 5-lipoxygenase pathway product known as $LTB_4$). The observations serve to underscore utility of inhibitors of the 5-lipoxygenase pathway in inflammatory bowel disease.

Another area of utility for an inhibitor of the 5-lipoxygenase pathway is in the treatment of psoriasis. It was demonstrated that involved psoriatic skin had elevated levels of $LTB_4$ [See, Brain et al., Lancet, 19, February 19, 1983]. The promising effect of benoxaprofen on psoriasis [See, Allen et al., Brit. J. Dermatol., 109, 126-129 (1983)], a compound with in vitro lipoxygenase inhibitory activity on psoriasis, lends support to the concept that inhibitors of the 5-lipoxygenase pathway can be useful in the treatment of psoriasis.

Lipoxygenase products have been identified in exudate fluids from gouty patients. This disorder is characterized by massive neutrophil infiltration during the acute inflammatory phases of the disease. Since a major 5-lipoxygenase product, $LTB_4$, is produced by neutrophils, it follows that inhibition of the synthesis of $LTB_4$ can block an amplification mechanism, in gout.

Another area in which inhibitors of the 5-lipoxygenase product can have utility is in myocardial infarction. Studies in dogs with the dual inhibitor, BW755-C, demonstrated that the area of infarction following coronary occlusion was reduced, and such reduction was attributed to inhibition of leukocyte infiltration into the ischaemic tissue [See, Mollane et al., J. Pharmacol. Exp. Therap., 228, 510-522 (1984)].

Yet another area of utility for inhibitors of the 5-lipoxygenase pathway is in the area of prevention of rejection of organ transplants. [See, e.g., Foegh et al., Adv. Prostaglandin, Thromboxane, and Leukotriene Research, 13, 209-217 (1983).]

Yet another utility for inhibitors of the 5-lipoxygenase pathway is in the treatment of tissue trauma. [See, e.g., Denzlinger et al. Science, 230 (4723), 330-332 (1985)].

Furthermore, another area of utility for inhibitors of the 5-lipoxygenase pathway is in the treatment of inflammatory reaction in the central nervous system, including multiple sclerosis. [See, e.g., Mackay et al., Clin. Exp. Immunology, 15, 471-482 (1973)].

Additionally, another area of utility for inhibitors of the 5-lipoxygenase pathway is in the treatment of asthma. [See, e.g., Ford-Hutchinson, J. Allergy Clin. Immunol., 74, 437-440 (1984)].

The pharmaceutically effective compounds of this invention are administered in conventional dosage forms prepared by combining a compound of Formula (I) ("active ingredient") in an amount sufficient to produce 5-lipoxygenase pathway inhibiting activity with standard pharmaceutical carriers according to conventional procedures. These procedures may involve mixing, granulating and compressing or dissolving the ingredients as appropriate to the desired preparation.

The pharmaceutical carrier employed may be, for example, either a solid or liquid. Exemplary of solid carriers are lactose, terra alba, sucrose, talc, gelatin, agar, pectin, acacia, magnesium stearate, stearic acid and the like. Exemplary of liquid carriers are syrup, peanut oil, olive oil, water and the like. Similarly, the carrier or diluent may include time delay material well known to the art, such as glyceryl monostearate or glyceryl distearate alone or with a wax.

A wide variety of pharmaceutical forms can be employed. Thus, if a solid carrier is used, the preparation can be tableted, placed in a hard gelatin capsule in powder or pellet form or in the form of a

troche or lozenge. The amount of solid carrier will vary widely but preferably will be from about 25 mg. to about 1 g. a liquid carrier is used, the preparation will be in the form of a syrup, emulsion, soft gelatin capsule, sterile injectable liquid such as an ampule or nonaqueous liquid suspension.

To obtain a stable water soluble dose form, a pharmaceutically acceptable salt of a compound of Formula (I) is dissolved in an aqueous solution of an organic or inorganic acid, such as a 0.3 M solution of succinic acid or, preferably, citric acid.

Preferably, each parenteral dosage unit will contain the active ingredient [i.e., the compound of Formula (I)] in an amount of from about 50 mg. to about 500 mg. Preferably, each oral dosage will contain the active ingredient in an amount of from about 100 mg to about 1000 mg.

The compounds of Formula (I) may also be administered topically to a mammal in need of the inhibition of the 5-lipoxygenase pathway of arachidonic acid metabolism. Thus, the compounds of Formula (I) may be administered topically in the treatment or prophylaxis of inflammation in an animal, including man and other mammals, and may be used in the relief or prophylaxis of 5-lipoxygenase pathway mediated diseases such as rheumatoid arthritis, rheumatoid spondylitis, osteoarthritis, gouty arthritis and other arthritic conditions, inflamed joints, eczema, psoriasis or other inflammatory skin conditions such as sunburn; inflammatory eye conditions including conjunctivitis; pyresis, pain and other conditions associated with inflammation.

The amount of a compound of Formula (I) (hereinafter referred to as the active ingredient) required for therapeutic effect on topical administration will, of course, vary with the compound chosen, the nature and severity of the inflammatory condition and the animal undergoing treatment, and is ultimately at the discretion of the physician. A suitable anti-inflammatory dose of an active ingredient is 1.5 $\mu$g to 500 mg of base for topical administration, the most preferred dosage being 1 $\mu$g to 1000 $\mu$g, for example 5 to 25 $\mu$g administered two or three times daily.

By topical administration is meant non-systemic administration and includes the application of a compound of Formula (I) externally to the epidermis, to the buccal cavity and instillation of such a compound into the ear, eye and nose, and where the compound does not significantly enter the blood stream. By systemic administration is meant oral, intravenous, intraperitoneal and intramuscular administration.

While it is possible for an active ingredient to be administered alone as the raw chemical, it is preferable to present it as a pharmaceutical formulation. The active ingredient may comprise, for topical administration, from 0.001% to 10% w/w, e.g. from 1% to 2% by weight of the formulation although it may comprise as much as 10% w/w but preferably not in excess of 5% w/w and more preferably from 0.1% to 1% w/w of the formulation.

The topical formulations of the present invention, both for veterinary and for human medical use, comprise an active ingredient together with one or more acceptable carrier(s) therefor and optionally any other therapeutic ingredient(s). The carrier(s) must be 'acceptable' in the sense of being compatible with the other ingredients of the formulation and not deleterious to the recipient thereof.

Formulations suitable for topical administration include liquid or semi-liquid preparations suitable for penetration through the skin to the site of inflammation such as: liniments, lotions, creams, ointments or pastes, and drops suitable for administration to the eye, ear or nose.

Drops according to the present invention may comprise sterile aqueous or oily solutions or suspensions and may be prepared by dissolving the active ingredient in a suitable aqueous solution of a bactericidal and/or fungicidal agent and/or any other suitable preservative, and preferably including a surface active agent. The resulting solution may then be clarified by filtration, transferred to a suitable container which is then sealed and sterilized by autoclaving or maintaining at 98-100 °C. for half an hour. Alternatively, the solution may be sterilized by filtration and transferred to the container by an aseptic technique. Examples of bactericidal and fungicidal agents suitable for inclusion in the drops are phenylmercuric nitrate or acetate (0.002%), benzalkonium chloride (0.01%) and chlorhexidine acetate (0.01%). Suitable solvents for the preparation of an oily solution include glycerol, diluted alcohol and propylene glycol.

Lotions according to the present invention include those suitable for application to the skin or eye. An eye lotion may comprise a sterile aqueous solution optionally containing a bactericide and may be prepared by methods similar to those for the preparation of drops. Lotions or liniments for application to the skin may also include an agent to hasten drying and to cool the skin, such as an alcohol or acetone, and/or a moisturizer such as glycerol or an oil such as castor oil or arachis oil.

Creams, ointments or pastes according to the present invention are semi-solid formulations of the active ingredient for external application. They may be made by mixing the active ingredient in finely-divided or powdered form, alone or in solution or suspension in an aqueous or non-aqueous fluid, with the aid of suitable machinery, with a greasy or non-greasy basis. The basis may comprise hydrocarbons such as hard, soft or liquid paraffin, glycerol, beeswax, a metallic soap; a mucilage; an oil of natural origin such as

almond, corn, arachis, castor or olive oil; wool fat or its derivatives, or a fatty acid such as steric or oleic acid together with an alcohol such as prolylene glycol or macrogols. The formulation may incorporate any suitable surface active agent such as an anionic, cationic or non-ionic sulfactant such as sorbitan esters or polyoxyethylene derivatives thereof. Suspending agents such as natural gums, cellulose derivatives or inorganic materials such as silicaceous silicas, and other ingredients such as lanolin, may also be included.

The compounds of Formula (I) may also be administered by inhalation. By "inhalation" is meant intranasal and oral inhalation administration. Appropriate dosage forms for such administration, such as an aerosol formulation or a metered dose inhaler, may be prepared by conventional techniques. The preferred daily dosage amount of a compound of Formula (I) administered by inhalation is from about 10 mg to about 100 mg per day.

This invention also relates to a method of treating a disease state which is mediated by the 5-lipoxygenase pathway in an animal in need thereof, including humans and other mammals, which comprises administering to such animal an effective, 5-lipoxygenase pathway inhibiting amount of a Formula (I) compound. By the term "treating" is meant either prophylactic or therapeutic therapy. By the term "mediated" is meant caused by or exacerbated by. Such Formula (I) compound can be administered to such animal in a conventional dosage form prepared by combining the Formula (I) compound with a conventional pharmaceutically acceptable carrier or diluent according to known techniques. It will be recognized by one of skill in the art that the form and character of the pharmaceutically acceptable carrier or diluent is dictated by the amount of active ingredient with which it is to be combined, the route of administration and other well-known variables. The Formula (I) compound is administered to an animal in need of inhibition of the 5-lipoxygenase pathway in an amount sufficient to inhibit the 5-lipoxygenase pathway. The route of administration may be oral, parenteral, by inhalation or topical. The term parenteral as used herein includes intravenous, intramuscular, subcutaneous, intrarectal, intravaginal or intraperitoneal administration. The subcutaneous and intramuscular forms of parenteral administration are generally preferred. The daily parenteral dosage regimen will preferably be from about 50 mg to about 1000 mg per day. The daily oral dosage regimen will preferably be from about 150 mg to about 2000 mg per day. It will be recognized by one of skill in the art that the optimal quantity and spacing of individual dosages of the Formula (I) compound will be determined by the nature and extent of the condition being treated, the form, route and site of administration, and the particular animal being treated, and that such optimums can be determined by conventional techniques. It will also be appreciated by one of skill in the art that the optimal course of treatment, i.e., the number of doses of the Formula (I) compound given per day for a defined number of days, can be ascertained by those skilled in the art using conventional course of treatment determination tests.

In the tests used to determine activity as 5-lipoxygenase pathway inhibitors, male Balb/c mice (20-28 g), were used. All mice were obtained from Charles River Breeding Laboratories, Kingston, N.Y. Within a single experiment, mice were age matched.

Reagents were employed as follows:

Compounds of Formula (I) were each used as the free base. The compounds were homogenized in 0.5% tragacanth. Compounds were administered by lavage at the indicated dose in a final volume of 10 ml/kg.

For in vitro experiments, compounds were dissolved at appropriate concentrations in ethanol (final concentration 1.0%) and then diluted to final concentrations using the buffers indicated in the text.

## Arachidonic Acid-Induced Mouse Ear Inflammation

Arachidonic acid in acetone (2 mg/20 µl) was applied to the inner surface of the left ear. The thickness of both ears was then measured with a dial micrometer one hour after treatment, and the data were expressed as the change in thickness ($10^{-3}$ cm) between treated and untreated ears.

Test compounds were given orally in acid/saline at the times indicated in the text prior to the topical application of arachidonic acid.

## Assay of 5-Lipoxygenase Activities

The activities of these enzymes in extracts of RBL-1 cells were assayed using the method of Jakschik and Lee, Nature, 287, 51-52 (1980). RBL-1 cells were obtained from the American Type Culture Collection (#CRL 1378) and were grown at 37°C (5% $CO_2$ in air) in spinner culture in MEM supplemented with 10% heat inactivated fetal calf serum. Harvested cells were washed with 50 mM sodium phosphate buffer, pH 7.0, containing 1 mM EDTA and 0.1% gelatin, resuspended in fresh buffer (5 x $10^7$ cells/ml) and disrupted

by nitrogen cavitation using the Parr bomb at 750 psi for 10 min. The broken cell extract was then centrifuged at 10,000 x g for 20 minutes (min) and the supernatant was centrifuged at 100,000 x g for 60 min. Aliquots (0.25 mls) of the supernatant were preincubated with or without drugs for 10 min, after which 10 ul $CaCl_2$ (2 mM) was added and the reaction was initiated with 2.5 $\mu$l of 2.5 mM arachidonic acid-1-$^{14}$C (final concentration was 25 $\mu$M; specific activity 20,000 dpm/nmole). After incubation for 5 minutes at 37°C, the reaction was terminated by addition of 2 volumes (0.5 ml) ice cold acetone and the sample was allowed to deproteinize on ice for 10 minutes prior to centrifugation at 1,000 x g for 10 minutes. The deproteinized supernatant was adjusted to pH 3.5 with 2N formic acid and extracted with 2 volumes of ice cold ethyl acetate. The extracted samples were dried under argon, redissolved in ethyl acetate and applied to Whatman LK5D thin layer chromatography (TLC) plates which were developed using the A-9 solvent system [organic phase of ethyl acetate: 2,2,5-trimethylpentane:acetic acid: water (110:50:20:10)] described by Hamberg and Samuelsson, J. Biol. Chem., 241, 257-263 (1966). Arachidonic acid, 5-HETE, $LTB_4$ and $PGD_2$ were quantified with a Berthold LB 2832 autoscanner.

Under these conditions, only the 5-lipoxygenase pathway metabolites were detectable. The 5-HETE and di-HETEs were formed at a linear rate, and substantial amounts of the arachidonic acid-1-$^{14}$C substrate were utilized.

Drug-induced effects on enzyme activities are described as the concentration of drug causing a 50% inhibition of metabolite synthesis ($IC_{50}$).

LTC-4 Production by Human Monocytes

Human monocytes were prepared from whole blood supplied by the American Red Cross. The blood was fractionated by a two-step procedure employing sedimentation on Ficoll follwed by sedimentation on Percoll. The mononuclear cell fraction recovered was composed of 80-90% monocytes with the remainder of the cells being predominantly lymphocytes. The monocytes were plated at $1x10^6$ cells per well in a Costar 24 well tissue culture plate and allowed to adhere for 1 hour at 37°. Non-adherent cells were removed by washing the cells were stimulated with 1 uM A23187 calcium ionophore for 3 hours at 37° to induce LTC-4 production when drugs were evaluated. They were added to the cells 30 minutes prior to the A23187. Supernatants were collected, clarified by centrifugation and store frozen at -20°C until assay. The LTC-4 content was determined by using a New England Nuclear Leukotriene C-4 ($^3$H) RIA Kit as per instructions.

Inhibition of the Eicosanoid Production Following Calcium Ionophore (60$\mu$M) Stimulation in Human Whole Blood

The eicosanoids, which include the 5-lipoxygenase products LTB , trans$LTB_4$, 20-hydroxy$LTB_4$, 5-HETE, and the 12-lipoxygenase product are extracted from the whole blood following A23187 calcium ionophore stimulation. The extracts are separated by reverse phase high pressure liquid chromatography and quantified by absorbance methods.

Venous human blood is collected into polypropylene tubes containing 1% heparin. The blood is then aliquoted into 4.5 ml volumes and preincubated at 37°C for 10 minutes in polypropylene tubes (15 ml size). Compound or carrier (50 $\mu$L dimethylsulfoxide) is added 5 minutes prior to stimulation. Calcium ionophore (0.5ml) is added, and the blood incubated for 10 minutes. Prostaglancin $B_2$(1 nmole) is added, and the blood extracted as described below.

The samples are centrifuged at 1000xg for 15 minutes at 5°C. The plasma is collected, and one volume of methanol is added to the plasma. This suspension is then centrifuged at 1000xg for ten minutes at 5°C. The supernatant is collected and diluted with 1.5 volumes of chilled aqueous 1% formic acid: 1% triethylamine. This mixture is loaded onto a preconditioned J.T. Baker C18 SPE cartridge (Phillipsburg, NJ) at a flow rate of 1-2 ml/minute. (The cartridge is preconditioned according to manufacture's recommendations.) The absorbed sample is washed in the following order with three (3) ml each of (i) aqueous 1% formic acid: 1% triethylamine; (ii) petroleium ether; and (iii) 20% acetonitrile: 1% triethylamine.

The eicosanoids are eluted in 3ml of 70% acetonitrile: 1% triethylamine. The solvent is removed under vacuum. The sample is resuspended in 200$\mu$L of 50% methanol buffered with ammonium acetate.

The sample (175 $\mu$l) is loaded into a WATERS (Milbord, MA) RCM NOVA PAK C18 (100x8mm) column with the starting mobile phase of 90% A (A = 10% acetonitrile buffered with 30mM ammonium acetate to pH6.8) and 10% B (B = 90% acetonitrile buffered with 30mM ammonium acetate to pH6.8). The flow rate for the separation is 2.5ml/minute. At one minute the %B is increased to 27% in a step fashion. By 12 minutes the %B has increased in a concave hyperbolic function (curve 9) to 40% and increases in a linear

manner to 60% by 22 minutes. Under these developing conditions, the retention times for the eicosanoids are: 20-hydroxy$LTB_4$, 4.6 minutes; trans$LTB_4$, 10 minutes; $LTB_4$, 10.5 minutes; 12-HETE, 10.4 minutes; 5-HETE, 21 minutes. The HPLC system consisted of WATERS 510 pumps, 840 controller, WISP injector and 990 detector.

The eicosanoids in the samples are verified by their retention times and their UV absorbance spectra. The peaks are quantified with reference to the internal standard and their absorbance response at their maximum absorbance wavelength.

The Effect of Compounds of Formula (I) on Arachidonic Acid-induced Inflammation

Elucidation of the antiinflammatory activity of the compounds of Formula (I) was achieved in a model of arachidonic acid-induced edema in mice. The mouse ear edematous response to arachidonic acid has been shown to be sensitive to agents that inhibit both lipoxygenase- and cyclooxygenase-generated mediators or that selectively inhibit lipoxygenase, but not cyclooxygenase, enzyme activity [See, Young et al., J. Invest. Dermatol., 82, 367-371 (1984)]. Compounds of Formula (I) produced marked inhibition of the edematous response normally seen 1 hour after the application of 2 mg of arachidonic acid to the ear (Table I). The cyclooxygenase inhibitors, indomethacin (10 mg/kg, p.o.), ibuprofen (250 mg/kg, p.o.) and naproxen (100 mg/kg, p.o.) do not exhibit detectable antiinflammatory activity in this assay.

These findings indicate that compounds of Formula (I) are potent inhibitors of both the cellular and edematous responses of inflammation in mice. These inflammatory responses were also inhibited by agents that inhibit lipoxygenase activity but not by selective cyclooxygenase inhibitors.

The Effect of Compounds of Formula (I) on Arachidonic Acid Metabolism

Experiments using a soluble extract preparation of RBL-1 cells containing only lipoxygenase activity confirmed the inhibitory effects of compounds of Formula (I) on $LTB_4$ production (Table II). Indomethacin at concentrations up to $10^{-4}$ M was inactive. The data presented in Table II indicate that compounds of Formula (I) are inhibitors of the 5-lipoxygenase pathway as confirmed by their ability to inhibit $LTB_4$, a 5-lipoxygenase pathway product. The data presented in Table III indicate that compounds of Formula (I) are inhibitors of the 5-lipoxygenase pathway as confirmed by their ability to inhibit 5-HETE, a 5-lipoxygenase pathway product.

$LTC_4$ Inhibition Assay

As shown in Table IV, compounds of Formula (I) were efficacious in inhibiting $LTC_4$ production, a 5-lipoxygenase pathway product, by human monocytes. These data confirm the ability of compounds of Formula (I) to inhibit the 5-lipoxygenase pathway.

Inhibition of Eicosanoid Production

As shown in Table V, compounds of Formula (I) were effective in inhibiting the production of various 5-lipoxygenase pathway products in human blood. This data demonstrates that the compounds of Formula (I) inhibit the 5-lipoxygenase pathway.

## Table I

The Effect of Compounds of Formula (I) on Arachidonic Acid Induced Ear Swelling

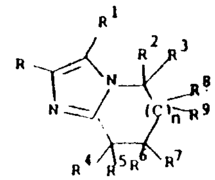

FORMULA (I)

| Compound Number | R | $R^1$ | $R^2$ | $R^3$ | $R^4$ | $R^5$ | $R^6$ | $R^7$ | $R^8$ | $R^9$ | n | % Inhibition of Ear Swelling (a,b,c) |
|---|---|---|---|---|---|---|---|---|---|---|---|---|
| 1 | 4-methylthiophenyl | 4-pyridyl | H | H | H | H | H | H | – | – | 0 | 62*** |
| 2 | 4-methylsulfinylphenyl | 4-pyridyl | H | H | H | H | H | H | – | – | 0 | 44*** p.o. |
| 3 | 4-methylsulfonylphenyl | 4-pyridyl | H | H | H | H | H | H | – | – | 0 | 3NS |
| 4 | 4-methoxyphenyl | 2-pyridyl | H | H | H | H | H | H | – | – | 0 | 29** |
| 5 | 4-methoxyphenyl | 3-pyridyl | H | H | H | H | H | H | – | – | 0 | 34** |
| 6 | 4-methoxyphenyl | 2,6-dimethyl-4-pyridyl | H | H | H | H | H | H | – | – | 0 | NT |
| 7 | 4-hydroxyphenyl | 4-pyridyl | H | H | H | H | H | H | – | – | 0 | 27***p.o. |
| 8 | 4-ethoxyphenyl | 4-pyridyl | H | H | H | H | H | H | – | – | 0 | 62***p.o. |
| 9 | 4-n-propoxyphenyl | 4-pyridyl | H | H | H | H | H | H | – | – | 0 | 42***p.o. |
| 10 | 4-isopropoxyphenyl | 4-pyridyl | H | H | H | H | H | H | – | – | 0 | 43***p.o. |

a) screened at 50 mg/kg s.c. or i.p. unless indicated as oral dosing (p.o.).

b) *=p .05, **=p .01, ***=p .001, NS = not significant.

c) NT = Not Tested

Table I (continued)

| Compound Number | R | $R^1$ | $R^2$ | $R^3$ | $R^4$ | $R^5$ | $R^6$ | $R^7$ | $R^8$ | $R^9$ | n | % Inhibition of Ear Swelling (a,b,c) |
|---|---|---|---|---|---|---|---|---|---|---|---|---|
| 11 | 4-acetylthiophenyl | 4-pyridyl | H | H | H | H | H | H | – | – | 0 | 46***p.o. |
| 12 | 4-trimethylacetylthiophenyl | 4-pyridyl | H | H | H | H | H | H | – | – | 0 | 45***p.o. |
| 13 | 4-acetoxymethylthiophenyl | 4-pyridyl | H | H | H | H | H | H | – | – | 0 | 51***p.o. |
| 14 | 4-ethylthiophenyl | 4-pyridyl | H | H | H | H | H | H | – | – | 0 | 56***p.o. |
| 15 | 4-ethylsulfinylphenyl | 4-pyridyl | H | H | H | H | H | H | – | – | 0 | 41***p.o. |
| 16 | 4-carbethoxymethylthiophenyl | 4-pyridyl | H | H | H | H | H | H | – | – | 0 | 15*** |
| 17 | 4-pyridyl | H | H | H | H | H | H | H | – | – | 0 | 20**p.o.<br>41*** |
| 18 | 4-pyridyl | 4-methylthiophenyl | H | H | H | H | H | H | – | – | 0 | 28***p.o.<br>32*** |
| 19 | 4-pyridyl | 4-methylsulfinylphenyl | H | H | H | H | H | H | – | – | 0 | 52***p.o.<br>56*** |
| 20 | 4-methylthiophenyl | 4-(2-methyl)pyridyl | H | H | H | H | H | H | – | – | 0 | 59***p.o.<br>59*** |
| 21 | 4-methylsulfinylphenyl | 4-(2-methyl)pyridyl | H | H | H | H | H | H | – | – | 0 | 59***p.o.<br>14* |
| 22 | 4-methoxyphenyl | 4-(2-methyl)pyridyl | H | H | H | H | H | H | – | – | 0 | 56***p.o.<br>57*** |

a) screened at 50 mg/kg s.c. or i.p. unless indicated as oral dosing (p.o.).

b) *=p .05, **=p .01, ***=p .001, NS = not significant.

c) NT = Not Tested

Table II

The Effect of Compounds of Formula (I) on 5-Lipoxygenase Activity ($LTB_4$ Production)

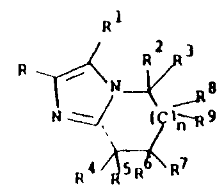

FORMULA (I)

| Compound Number | R | $R^1$ | $R^2$ | $R^3$ | $R^4$ | $R^5$ | $R^6$ | $R^7$ | $R^8$ | $R^9$ | n | 5-LO[a,b] $IC_{50}$ (μM) |
|---|---|---|---|---|---|---|---|---|---|---|---|---|
| 1 | 4-methylthiophenyl | 4-pyridyl | H | H | H | H | H | H | – | – | 0 | 20 |
| 2 | 4-methylsulfinylphenyl | 4-pyridyl | H | H | H | H | H | H | – | – | 0 | >100 |
| 3 | 4-methylsulfonylphenyl | 4-pyridyl | H | H | H | H | H | H | – | – | 0 | >100 |
| 4 | 4-methoxyphenyl | 2-pyridyl | H | H | H | H | H | H | – | – | 0 | >100 |
| 5 | 4-methoxyphenyl | 3-pyridyl | H | H | H | H | H | H | – | – | 0 | NT |
| 6 | 4-methoxyphenyl | 2,6-dimethyl-4-pyridyl | H | H | H | H | H | H | – | – | 0 | NT |
| 7 | 4-hydroxyphenyl | 4-pyridyl | H | H | H | H | H | H | – | – | 0 | 17 |
| 8 | 4-ethoxyphenyl | 4-pyridyl | H | H | H | H | H | H | – | – | 0 | 6 |
| 9 | 4-n-propoxyphenyl | 4-pyridyl | H | H | H | H | H | H | – | – | 0 | 3.5 |
| 10 | 4-isopropoxyphenyl | 4-pyridyl | H | H | H | H | H | H | – | – | 0 | NT |

[a] $IC_{50}$ determined on $LTB_4$ production by RBL-1 high speed supernatant.

[b] NT = Not tested.

## Table III

The Effect of Compounds of Formula (I) on 5-Lipoxygenase Activity (Total HETE and Di-HETE) Production

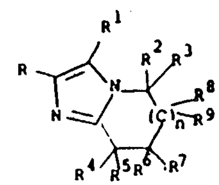

FORMULA (I)

| Compound Number | R | $R^1$ | $R^2$ | $R^3$ | $R^4$ | $R^5$ | $R^6$ | $R^7$ | $R^8$ | $R^9$ | n | 5-LO[a,b] $IC_{50}$ (μM) |
|---|---|---|---|---|---|---|---|---|---|---|---|---|
| 2 | 4-methylsulfinylphenyl | 4-pyridyl | H | H | H | H | H | H | - | - | 0 | >100 |
| 3 | 4-methylsulfonylphenyl | 4-pyridyl | H | H | H | H | H | H | - | - | 0 | NT |
| 5 | 4-methoxyphenyl | 3-pyridyl | H | H | H | H | H | H | - | - | 0 | NT |
| 6 | 4-methoxyphenyl | 2,6-dimethyl-4-pyridyl | H | H | H | H | H | H | - | - | 0 | NT |
| 7 | 4-hydroxyphenyl | 4-pyridyl | H | H | H | H | H | H | - | - | 0 | NT |
| 9 | 4-n-propoxyphenyl | 4-pyridyl | H | H | H | H | H | H | - | - | 0 | 8 |
| 10 | 4-isopropoxyphenyl | 4-pyridyl | H | H | H | H | H | H | - | - | 0 | NT |

[a] $IC_{50}$ determined on total HETE and Di-HETE production by RBL-1 high speed supernatant.

[b] NT - Not tested.

Table III (Continued)

| Compound Number | R | $R^1$ | $R^2$ | $R^3$ | $R^4$ | $R^5$ | $R^6$ | $R^7$ | $R^8$ | $R^9$ | n | 5-LO[a,b] $IC_{50}$ (μM) |
|---|---|---|---|---|---|---|---|---|---|---|---|---|
| 11 | 4-acetylthiophenyl | 4-pyridyl | H | H | H | H | H | H | - | - | 0 | 25 |
| 12 | 4-trimethylacetylthiophenyl | 4-pyridyl | H | H | H | H | H | H | - | - | 0 | 6 |
| 13 | 4-acetoxymethylthiophenyl | 4-pyridyl | H | H | H | H | H | H | - | - | 0 | 47 |
| 14 | 4-ethylthiophenyl | 4-pyridyl | H | H | H | H | H | H | - | - | 0 | 30 |
| 15 | 4-ethylsulfinylphenyl | 4-pyridyl | H | H | H | H | H | H | - | - | 0 | >100 |
| 16 | 4-carbethoxymethylthio | 4-pyridyl | H | H | H | H | H | H | - | - | 0 | 50 |

[a] $IC_{50}$ determined on total HETE and Di-HETE production by RBL-1 high speed supernatant.

[b] NT - Not tested.

Table IV

The Effect of Compounds of Formula (I) on 5-Lipoxygenase Activity ($LTC_4$ Production)

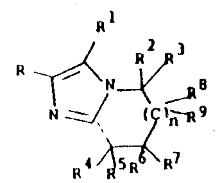

FORMULA (I)

| Compound Number | R | $R^1$ | $R^2$ | $R^3$ | $R^4$ | $R^5$ | $R^6$ | $R^7$ | $R^8$ | $R^9$ | n | 5-LO[a,b] $IC_{50}$ (μM) |
|---|---|---|---|---|---|---|---|---|---|---|---|---|
| 1 | 4-methylthiophenyl | 4-pyridyl | H | H | H | H | H | H | - | - | 0 | 7 |
| 2 | 4-methylsulfinylphenyl | 4-pyridyl | H | H | H | H | H | H | - | - | 0 | 95 |
| 3 | 4-methylsulfonylphenyl | 4-pyridyl | H | H | H | H | H | H | - | - | 0 | 37 |
| 4 | 4-methoxyphenyl | 2-pyridyl | H | H | H | H | H | H | - | - | 0 | 26 |
| 5 | 4-methoxyphenyl | 3-pyridyl | H | H | H | H | H | H | - | - | 0 | 21 |
| 6 | 4-methoxyphenyl | 2,6-dimethyl-4-pyridyl | H | H | H | H | H | H | - | - | 0 | NT |
| 7 | 4-hydroxyphenyl | 4-pyridyl | H | H | H | H | H | H | - | - | 0 | 27 |
| 8 | 4-ethoxyphenyl | 4-pyridyl | H | H | H | H | H | H | - | - | 0 | 12 |
| 9 | 4-n-propoxyphenyl | 4-pyridyl | H | H | H | H | H | H | - | - | 0 | 13 |
| 10 | 4-isopropoxyphenyl | 4-pyridyl | H | H | H | H | H | H | - | - | 0 | 13 |

a $IC_{50}$ determined on $LTC_4$ production by human monocytes.
b NT = not tested

Table IV (Continued)

| Compound Number | R | $R^1$ | $R^2$ | $R^3$ | $R^4$ | $R^5$ | $R^6$ | $R^7$ | $R^8$ | $R^9$ | n | 5-LO[a,b] $IC_{50}$ (μM) |
|---|---|---|---|---|---|---|---|---|---|---|---|---|
| 11 | 4-acetylthiophenyl | 4-pyridyl | H | H | H | H | H | H | - | - | 0 | 3 |
| 12 | 4-trimethylacetylthiophenyl | 4-pyridyl | H | H | H | H | H | H | - | - | 0 | 4 |
| 13 | 4-acetoxymethylthiophenyl | 4-pyridyl | H | H | H | H | H | H | - | - | 0 | 2.6 |
| 14 | 4-ethylthiophenyl | 4-pyridyl | H | H | H | H | H | H | - | - | 0 | 9 |
| 15 | 4-ethylsulfinylphenyl | 4-pyridyl | H | H | H | H | H | H | - | - | 0 | - |
| 16 | 4-carbethoxymethylthiophenyl | 4-pyridyl | H | H | H | H | H | H | - | - | 0 | 4 |

[a] $IC_{50}$ determined on $LTC_4$ production by human monocytes.
[b] NT = not tested

Table V

The Effect of Compounds of Formula (I) Eicosanoid Production

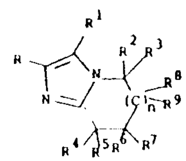

FORMULA (I)

| Compound Number | R | $R^1$ | $R^2$ | $R^3$ | $R^4$ | $R^5$ | $R^6$ | $R^7$ | $R^8$ | $R^9$ | n | 5-LO, $IC_{50}$ (uM)[a,b] tLTB$_4$ | $LTB_4$ | 5-HETE | 12-HETE | 20-OH-$LTR_4$ |
|---|---|---|---|---|---|---|---|---|---|---|---|---|---|---|---|---|
| 1 | 4-methylthiophenyl | 4-pyridyl | H | H | H | H | H | H | - | - | 0 | 30 | 30 | 30 | 60 | 30 |
| 2 | 4-methylsulfinylphenyl | 4-pyridyl | H | H | H | H | H | H | - | - | 0 | 80 | 80 | 80 | 80 | NT |

a $IC_{50}$ determined on human blood stimulated with calcium ionophore.

b NT = Not Tested

TABLE VI

| | 5-LO Inhibition relative to Compound C | Adverse CNS activity* (Log D) (Lipophilicity) | P-450 Inhibition IC50-uM | AAEE(MOUSE ED50 mg/kg (po) |
|---|---|---|---|---|
| A. 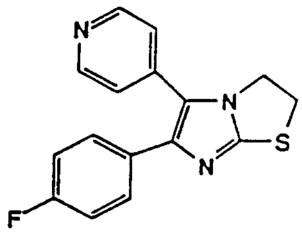 | 1.5 | +++ (2.19) | 0.6 | 27 |
| B. 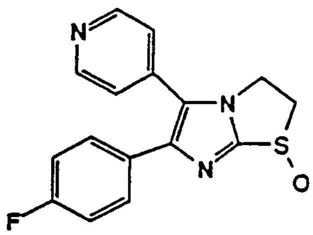 | | (1.20) | 5.0 | |
| C. 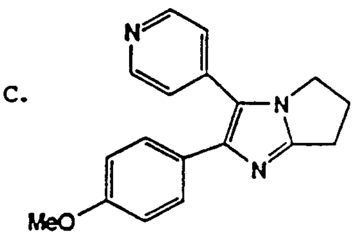 | 1 | +++ (2.14) | 21.4 | 28 |
| D. 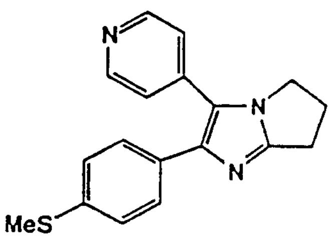 | | + (2.68) | 43.7 | 20 |
| E. 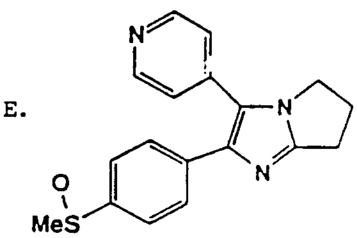 | NA | (0.85) | 866 | 44 |

*Plus sign indicates presence and minus sign absence.

Table VIII

P-450 Inhibition

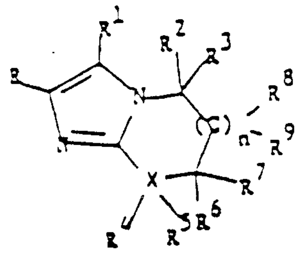

| No. | X | $R^1$ | R | $IC_{50}$ µM |
|---|---|---|---|---|
| 1 | S | 4-pyridyl | 4-methoxyphenyl | 12.5 |
| 2 | SO | 4-pyridyl | 4-methoxyphenyl | >100* |
| 3 | $SO_2$ | 4-pyridyl | 4-methoxyphenyl | 26.2 |
| 4 | S | 4-pyridyl | 4-fluorophenyl | 0.9 |
| 5 | $SO_2$ | 4-pyridyl | 4-fluorophenyl | 21.4 |
| 6 | C | 4-fluorophenyl | 4-pyridyl | 0.5 |
| 7 | C | 4-pyridyl | 4-(1-ethoxy)phenyl | 8.4 |
| 8 | C | 4-pyridyl | 4-(1-propoxy)phenyl | 62.7 |
| 9 | C | 4-pyridyl | 4-(2-propoxy)phenyl | 2.5 |
| 10 | C | 4-N-methylpyridyl | 4-methoxyphenyl | 108 |
| 11 | C | 4-pyridyl | 4-methylthiophenyl | 43.7 |
| 12 | C | 4-pyridyl | 4-methylsulfinylphenyl | 866 |
| 13 | C | 4-pyridyl | 4-methylsulfonylphenyl | >100 |
| 14 | C | 4-pyridyl | 4-ethylthiophenyl | 28.1 |
| 15 | C | 4-pyridyl | 4-ethylsulfinylphenyl | >100 |
| 16 | C | 2-pyridyl | 4-methoxyphenyl | >100 |
| 17 | C | 3-pyridyl | 4-methoxyphenyl | >100 |
| 18 | C | H | 4-pyridyl | 80.1 |
| 19 | C | 4-methylthiophenyl | 4-pyridyl | 5.2 |
| 20 | C | 4-methylsulfinylphenyl | 4-pyridyl | 13.7 |
| 21 | C | 4-(2-methyl)pyridyl | 4-methylthiophenyl | 57.5 |
| 22 | C | 4-(2-methyl)pyridyl | 4-methylsulfinylphenyl | >1000 |
| 23 | C | 4-(2-methyl)pyridyl | 4-methoxyphenyl | 56.6 |

It has now been found that the compounds of the present invention have superior properties over previously known compounds as summarized by the data in Table VI. Compound A, 5-(4-pyridyl)-6-(4-fluorophenyl)-2,3-dihydroimidazo[2,1-b]thiazole, and compound B, 5-(4-pyridyl)-6-(4-fluorophenyl)-2,3-dihydroimidazo [2,1-b]thiazole oxide, are representative of compounds taught in U.S. Patent 4,175,127 issued November 20, 1979. Compound C,2-(4-methoxyphenyl)-3-(4-pyridyl)-6,7-dihydro-[5H]-pyrrolo[1,2-a]-

imidazole is specifically taught in European Patent Application 231,622 published August 12, 1987. Compound D, 2-(4-methylthiophenyl)-3-(4-pyridyl)-6,7-dihydro-[5H]-pyrrolo[1,2-a]imidazole and Compound E, 2-(4-methylsulfinylphenyl)-3-(4-pyridyl)-6,7-dihydro-[5H]-pyrrolo[1,2-a]imidazole are representative of the present invention.

Structural modifications described in this application have reduced the severity, when compared to prior art compounds, of two undesirable side effects: 1) CNS toxicity; and 2) inhibition of cytochrome P-450 dependent enzyme activities, which is a deficiency that could result in clinically relevant drug interactions. More specifically, the elimination of the sulfur on the bicyclic fused ring nucleus of the compounds and replacement of the fluorine with a methoxy group reduced inhibition of cytochrome P-450 dependent enzymes, but did not eliminate the presence of adverse central nervous system (CNS) activity. This is shown by a comparison of the data in Table VI for compound C with A and B. It was believed that the CNS effects were related to the ability of a compound to penetrate the CNS and hence to lipophilicity. Compounds A and C are both highly lipophilic and demonstrated similar CNS effects. The log D shown on Table VI is a measure of lipophilicity determined via high pressure liquid chromatography. However, it was found that introduction of polarity into the phenyl ring, pyridyl ring, or bicyclic fused ring reduced 5-lipoxygenase inhibition activity. A comparison of compounds D and E in Table VI demonstrate this effect for introduction of polarity into the phenyl ring and comparison of compounds A and B demonstrates this effect for the bicyclic fused ring.

A comparison of compounds A and B demonstrated a reduction in the undesirable CNS activity. A similar effect is shown comparing claimed compounds D and E. Incorporation of polarity into A yielded compound B, and reduced CNS toxicity. Incorporation of polarity into compound D yielded compound E, and reduced CNS toxicity. Further compound E is metabolized in vivo to compound D. Therefore, conversion of a polar but inactive prodrug (E) in vivo to its metabolite (D) reduces CNS toxicity. In addition compound D has less CNS toxicity than prior art compound A. Thus the claimed compounds D and E have reduced inhibition of cytochrome P-450 dependent enzymes and reduced adverse CNS activity. This conclusion is further supported by the following data.

LOG D Determination

The procedure used to determine the log D's listed on Table VI was as follows. A 20 μl sample was injected into a Shandon Hypersil ODS, 5μ (100 mm x 4.6 mm ID) column and was eluted using a mobile phase of 64:35 MeOH:$H_2O$ (The aqueous portion was .01 M in $KH_2PO_4$ and adjusted to pH 7.4 with KOH after mixing the MeOH), at a flow rate of 2 ml per minute. Eluting peaks were detected by UV absorbance at 0.01 mg/ml. All samples were made up at 0.1 mg/ml. (Retentions were identical at 0.01 mg/ml.)

The data was analyzed by determining the regression line corresponding to the log k′ vs. literature log P of the reference standards. (See Unger, S.H. et al., J. Pharm. Sci., 67, 1364 (1978). The log P (log D) was then determined for the test sample from its log k′ on this line. Reproduciblity was usually better than 0.5%.

The reference standards and their literature log P's included $NaNO_2$, 0.0; acetanalide, 1.16; acetophenone, 1.66; anisole, 2.08; chlorobenzene, 2.84; benzophenone, 3.18; anthracene, 4.45; and pentachlorobenzene, 5.12.

CNS Activity

Effects on the central nervous system (CNS) of the claimed and prior art compounds was demonstrated in cynomolgus monkeys.

Oral administration of 90 mg/kg/day of compound A on Table VI, 5-(4-pyridyl)-6-(4-fluorophenyl)-2,3-dihydro imidazo[2,1-b]thiazole to two cynomolgus monkeys (1 female, 1 male) for two consecutive days induced body tremors in both monkeys and severe, recurrent convulsions in the male animal. Administration of 30 mg/kg/day of compound A on Table VI to two cynomolgus monkeys (1 female, 1 male) for 5 or 6 consecutive days was associated with emesis and gastric ulceration in both monkeys but with no evidence of convulsions or body tremors. Monkeys, when administered a second dose of 90 mg/kg of compound A died with convulsions within 1 to 5 hours after dosing.

A single oral dose of 90 mg/kg of compound C in Table VI, 2-(4-methoxyphenyl)-3-(4-pyridyl)-6,7-dihydro-[5H]-pyrrolo[1,2-a]imidazole to 1 female and 1 male monkey resulted in death of both animals. The male became sedated, lost consciousness and died within 1.5 hours of dosing; the female demonstrated both decreased motor activity and convulsions prior to death within 3.5 hours of dosing. A single oral dose of 60 mg/kg to two additional monkeys resulted in sedation, loss of consciousness and death of one (male) within 1 hour of dosing. Additional monkeys tolerated repeated doses of 45 mg/kg or 30 mg/kg and one

animal tolerated an escalating dose schedule of 30-90-120 mg/kg.

Two monkeys (1 female, 1 male) were gavaged with 90 mg/kg of compound E, 2-(4-methylsulfinylphenyl-3-(4-pyridyl)-6,7-dihydro-[5H]-pyrrolo[1,2-a]imidazole, and clinical effects were not observed. Additional monkeys were orally administered 200, 400 or 800 mg/kg of compound E to probe the limiting dose and toxic effect(s). Both monkeys receiving 800 mg/kg died, the female within 2 hours of dosing and the male between 12-24 hours of dosing. Convulsions in these animals were not observed.

The two monkeys administered 400 mg/kg were repeatedly administered this dose for 7 consecutive days; each animal experienced emesis within 1 to 5 hours of dosing after the 1st, 3rd and 4th doses, whereas only the female experienced emesis after the 5th, 6th and 7th doses. A complete necropsy, serum clinical chemistry, hematology and histological examination was completed on both monkeys. Evidence of drug related change was not observed. Emesis was the only observation in monkeys administered 400 mg/kg/day of compound E for 7 consecutive days.

In summary, compounds A and C each caused convulsions and death after either two or one doses respectively of 90 mg/kg, while compound E at that dosage caused no observable clinical effects. Compound E administered at 400 mg/kg/day for seven days caused only emesis. Thus compound E does not have the adverse CNS effects demonstrated by the prior art compounds A and C.

Cytochrome P-450 Inhibition

The inhibitory effect of several compounds on hepatic cytochrome P-450 dependent mixed function oxidase activity was evaluated in vitro in rat microsomes using the prototypical substrate, ethoxycoumarin, as follows. Animals: Male Sprague-Dawley rats, 9-10 weeks of age and weighing 300-340 g, were dosed daily with Na-phenobarbital for three days, i.p. (1 ml/kg in ultrapure $H_2O$), at 80 mg/kg/day. The animals were killed by cervical dislocation 24 hours after the last dose and pooled hepatic microsomes were prepared by differential centrifugation. Microsomes were stored at -80 °C.

In vitro enzyme studies: The possible inhibitory effects of several compounds listed on Table VIII on hepatic cytochrome P-450 dependent mixed function oxidase activity were assessed using ethoxycoumarin-O-deethylase (ECOD) activity. The deethylation of the substrate, 7-ethoxycoumarin, is detected by measuring the fluorescence of 7-hydroxycoumarin according to the method of Lee N.H. et al., Toxicologist, 5, 164 (1985). Microsomal incubations consisting of 15 µl pooled Na-phenobarbital-induced microsomes (approx. 0.3 mg/ml microsomal protein) and 875 µl reaction mixture of 0.45 mM 7-ethoxycoumarin, 5 mM glucose-6-phosphate, 0.5 units/ml glucose-6-phosphate dehydrogenase and 5 mM $MgCl_2$ in 0.1 M N-2-hydroxyethylpiperazine-N'-2-ethanesulfonic acid,, pH 7.8 were prepared. Varying concentrations of the test compounds, dissolved in a small amount of dimethyl sulfoxide (10 µ), were added directly to the incubations. Solvent controls were incubated in the presence of dimethyl sulfoxide. After a two minute preincubation at 37 °C, the deethylation reaction was initiated by adding 100 µl 0.74 mM B-nicotinamideadenine dinucleotide phosphate/0.74 mM B-nicotinamideadenine dinucleotide. The reaction was stopped after incubating for 10 minutes at 37 °C by adding 2.5 ml basic MeOH, pH 9.0. The samples were spun at 2500 revolutions per minute for 15 minutes. Two mls of the supernatent was transferred into disposable fluorescence cuvets and the fluoroscence of each sample was measured at the excitation wavelength 390 nm and emission wavelength 454 nm. The PROBIT procedure was used to calculate $IC_{50}$ values according to the SAS Institute Inc., SAS User's guide: Statistics, 1982 Edition, Cary NC: SAS Institute Inc. 1982, 287 pp.

The results were summarized in Table VIII and demonstrate that compounds of the claimed invention have reduced inhibition of P-450 enzyme compared to previously known compounds. Compounds 1 to 6 represent previously known compounds. The $IC_{50}$, which is the concentration at which 50% of the enzyme activity is inhibited, was less than 30 µM for each of these comounds except compound 2. Compounds 7 to 17 represent the claimed invention. The majority of these compounds have an $IC_{50}$ above 30 µM. Compounds of the claimed invention, because of the reduced inhibition of cytochrome P-450 dependent enzymes, would be expected to have significantly less clinically relevant drug interactions than prior art compounds.

The following examples are to be construed as merely illustrative and not a limitation of the scope of the present invention in any way.

Temperature is in degrees Centigrade ( °C).

EXAMPLE 1

2-(4-Fluorophenyl)-6,7-dihydro-(5H)-pyrrolo[1,2-a]imidazole (Formula (E) Compound)

Method A.

A stirred solution of 15g (87 mmoles) of 2-chloro-4-fluoroacetophenone in 75ml of SD 30 alcohol was treated at 25° C with 10.65g (104 mmoles) of 2-iminopyrrolidine, resulting in an exothermic temperature rise to 40°C. After stirring for one hour (hr), approximately 75ml of ethyl acetate was added, and the mixture was extracted with dilute HCl to dissolve the precipitate. The aqueous acidic extract was separated from the organic phase, adjusted to a pH between 4 and 5, and heated on a steam bath for 24 hrs. The solution was adjusted to pH 2, extracted with ether, brought to pH 8, and extracted with methylene chloride. The basic organic phase was chromatographed on silica, eluting with 4% methanol in methylene chloride. The residue obtained on concentration of the pooled fractions was recrystallized from CC14, melting point (mp) 137.5-139°C.

Method B.

(a) 1-(4-Fluorophenyl)-2-(2-iminopyrrolidin-1-yl)-ethanone hydrocholoride (Formula (H) compound

A stirred solution of 37.3g (216 mmoles) of 2-chloro-1-(4-fluorophenyl)ethanone (prepared as described by Joshi et al., J. Heterocyclic Chem. 16, 1141 (1979)) in 70 ml of chloroform chilled in a methanol-ice bath between 15-18°C, was treated with a solution of 20g (238 mmoles) of 2-imino-pyrrolidine in 50 ml of chloroform at such a rate as to maintain the temperature of the reaction mixture. After an additional 2 hours, the mixture was triturated with 300 ml $Et_2O$, filtered, and the crystals were washed with ether and recrystallized from alcohol to give white needles of the named Formula (H) compound, mp 207-208°C. Anal. Calcd. for $C_{12}H_{14}Cl\,F\,N_2O$:C, 56.15; H, 5.50; N, 10.91. Found: C, 56.14; H, 5.50; N, 10.90.

(b) 2-(4-Fluorophenyl)-6,7-dihydro-(5H)-pyrrolo [1,2-a]imidazole (Formula (E) Compound)

An aqueous solution of 31g (0.12 mole) of the named Formula (H) compound of Method B, part a above, was heated in 300 ml of water on a steam bath for 8 hours. The solution was adjusted to pH 6.5, and the resulting precipitate was filtered, dried under vacuum and recrystallized from CC14 to give the named Formula (E) compound, mp 137.5-139°C. Anal. Calcd. for $C_{12}H_{11}FN_2$: C, 71.27; H, 5.48; N, 13.85. Found: C, 71.00; H, 5.61; N, 13.73.

EXAMPLE 2

2-(4-Fluorophenyl)-3-(4-pyridyl)-6,7-dihydro-[5H]-pyrrolo[1,2-a]imidazole

A stirred solution of 13.1g (0.065 mole) of 2-(4-fluorophenyl)-6,7-dihydro-[5H]-pyrrolo[1,2-a]imidazole, prepared as described in Example 1, and 51.4g (0.65 mole) of dry pyridine in 17 ml of dry methylene chloride at 22°-25°C was treated over 1.5 hours (hr) with 35.3g (0.325 mmole) of ethyl chloroformate. The solution was stirred at 25°C overnight, and the treatment with pyridine and ethyl chloroformate repeated as before, followed by a 24 hr period of stirring. After 3 more treatments as described above, the solvent was removed in vacuo. The residue was dissolved in 5% aqueous $NaHCO_3$ and extracted into methylene chloride. The organic phase was washed with 5% aqueous $NaHCO_3$ and dried over anhydrous $K_2CO_3$. The volatile solvents were removed in vacuo and the residue extracted into methylene chloride. The organic phase was extracted repeatedly with 0.2M HCl until traces of starting material were removed, then washed with 5% $Na_2CO_3$ solution, dried over $K_2CO_3$ (anhydrous), and striped in vacuo. The residue was crystallized from toluene-hexane to give the compound of Formula (F) known as 3-(N-ethoxycarbonyl-1,4-dihydro-4-pyridyl)-2-(4-fluoro-phenyl)-6,7-dihydro[5H]-pyrrolo(1,2-a]imidazole, 146-147°C.

Method A. 0.5g (1.4 mmoles) of the Formula (F) product described in Example 2 was heated with stirring in 5ml of decalin under argon. Upon reaching a temperature of 80°C, 0.06g (1.8 mmoles) of sulfur was added and the mixture heated to 165°C until starting material was consumed. The cooled mixture was filtered and the solid washed with petroleum ether and dissolved in chloroform-ethyl acetate (1:1). This solution was decolorized with Darco, and chromatographed on silica. Elution with 20% methanol in chloroform-ethyl acetate (1:1) afforded a fraction which was concentrated in vacuo, and recrystallized from

carbon tetrachloride to give the desired Example II title product, mp 163-164.5°C.

Method B. 15.0g (42.4 mmoles) of a Formula (F) compound, i.e., 3-(N-ethoxycarbonyl-1,4-dihydro-4-pyridyl)-2-(4-fluorophenyl)-6,7-dihydro[5H)-pyrrolo[1,2-a]imidazole, prepared as described above, was added to a stirred solution of 28.6g (255 mmoles) of potassium tert.-butoxide dissolved in tert.-butanol (250 ml) into which $O_2$ was being bubbled. The solution was heated to reflux for 15 minutes, and the solvent then removed in vacuo. The solid product was extracted into methylene chloride, washed with water and then extracted into aqueous 3N HCl. This aqueous acidic phase was made basic with cold 10% aqueous sodium hydroxide and extracted with methylene chloride. The resulting organic phase was dried over anhydrous $K_2CO_3$ and the solvent was removed in vacuo. Two recystallizations from toluene gave the Example II title product, mp 165-166°C. Anal. Calcd. for $C_{17}H_{14}FN_3$:C, 73.10; H, 5.05; N, 15.04. Found: C, 73.31; H, 5.11; N, 15.08.

EXAMPLE 3

2-(4-Methylthiophenyl)-3-(4-pyridyl)-6,7-dihydro-[5H]-pyrrolo[1,2-a]imidazole

A stirred solution of 5.5 g (19.7 mmoles) of 2-(4-fluorophenyl)-3-(4-pyridyl)-6,7-dihydro-[5H]-pyrrolo[1,2]-imidazole, prepared as described in Example 2 in 75 ml of dry (sieve) dimethylformamide was treated with 1.65 g (23.6 mmoles) of sodium thiomethylate under argon atmosphere. The reaction mixture was heated overnight at 75°C followed by an additional 2 hours at 95°C, poured into cold water and extracted twice with ethyl acetate. The organic phase was washed three times with water, dried over anhydrous potassium carbonated, and stripped in vacuo. The residue was recrystallized twice from ethyl acetate to afford the titled compound, mp 171-172°C. Anal. Calcd. for $C_{18}H_{17}N_3S$: C, 70.33; H, 5.57; N, 13.67; S, 10.43. Found: C, 69.93; H, 5.40; N, 13.76; S, 10.75.

EXAMPLE 4

2-(4-Methylsulfinylphenyl)-3-(4-pyridyl)-6,7-dihydro-[5H]-pyrrolo[1,2-a]imidazole.

To a stirred solution of 5.0 g (16.3 mmoles) of 2-(4-methylthiophenyl)-3-(4-pyridyl)-6,7-dihydro-[5H]-pyrrolo[1,2-a]imidazole of Example 3 dissolved in 75 ml of chloroform, chilled in an ice bath, was added dropwise a solution of 3.30 g (16.3 mmoles) of 85% 3-chloroperbenzoic acid in chloroform. After stirring at 25°C overnight, the reaction mixture was washed with 5% sodium carbonate, dried over anhydrous potassium carbonate, and stripped in vacuo. The residue was flash chromatographed on silica eluting with 5 to 10% methanol in methylene chloride: 2-propanol (9:1). The solvent was removed in vacuo and the residue recrystallized from ethyl acetate to give the desired titled compound, mp 163.5-165.5°C. $^1H$ NMR (360 MHz, $CDCl_3$) $\delta$ 8.62 (2H,d), 7.68 (2H,d), 7.57 (2H,d), 7.25 (2H,d), 4.05 (2H,t), 3.02 (2H,t), 2.72 (s) superimposed upon 2.69 (m) (5H total). Mass Spec. (CI) (M+H) 324 (MW = 323).

EXAMPLE 5

2-(4-Methylsulfonylphenyl)-3-(4-pyridyl)-6.7-dihydro-[5H]-pyrrolo[1,2-a]imidazole.

A stirred solution of 0.64 g (1.98 mmoles) of 2-(4-methylsulfinylphenyl)-3-(4-pyridyl)-6,7-dihydro-[5H]-pyrrolo[1,2-a]imidazole of Example 4 in water was treated dropwise over 45 minutes with an aqueous solution of 0.209 g (1.32 mmoles) of potassium permanganate. After stirring overnight, the suspension was extracted with methylene chloride. The organic phase was dried over anhydrous potassium carbonate and stripped in vacuo. The residue flash chromatographed in silica eluting with 2 to 4% methanol in chloroform. The solvent was removed in vacuo and the residue recrystallized from ethyl acetate to afford the desired titled compound, mp 222.5-224°C. $^1H$ NMR (250 MHz, $CDCl_3$) $\delta$ 8.62 (2H,d) 7.85 (2H,d), 7.72 (2H,d), 7.26 (2H,d), 4.05 (2H,t), 3.05(s) superimposed upon 3.03(t) (5H total), 2.70 (2H,p).

EXAMPLE 6

2-(4-Methoxyphenyl)-6,7-dihydro-[5H]-pyrrolo-[1,2-a] imidazol-3-yl-tri-n-butyltin

a) 2-(4-Methoxyphenyl)-6,7-dihydro-[5H]-pyrrolo [1,2-a]imidazole (Formula (E) Compound).

To a solution of 6.8 g (29.7 mmoles) of 2-bromo-4′-methoxyacetophenone in 50 ml of $CHCl_3$ was added a solution of 5 g (59.4 mmoles) of 2-iminopyrrolidine in 30 ml of $CHCl_3$ with chilling. After 4 hours of stirring at 25 °C, the solvent was removed in vacuo. The residue was dissolved in water, the pH adjusted to 2.5 and the solution heated on a steam bath under argon atomosphere for 8 hours. The cooled solution was adjusted to pH 6. The resulting precipitate was filtered, washed with water and dried in vacuo to afford the titled compound, mp 116-117.5 °C.

b) 2-(4-Methoxyphenyl)-6,7-dihydro-[5H]-pyrrolo[1,2-a]imidazol-3-yl-tri-n-butyltin

To an ice-cold (0 °C) solution of [16.8 g, 0.078 mol] 2-(4-methoxyphenyl)-6,7-dihydro-[5H]-pyrrolo[1,2-a]imidazole in 200 ml of dry tetrahydrofuran under argon was added dropwise over 20 minutes 35 mL [0.0858 mol] of a 2.5M solution of n-butyl lithium in hexane. Once the addition was complete, the deep-red solution was stirred in the cold for five minutes and then a solution of the tributyltin chloride [26.4 mo, 0.0975 mol] in 50 ml of dry tetrahydrofuran was added over 20 min. The reaction mixture was stirred at ice-bath temperatures for 1.5 hours and then saturated ammonium chloride was added. The layers were shaken together and separated and the organic extract was washed an additional time with saturated ammonium chloride and then dried with anhydrous potassium carbonate. The solvent was removed in vacuo to give 50g of a crude oil, which was taken up twice in cold hexane filtering off the unreacted 2-(4-methoxyphenyl)-6,7-dihydro-[5H]-pyrrolo[1,2-a]imidazole each time. The product was purified on a column of silica, eluting with 1:1 ethyl acetate/hexane in the presence of 1% diethylamine, to give 19.2 g (49% of a yellow oil.
Anal. Calcd for $C_{25}H_{40}Sn\ N_2O$:
C, 59.66, H, 8.01; N, 5.57. Found: C,59.32; H, 8.01, N,5.41.

EXAMPLE 7

2-(4-Methoxyphenyl)-3-(2,6-dimethyl-4-pyridyl)-6-7-dihydro-[5H]-pyrrolo[1,2-a] imidazole

The 4-bromo-2,6-lutidine used in the palladium catalyzed coupling reaction was synthesized from commercially-available 2,6-lutidine N-oxide as described in the literature [J.O.C., 27, 1665 (1962), R. F. Evans and H.C. Brown]. The coupling reaction was carried out as described previously in Example 6.

The product was purified by flash chromatography, on silica, eluting with 20-50% isopropanol in hexane.

NMR($CDCl_3$)δ: 7.4 (d, 2H), 7.2(s,2H)
6.8 (d, 2H), 4.2 (t, 2H),
3.8 (s, 3H), 2.9 (s, 2H)
2.65 (m, 2H), 2.55 (s, 6H)

EXAMPLE 8

2-(4-Ethylthiophenyl)-3-(4-pyridyl)-6,7-dihydro-[5H]-pyrrolo[1,2-a]imidazole

Sodium hydride (60%) (0.75 g, 19 mmol) was added to a solution of ethanethiol (2.1 ml, 1.7 g, 28 mmol) in N,N-dimethylformamide (15 ml) at 0 °C under an argon atmosphere. After stirring for 0.5 hours, 2-(4-fluorophenyl)-3-(4-pyridyl)-6,7-dihydro-[5H]-pyrrolo[1,2-a]imidazole (3.5 g, 12.5 mmol) of Example 2 was added and the resulting solution heated to 95 ° C for 6 hours. The cooled reaction mixture was evaporated under reduced pressure and the residue partitioned between 1N aqueous sodium hydroxide and dichloromethane. The organic layer was washed successively with water and brine, dried (magnesium sulfate) and concentrated. The residue was chromatographed on silica gel eluting with 25:1 chloroform/methanol. Fractions containing product were combined, the solvent evaporated and the residue recrystallized from ethyl acetate to afford the titled compound; mp. 124-125 ° C.

| Anal. Calcd. for | | | | |
|---|---|---|---|---|
| $C_{19}H_{19}N_3S$:<br>Found: | C,70.99;<br>C, 70.99; | H, 5.96;<br>H, 5.92; | N, 13.08;<br>N, 13.07; | S, 9.97:<br>S, 9.81. |

EXAMPLE 9

2-(4-Ethylsulfinylphenyl)-3-(4-pyridyl)-6,7-dihydro-[5H]-pyrrolo[1,2-a]imidazole

The title comopund was prepared from 6,7-dihydro-2-(4-ethylthiophenyl)-3-(4-pyridyl)-[5H]-pyrrolo[1,2-a]imidazole of Example 8 by the procedure described in Example 4. mp. 108-110°. $^1$H NMR (250 MHz, $CDCL_3$)δ 8.61 (2H, d), 7.65 (2H, d), 7.53 (2H, d), 7.24 (2H, d), 4.05 (2H, t), 3.02 (2H, t), 2.86 (2H, m), 2.69 (2H, m), 1.23 (3H, t).

EXAMPLE 10

2-(4-Mercaptophenyl)-3-(4-pyridyl)-6,7-dihydro-[5H]-pyrrolo-[1,2-a]imidazole

To 5 g (15.5 mmole) of 2-(4-methylsulfinylphenyl)-3-(4-pyridyl)-6,7-dihydro-[5H]-pyrrolo[1,2-a]imidazole prepared as in Example 4 dissolved in 100 ml methylene chloride and cooled to 0° was added 9.7 g (46.4 mmole, 6.5 ml) of trifluoroacetic anhydride in 25 ml of methylene chloride. The mixture was heated to reflux for 1 hour. The reaction mixture was stripped on the rotovap, then treated with water, and extracted with methylene chloride. The extract was washed with 3N $NaHCO_3$ and saturated NaCl and treated with $Na_2SO_4$, then stripped to leave 5.1 g of crude product. This material was dissolved in anhydrous methanol (50 ml) and treated with a 25% solution of $NaOCH_3$/MeOH (5 ml, 23 mmole). This mixture was stirred at room temperature for 3 hours, then poured onto ice water and neutralized with 3N $NaHCO_3$. After removing most of the methanol on the rotovap, the residue was partitioned between methylene chloride and water. The organic layer was washed with water and saturated NaCl, treated with $Na_2SO_4$ and stripped. The residue was flash chromatographed on a silica gel column using a gradient of 1 to 5% MeOH in methylene chloride to give 3.1 g (10.5 mmole) of the titled compound.

EXAMPLE 11

2-(4-Trimethylacetylthiophenyl)-3-(4-pyridyl)-6,7-dihydro-[5H]-pyrrolo[1,2-a]imidazole

To 1.0 g (3.4 mmole) of 2-(4-mercaptophenyl)-3-(4-pyridyl)-6,7-dihydro-[5H]-pyrrolo[1,2-a] imidazole prepared as in Example 10 in 50 ml methylene chloride at 0°C was added a solution of 0.3 g (3.7 mmole, 0.26 ml) trimethylacetyl chloride in 10 ml $CH_2Cl_2$ over a period of 10 minutes. The reaction was allowed to come to room temperature and was stirred for 30 minutes. The mixture was then diluted with methylene chloride and washed with 3N $NaHCO_3$, saturated NaCl, treated with $Na_2SO_4$,stripped, then flash chromatographed on silica with methylene chloride containing 1% to 5% MeOH. The isolated material was recrystallized from ethyl acetate to give 0.43 g of the titled compound. 33.5% yield, mp 216-217.5 °C. $C_{22}H_{23}N_3OS$, Calculated, C: 70.00, H: 6.14, N: 11.13; Found, C: 70.01, H: 6.20, N: 10.99.

EXAMPLE 12

2-(4-Acetylthiophenyl)-3-(4-pyridyl)-6,7-dihydro-[5H]-pyrrolo[1,2-a]imidazole

To 1.0 gm (3.4 mmole) of 2-(4-mercaptophenyl)-3-(4-pyridyl)-6,7-dihydro-[5H]-pyrrolo[1,2-a] imidazole prepared as in Example 10 in 50 ml methylene chloride at 0°C was added a solution of 0.3 g (3.7 mmole, 0.26 ml) acetyl chloride in 10 ml $CH_2Cl_2$ over a period of 10 minutes. The reaction was allowed to come to room temperature and was stirred for 30 minutes. The mixture was then diluted with methylene chloride and washed with 3N $NaHCO_3$, saturated NaCl, treated with $Na_2SO_4$,stripped, then flash chromatographed on silica with methylene chloride containing 1% to 5% MeOH. The isolated material was recrystallized twice from ethyl acetate to give 0.20 g of the titled compound. 17.6% yield, mp 152-54°C. $C_{19}H_{17}N_3OS$, Calculated, C: 68.03, H: 5.11, N: 12.53; Found, C: 68.25, H: 5.40, N: 12.14.

EXAMPLE 13

2-[4-(2-Methyl-propenylthio)phenyl]-3-(4-pyridyl)-6,7-dihydro-[5H]-pyrrolo[1,2-a]imidazole.

A solution of 5g (17 mmoles) of 2-(4-mercaptophenyl)-3-(4-pyridyl)-6,7-dihydro-[5H]-pyrrolo[1,2-a)-imidazole in dry tetrahydrofuran is treated at -20°C with a solution of 17 mmoles of lithium diethylamide from 6.8 ml of 2.5M n-butyl lithium. After warming, a solution of 1.57g (17 mmoles) of trimethylsilylmethylchloride in tetrahydrofuran is added dropwise. When the reaction is complete, the mixture is immersed in an ice bath and a second solution (17 mmoles) of lithium diethylamide is added. After stirring for 15 minutes, a solution of 0.99 g (17 mmoles) of acetone in tetrahydrofuran is added, and the mixture is stirred 15 minutes at 0° and 15 minutes at 25°C. The mixture is poured into water, extracted with methylene chloride, and the organic layer dried, and chromatographed on silica to afford the desired titled compound.

EXAMPLE 14

2-(4-Methylsulfinylphenyl)-3-[4-(2-methyl)pyridyl]-6,7-dihydro-[5H]-pyrrolo[1,2-]imidazole

a) 2-(4-Fluorophenyl)-3-[4-(2-methyl-1,2-dihydropyridyl)]-6,7-dihydro-[5H]-pyrrolo[1,2,a]imidazole.

To a solution of 3.3g (11.9 mmole) of 2-(4-fluorophenyl)-3-(4-pyridyl)-6,7-dihydro-[5H]-pyrrolo-[1,2,a]-imidazole in dry tetrahydrofuran at -20°C was added 1.84 g (23.8 mmole) of acetyl chloride. The reaction was stirred at -20°C for 10 minutes and then 8.81 ml of 2.7M methylmagnesium bromide (20 mmol) was added. The reaction was stirred an additional 15 minutes and then warmed to room temperature for 30 minutes. The reaction was quenched with aqueous $NH_4Cl$, adjusted to pH 7.5 with bicarbonate, and extracted repeatedly with methylene chloride. The combined organic extracts were dried over sodium sulfate, filtered, and concentrated in vacuo to afford the crude dihydropyridine.

b) 2-(4-Fluorophenyl)-3-[4-(2-methyl)pyridyl]-6,7-dihydro-[5H]-pyrrolo[1,2,a]imidazole.

The crude dihydropyridine was aromatized by heating at 190°C for 1 hour in a solution composed of 150 ml decalin, 15 ml diglyme, and 1.0 g of sublimed sulfur. The reaction was filtered, diluted with petroleum ether, and chilled. The resulting solid was collected and purified by flash chromatography on silica gel and crystallized from ethyl acetate to afford 2-(4-fluorophenyl)-3-[4-(2-methyl)pyridyl]-6,7-dihydro-[5H]-pyrrolo[1,2,a]imidazole.

c) 2-(4-Methylthiophenyl)-3-[4-(2-methyl)pyridyl]-6,7-dihydro-[5H]-pyrrolo[1,2,a]imidazole.

A stirring solution of 0.55 g of compound b) above and 0.16 g of sodium thiomethylate in 7.5 ml of dry dimethylformamide was heated under an argon atmosphere overnight at 120°C. The reaction was poured into cold water and extracted twice with ethyl acetate. The organic phase was filtered, washed three times with water, dried over anhydrous potassium carbonate, and stripped in vacuo. The residue was recrystallized from ethyl acetate to afford the titled compound. NMR 250MHz ($CDCl_3$)δ: 8.5 (d, 1H), 7.45 (d, 2H), 7.15 (d, 2H), 7.14-7.1 (m, 2H), 4.01 (t, 2H), 3.0 (t, 2H), 2.62 (m, 2H), 2.51 (s, 3H), 2.47 (s, 3H). mp 131-132°C.

d) 2-(4-Methylsulfinylphenyl)-3-[4-(2-methyl)pyridyl]-6,7-dihydro-[5H]-pyrrolo[1,2,a]imidazole.

A solution of 200 mg of compound c) above dissolved in 1.5 ml of water containing 1 ml of 1.2 N HCl was treated dropwise at 5°C over 1.5 hours with a solution of 1.19 mg of sodium periodate in 1.5 ml of water. The reaction mixture was treated as in Example 21 (c) to yield the titled compound. mp 128-131°C NMR 250MHz ($CDCl_3$)δ: 8.5(d, 1H), 7.7(d, 2H), 7.55(d, 2H), 7.15-7.05(m, 2H), 4.05(t, 2H), 3.05(t, 2H), 2.75(s, 3H), 2.68(m, 2H).
In an analogous manner to the process of Example 14(a) and (b), 2-(4-methoxyphenyl)-3-[4-(2-methyl)-pyridyl]-6,7-dihydro-[5H]-pyrrolo[1,2-a]imidazole was prepared. mp 158-60°C.

EXAMPLE 15

2-(4-Carbethoxymethylthiophenyl)-3-(4-pyridyl)-6,7-dihydro-[5H]-pyrrolo[1,2-a]imidazole

To 1.0 gm (3.4 mmole) of 2-(4-mercaptophenyl)-3-(4-pyridyl)-6,7-dihydro-[5H]-pyrrolo[1,2-a]imidazole in 50 ml methylene chloride at 0° was added a solution of 0.63 g (3.7 mmole, 0.43 ml) ethyl bromoacetate in 10 ml $CH_2Cl_2$ over a period of 10 minutes. The reaction was allowed to come to room temperature and was stirred for 30 minutes. The mixture was then diluted with methylene chloride and washed with 3N $NaHCO_3$, saturated NaCl, treated with $Na_2SO_4$, stripped, then flash chromatographed on silica with methylene chloride containing 1% to 5% MeOH. The isolated material was recrystallized from ethyl acetate to give 0.35g of the titled product. 27.2% yield, mp 102-103°C. Analyzed for $C_{21}H_{21}N_3O_2S$, Calculated, C: 66.47, H:5.58, N: 11.07; Found, C: 66.39, H: 5.62, N: 10.97.

EXAMPLE 16

2-(4-Acetoxymethylthiophenyl)-3-(4-pyridyl)-6,7-dihydro-[5H]-pyrrolo[1,2-a]imidazole

To 1 g (3.1 mmole) of 2-(4-methylsulfinylphenyl)-3-(4-pyridyl)-6,7-dihydro-[5H]-pyrrolo[1,2-a]imidazole was added 25 ml acetic anhydride. The mixture was heated to reflux for 1 hour. The reaction mixture was stripped on the rotovap, then treated with water, and extracted with methylene chloride. The extract was washed with 3N $NaHCO_3$ and saturated NaCl and treated with $Na_2SO_4$,then stripped to leave 1.1 g of crude product. This crude material was then flash chromatographed on a silica gel column using a gradient of 1 to 5% MeOH in methylene chloride to give after recrystallization from EtOAc 0.80 g (2.2 mmole) of the titled product. 71% yield, mp 125.5-126.5°C. Analyzed for $C_{20}H_{19}N_3O_2S$, Calculated, C: 65.73, H:5.24, N: 11.50; Found, C: 66.03, H: 5.26, N: 11.30.

EXAMPLE 17 CAPSULE COMPOSITION

A pharamceutical composition of this invention in the form of a capsule is prepared by filling a standard two-piece hard gelatin capsule with 50 mg. of a compound of Formula (I), in powdered form, 110 mg. of lactose, 32 mg. of talc and 8 mg. of magnesium stearate.

EXAMPLE 18 - INJECTABLE PARENTERAL COMPOSITION

A pharmaceutical composition of this invention in a form suitable for administration by injection is prepared by stirring 1.5% by weight of a compound of Formula (I) in 10% by volume propylene glycol and water. The solution is sterilized by filtration.

EXAMPLE 19 - OINTMENT COMPOSITION

Compound of Formula (I) 1.0 g
White soft paraffin to 100.0 g

The compound of Formula (I) is dispersed in a small volume of the vehicle and this dispersion is gradually incorporated into the bulk to produce a smooth, homogeneous product which is filled into collapsible metal tubes.

EXAMPLE 20 - TOPICAL CREAM COMPOSITION

Compound of Formula (I) 1.0 g
Polawax GP 200 20.0 g
Lanolin Anhydrous 2.0 g
White Beeswax 2.5 g
Methyl hydroxybenzoate 0.1 g
Distilled Water to 100.0 g

The polawax, beeswax and lanolin are heated together at 60°C and added to a solution of methyl hydroxybenzoate. Homogenization is achieved using high speed stirring and the temperature is allowed to fall to 50°C. The compound of Formula (I) is added and dispersed throughout, and the composition is allowed to cool with slow speed stirring.

EXAMPLE 21 - TOPICAL LOTION COMPOSITION

Compound of Formula (I) 1.0 g
Sorbitan Monolaurate 0.6 g
Polysorbate 20 0.6 g
Cetostearyl Alcohol 1.2 g
Glycerin 6.0 g
Methyl Hydroxybenzoate 0.2 g
Purified Water B.P. to 100.00 ml

The methyl hydroxybenzoate and glycerin are dissolved in 70 ml of the water at 75°. The sorbitan monolaurate, polysorbate 20 and cetostearyl alcohol are melted together at 75°C and added to the aqueous solution. The resulting emulsion is homogenized, allowed to cool with continuous stirring and the compound of Formula (I) is added as a suspension in the remaining water. The whole suspension is stirred until homogenized.

EXAMPLE 22 - EYE DROP COMPOSITION

Compound of Formula (I) 0.5 g
Methyl Hydroxybenzoate 0.01 g
Propyl Hydroxybenzoate 0.04 g
Purified Water B.P. to 100.00 ml

The methyl and propyl hydroxybenzoates are dissolved in 70 ml purified water at 75°C and the resulting solution is allowed to cool. The compound of Formula (I) is then added, and the solution is made up to 100 ml with purified water. The solution is sterilized by filtration through a membrane filter (0.22 mu m pore size) and packed aseptically into suitable sterile containers.

EXAMPLE 23 - COMPOSITION FOR ADMINISTRATION BY INHALATION

For an aerosol container with a capacity of 15-20 ml: Mix 10 mg of a compound of Formula (I) with 0.1-0.2% of a lubricating agent, such as Span 85 or oleic acid, and disperse such mixture in a propellant (c.a.), such as freon, preferably a combination of freon 114 and freon 12, and put into an appropriate aerosol container adapted for either intranasal or oral inhalation administration.

EXAMPLE 24 - COMPOSITION FOR ADMINISTRATION BY INHALATION

For an aerosol container with a capacity of 15-20 ml: Dissolve 10 mg of a compound of Formula (I) in ethanol (6-8 ml), add 0.1-0.2% of a lubricating agent, such as Span 85 or oleic acid, and disperse such in a propellant (c.a.), such as freon, preferably a combintion of freon 144 and freon 12, and put into an appropriate aerosol container adapted for either intranasal or oral inhalation administration.

**Claims**

**Claims for the following Contracting States : AT, BE, CH, DE, FR, GB, IT, LI, LU, NL, SE,**

**1.** A compound of the formula

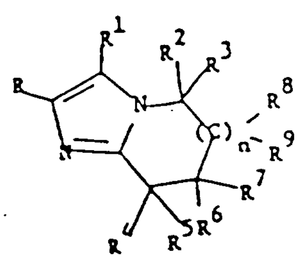

FORMULA (I)

wherein

1) One of R or $R^1$ must be alkyl substituted pyridyl and the other is selected from:

monosubstituted phenyl wherein said substituent is selected from H, halo, hydroxy $C_{1-3}$ alkoxy, $C_{1-3}$ alkylthio, $C_{1-4}$ alkyl,$C_{1-3}$ alkylsulfinyl, $C_{1-3}$ alkylsulfonyl, $C_{1-3}$ alkylamino, $C_{1-3}$ dialkylamino, $CF_3$, N-($C_{1-3}$ alkyl)-N-($C_{1-3}$alkanamido), N-pyrrolidino, N-piperidino, prop-2-ene-1-oxy or 2,2,2-trihaloethoxy; or

2) $R^1$ is 4-pyridyl and R selected from:

monosubstituted phenyl wherein said substituent is selected from $C_{1-3}$ alkylthio, $C_{1-3}$ alkylsulfinyl, $C_{1-3}$ alkylsulfonyl, or a branched or unbranched $C_{2-5}$alkenylthio or $C_{2-5}$alkenylsulfinyl; or

3) One of $R^1$ or R is pyridyl or alkyl substituted pyridyl and the other is selected from monosubstituted or disubstituted phenyl wherein said substituent is selected from thiol [HS-], acylthio [AC(O)-S-], dithioacyl [AC(S)S-], dialkylthiocarbamyl [$A_2$NC(O)S-], dialkyldithiocarbamyl [$A_2$NC(S)S-], alkylcarbonylalkylthio [AC(O)$CH_2$S-], carbalkoxyalkylthio [AOC(O)$CH_2$S-], or acyloxyalkylthio [AC(O)-O$CH_2$S-] wherein the $CH_2$ is optionally substituted with $C_{1-4}$alkyl, and A is $C_{1-9}$alkyl;

and $R^2$, $R^3$, $R^4$, $R^5$, $R^6$, $R^7$, $R^8$ and $R^9$ are H, or one or two of $R^2$, $R^3$, $R^3$, $R^4$, $R^5$, $R^6$, $R^7$, $R^8$ and $R^9$ are independently selected from H or $C_{1-2}$ alkyl; n is 0 or 1;

or a pharmaceutically acceptable salt thereof.

**2.** The compound of Claim 1 which is 2-(4-methylthiophenyl)-3-(4-pyridyl)-6,7-dihydro-[5H]-pyrrolo[1,2-a]-imidazole.

**3.** The compound of Claim 1 which is 2-(4-methylsulfinylphenyl)-3-(4-pyridyl)-6,7-dihydro-[5H]-pyrrolo[1,2-a]imidazole.

**4.** The compound of Claim 1 which is: 2-(4-methylsulfonylphenyl)-3-(4-pyridyl)-6,7-dihydro-[5H]-pyrrolo-[1,2-a]imidazole,

2-(4-methoxyphenyl)-3-(2,6-dimethyl-4-pyridyl)-6,7-dihydro-[5H]-pyrrolo[1,2-a]imidazole,
2-(4-ethylsulfinylphenyl)-3-(4-pyridyl)-6,7-dihydro-[5H]-pyrrolo[1,2-a]imidazole,
2-(4-ethylthiophenyl)-3-(4-pyridyl)-6,7-dihydro-[5H]-pyrrolo[1,2-a]imidazole,
2-(4-mercaptophenyl)-3-(4-pyridyl)-6,7-dihydro-[5H]-pyrrolo[1,2-a]imidazole,
2-(4-trimethylacetylthiophenyl)-3-(4-pyridyl)-6,7-dihydro-[5H]-pyrrolo[1,2-a]imidazole,
2-(4-acetylthiophenyl)-3-(4-pyridyl)-6,7-dihydro-[5H]-pyrrolo[1,2-a]imidazole,
2-(4-ethylsulfonylphenyl)-3-(4-pyridyl)-6,7-dihydro-[5H]-pyrrolo[1,2-a]imidazole,
2-(4-fluorophenyl)-3-[4-(2-methyl)pyridyl]-6,7-dihydro-[5H]-pyrrolo[1,2-a]imidazole,
2-(4-methylthiophenyl)-3-[4-(2-methyl)pyridyl]-6,7-dihydro-[5H]-pyrrolo[1,2-a]imidazole,
2-(4-methylsulfinylphenyl)-3-[4-(2-methyl)pyridyl]-6,7-dihydro-[5H]-pyrrolo[1,2-a]imidazole,
2-(4-carbethoxymethylthiophenyl)-3-(4-pyridyl)-6,7-dihydro-[5H]-pyrrolo[1,2-a]imidazole, or
2-(4-acetoxymethylthiophenyl)-3-(4-pyridyl)-6,7-dihydro-[5H]-pyrrolo[1,2-a]imidazole.

**5.** A pharmaceutical composition comprising a pharmaceutically acceptable carrier or diluent and a compound as defined in any one of Claims 1 to 4.

**6.** The composition of Claim 5 wherein the composition is in dosage unit form adapted for parenteral administration and which comprises from 50 mg to 500 mg of the active compound.

**7.** The composition of Claim 5 wherein the composition is in dosage unit form adapted for oral administration and which comprises from 100 mg to 1000 mg of the active compound.

**8.** The composition of Claim 5 wherein the composition is in a dosage unit form adapted for administration by inhalation or topical administration.

**9.** A compound of the formula (I) as defined in any one of Claims 1 to 4 for use in the treatment of a 5-lipoxygenase pathway mediated disease in an animal.

**10.** A compound of the formula (I), as defined in any one of Claims 1 to 4, for use in the treatment or prophylaxis of an inflammatory condition in an animal, or pyresis, pain and other conditions associated with inflammation.

11. A compound of the formula (I) for use as defined in Claim 10 wherein the inflammatory condition is selected from rheumatoid arthritis, rheumatoid spondylitis, osteoarthritis, gouty arthritis and other arthritic conditions, inflamed joints, eczema, psoriasis or other inflammatory skin conditions such as sunburn; and inflammatory eye conditions such as conjunctivitis.

12. A compound of the formula

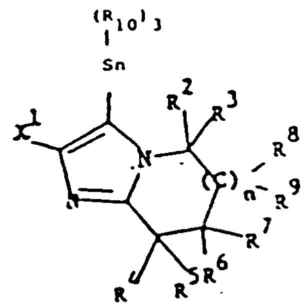

FORMULA (J)

wherein
n is 0 or 1;
$R^2$, $R^3$, $R^4$, $R^5$, $R^6$, $R^7$, $R^8$ and $R^9$ are H, or one or two of $R^2$, $R^3$, $R^4$, $R^5$, $R^6$, $R^7$, $R^8$ and $R^9$ are independently selected from H or $C_{1-2}$ alkyl;
$R_{10}$ is $C_{1-4}$ alkyl;
and $X^1$ is selected from
(a) monosubstituted phenyl wherein said substituent is selected from H, fluoro, chloro, $C_{1-3}$ alkoxy, $C_{1-4}$ alkyl, $C_{1-3}$ dialkylamino, $CF_3$, $C_{1-3}$ alkylamino, N-pyrrolidino, N-piperidino, prop-2-ene-1-oxy or 2,2,2-trihaloethoxy;
(e) pyridyl or alkyl substituted pyridyl; or a pharmaceutically acceptable salt thereof.

13. The compound of Claim 12 which is 2-(4-methoxyphenyl)-6,7-dihydro-[5H]-pyrrolo[1,2-a]imidazol-3-yl-tri-n-butyltin.

14. A compound of the formula

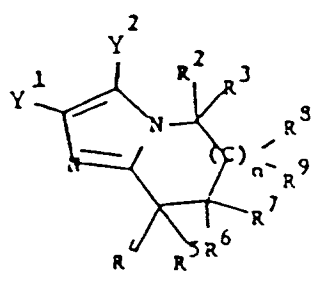

FORMULA (L)

wherein:
n is 0 or 1,
$R^2$, $R^3$, $R^4$, $R^5$, $R^6$, $R^7$, $R^8$, $R^9$ are all H, or one or two of $R^2$, $R^3$, $R^4$, $R^5$, $R^6$, $R^7$, $R^8$ and $R^9$, are independently selected from H or $C_{1-2}$ alkyl; one of $Y^1$ or $Y^2$ is independently selected from 4-[1,2-dihydro-2-($C_{1-4}$-alkyl]pyridyl substituted with N-($C_{1-8}$ alkanoyl), N-($C_{1-8}$ alkoxycarbonyl), N-(benzoyl), N-(phenoxycarbonyl), N-(phenylacetyl), or N-(benzyloxycarbonyl);

and the other is selected from

(a) monosubstituted phenyl wherein said substituent is selected from H, halo, $C_{1-3}$ alkoxy, $C_{1-3}$ alkylthio, $C_{1-4}$ alkyl, N-($C_{1-3}$ alkyl)-N-($C_{1-3}$ alkanamido), $C_{1-3}$ dialkylamino, $CF_3$, N-pyrrolidino, N-piperidino, prop-2-ene-1-oxy or 2,2,2-trihaloethoxy;

or a pharmaceutically acceptable salt thereof.

**15.** A process for the preparation of compound of the formula (I) as defined in claim 1 or a pharmaceutically acceptable salt thereof, which comprises

A. when it is required to prepare a compound of Formula (I) wherein $R^1$ is 4-pyridyl, and R is other than pyridyl, reacting a compound of the Formula (E) represented by the structure:

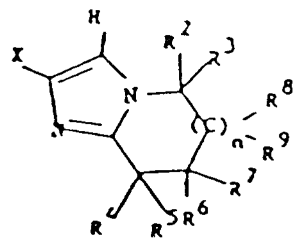

FORMULA (E)

wherein

n is 0 or 1;

$R^2$, $R^3$, $R^4$, $R^5$, $R^6$, $R^7$, $R^8$ and $R^9$ are H, or one or two of $R^2$, $R^3$, $R^4$, $R^5$, $R^6$, $R^7$, $R^8$ and $R^9$ are independently selected from H or $C_{1-2}$alkyl;

X is $C_{1-3}$alkylthiophenyl in pyridine with an aroyl halide, an arylalkyl haloformate ester, or an alkyl haloformate ester, or with the preformed acyl pyridinium salt to yield a compound of formula (F) represented by the structure:

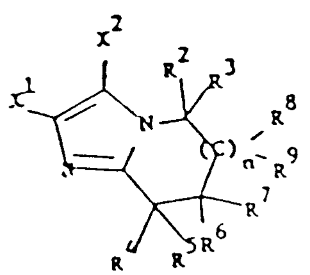

FORMULA (F)

wherein

n is 0 or 1

$R^2$, $R^3$, $R^4$, $R^5$, $R^6$, $R^7$, $R^8$ and $R^9$ are H, or one or two of $R^2$, $R^3$, $R^4$, $R^5$, $R^6$, $R^7$, $R^8$ and $R^9$ are independently selected from H or $C_{1-2}$ alkyl;

$X^1$ is phenyl monosubstituted by $C_{1-3}$alkylthio, and

$X_2$ is 4-(1,4-dihydro)pyridyl substituted with N-($C_{1-8}$alkanoyl), N-($C_{1-8}$alkoxycarbonyl), N-(benzoyl), N-(phenoxycarbonyl), N-(phenylacetyl) or N-(benzyloxycarbonyl); followed by deacylation/oxidation of a compound of Formula (F) to yield a compound of Formula (I); or

B. when it is required to prepare a hydroxy compound of Formulae (I) or (E), demethylating a methoxyphenyl compound of Formulae (E), or (I), wherein Formula (E) is as defined above except that X is methoxyphenyl, Formula (I) is represented by the structure:

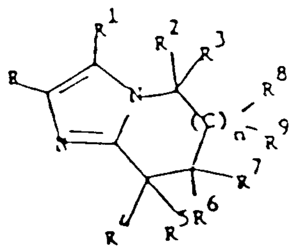

FORMULA (I)

wherein

n is 0 or 1;

$R^2$, $R^3$, $R^4$, $R^5$, $R^6$, $R^7$, $R^8$, and $R^9$ and H or one or two of $R^2$, $R^3$, $R^4$, $R^5$, $R^6$, $R^7$, $R^8$ and $R^9$ are independently selected from H or $C_{1-2}$ alkyl;

One of $R^1$ and R is alkyl substituted pyridyl and the other is methoxyphenyl or a pharmaceutically acceptable salt thereof;

with HBr in acetic acid or $BBr_3$ in methylene chloride to give the corresponding hydroxy compound of Formulae (I), or (E); or

C. O-alkylating the hydroxy compound of Formula (I), or (E), as defined above when one of $R^1$ and R for (I), or X for (E) is a phenyl group substituted with at least one hydroxy, to give the corresponding $C_{1-3}$ alkoxyphenyl, 2,2,2-trihaloethoxyphenyl of prop-2-ene-1-oxy-phenyl substituted compound of Formulae (I) or (E); or

D. acylating an aminophenyl or ($C_{1-3}$ alkylamino)phenyl compound of Formula (I) with the correspondingly substituted acyl halide or anhydride in pyridine to give the corresponding N-($C_{1-3}$ alkyl)-($C_{1-3}$ alkanamido)phenyl compound of Formula (I); or

E. alkylating a ($C_{1-3}$ alkanamido)phenyl compound of Formulae (I) or (E) as previously defined with an alkylating agent in the presence of a base to give the corresponding N-($C_{1-3}$ alkanamido)phenyl or N-($C_{1-3}$ alkyl)-($C_{1-3}$ alkanamido)phenyl compound of Formulae (I), or (E); or

F. hydrolyzing a ($C_{1-3}$ alkanamido)phenyl or N-($C_{1-3}$ alkyl)-($C_{1-3}$ alkanamido)phenyl compound of Formulae (I), or (E) as previously defined or of Formula (G) represented by the structure:

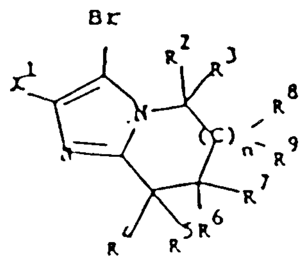

FORMULA (G)

wherein:

n is 0 or 1;

$R^2$, $R^3$, $R^4$, $R^5$, $R^6$, $R^7$, $R^8$, $R^9$ are H; or one or two of $R^2$, $R^3$, $R^4$, $R^5$, $R^6$, $R^7$, $R^8$ and $R^9$ are independently selected from H or $C_{1-2}$ alkyl;

$X^1$ is ($C_{1-3}$alkanamido)phenyl or N-($C_{1-3}$alkyl)-($C_{1-3}$alkanamido)phenyl to give the corresponding aminophenyl or ($C_{1-3}$ alkylamino)phenyl compound of Formulae (I) (E) or G; or

G. oxidizing a compound of Formula (I) when one of $R^1$ and R is phenyl substituted by one or more $C_{1-3}$ alkylthio, $C_{1-3}$ alkylsulfinyl, $C_{1-3}$ alkenylthio, or acyloxyalkylthio with an oxidizing agent to give the corresponding $C_{1-3}$ alkylsulfinyl, $C_{1-3}$ alkylsulfonyl, acyloxyalkylsulfinyl or $C_{1-3}$ alkenylsulfinyl compound of Formula (I); or

H. reducing of a compound of Formulae (E) or (I) as previously defined, wherein X of Formula (E), or one of R and $R^1$ of Formula (I), is mono- or disubstituted phenyl containing one nitro group, with hydrogen gas on a catalyst to give the corresponding aminophenyl compound of Formulae (I) or (E); or

I. cycloalkylating a compound of Formulae (I), or (E) as previously defined wherein one of $R^1$ and R of Formula (I), or X of Formula (E), is aminophenyl, with dihalobutane or dihalopentane as appropriate in the presence of a base to give the corresponding N-piperidino or N-pyrrolidino compound of Formula (I), or (E); or

J. when it is required to prepare compounds of Formulae (I) or (E) as previously defined, wherein $R^1$ and R of Formula (I), or X of Formula (E), is ($C_{1-3}$ alkylamino)phenyl, reducing the corresponding $C_{1-3}$ alkanamido compounds with borane or borane dimethylsulfide complex in tetrahydrofuran to yield the desired compounds of Formulae (I) or (E); or

K. when it is required to prepare compounds of Formulae (I) or (E) as previously defined wherein one of $R^1$ and R of Formula (I), or X of Formula (E), is ($C_{1-3}$ dialkylamino)phenyl, reducing the corresponding N-($C_{1-3}$ alkyl)($C_{1-3}$alkanamido) compounds with borane or borane dimethylsulfide complex in tetrahydrofuran to yield the desired compounds of Formulae (I) or (E); or

L.

(i) reacting a compound of Formulae (E) or (G) with a $C_{1-5}$ alkyllithium reagent to yield the corresponding lithium reagent by metallation or lithium-halogen interexchange respectively;

(ii) adding excess magnesium halide etherate to the lithium reagent to yield the corresponding Grignard reagent by transmetallation;

(iii) adding the Grignard reagent to an N-acylpyridium salt to yield the corresponding compound of Formula (F) as defined above;

(iv) deacylating/oxidizing the compound of Formula (F) to yield the corresponding compound of Formula (I); or

M. treating the 3-lithio derivative of a compound of Formulae (E) or (G), each as previously defined, with trialkyltin chloride to yield a compound of Formula (J) having the following formula

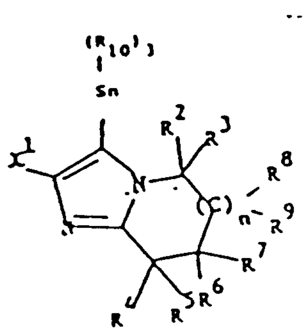

FORMULA J

wherein

n is 0 or 1;

$R^2$, $R^3$, $R^4$, $R^5$, $R^6$, $R^7$, $R^8$ and $R^9$ are H, or one or two of $R^2$, $R^3$, $R^4$, $R^5$, $R^6$, $R^7$, $R^8$ and $R^9$ are independently selected from H or $C_{1-2}$ alkyl;

$R_{10}$ is $C_{1-4}$ alkyl;

and $X^1$ is selected from

(a) monosubstituted phenyl wherein said substituent is selected from H, fluoro, chloro, $C_{1-3}$ alkoxy, $C_{1-4}$ alkyl, $C_{1-3}$ dialkylamino, $CF_3$, $C_{1-3}$ alkylamino, N-pyrrolidino, N-piperidino, prop-2-ene-1-oxy or 2,2,2-trihaloethoxy; or

(e) pyridyl or alkyl substituted pyridyl;

and reacting the Formula (J) compound with a mixture of an aryl or heteroaryl halide or triflate and tetrakis(triphosphine)palladium in a mixture of tetrahydrofuran and hexamethylphosphoramide to yield a compound of Formula (I); or

N. when it is required to prepare a compound of Formula (I) wherein one of R or $R^1$ is an substituted phenyl containing one or more alkylthio groups, treating the corresponding fluoro substituted phenyl

compound of Formula (I) with a metal salt of the alkylmercaptan in an aprotic polar solvent to yield the desired compound of Formula (I); or

O. when it is required to prepare a compound of Formula (I) wherein one of R or $R^1$ is phenyl substituted with acyloxyalkylthio wherein the alkyl is optionally substituted with $C_{1-4}$ alkyl, treating a compound of Formula (I) wherein $R^1$ is phenyl substituted by at least one alkylsulfinyl with an alkanoic acid anhydride to yield the desired compound of Formula (I); or

P. when it is required to prepare a compound of Formula (I) wherein one of $R^1$ or R is phenyl substituted with at least one sulfhydryl group, hydrolyzing the product of process O as described above to yield the desired compounds of Formula (I); or

Q. when it is required to prepare a compound of Formula (I) wherein one of $R^1$ or R is phenyl substituted with at least one acylthio, dithioacyl, thiocarbamyl or dithiocarbamyl group, treating the product of process P above with an acyl halide, alkanoic acid anhydride, thioacyl halide, dithioalkanoic acid anhydride, dialkylcarbamyl halide, or dialkylthiocarbamyl halide in the presence of a base such as pyridine to yield the desired compounds of Formula (I); or

R. when it is required to prepare a compound of Formula (I), wherein $R^1$ or R is phenyl substituted with an alkenylthio group, alkylating a compound of Formula (I) wherein one of $R^1$ or R is phenyl substituted by at least one sulfhydryl group with an appropriately substituted alkenylhalide, such as allylbromide, to yield compounds of Formula (I) wherein $R^1$ or R is phenyl substituted by at least one alkenylthio group; or

S. when it is required to prepare a compound of Formula (I) wherein $R^1$ or R is phenyl substituted with an alkenylthio group, wherein the sulfur is attached to the carbon bearing the double bond,

i) treating the mercapto product of process P above with a strong base to yield the corresponding metal mercaptide salt compound,

ii) treating the metal mercaptide salt compound with trialkylsilylmethylchloride to yield a compound of Formula (I) wherein the phenyl has at least one trialkylsilylmethylsulfide substituent,

iii) treating the trialkylsilylmethylsulfide substituted compound in an aprotic solvent with a lithiating reagent followed by the appropriate aliphatic aldehyde or ketone to yield a compound of Formula (I) wherein R or $R^1$ is phenyl substituted by at least one alkenylthio group; or

T. when it is required to prepare a compound of Formula (I) wherein R or $R^1$ is alkyl substituted pyridyl, deacylating and aromatizing a compound of Formula (L)

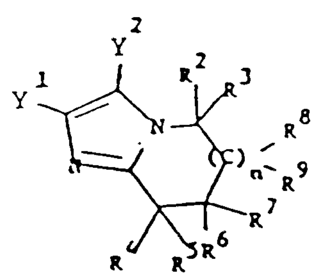

FORMULA (L)

wherein:

n is 0 or 1,

$R^2$, $R^3$, $R^4$, $R^5$, $R^6$, $R^7$, $R^8$, $R^9$ are all H, or one or two of $R^2$, $R^3$, $R^4$, $R^5$, $R^6$, $R^7$, $R^8$ and $R^9$, are independently selected from H or $C_{1-2}$ alkyl;

One of $Y^1$ or $Y^2$ is independently selected from 4-[1,2-dihydro-2-($C_{1-4}$alkyl)]pyridyl substituted with N-($C_{1-8}$ alkanoyl), N-($C_{1-8}$ alkoxycarbonyl), N-(benzoyl), N-(phenoxycarbonyl), N-(phenylacetyl), or N-(benzyloxycarbonyl);

and the other is selected from

(a) monosubstituted phenyl wherein said substituent is selected from H, halo, $C_{1-3}$ alkoxy, $C_{1-3}$ alkylthio, $C_{1-4}$ alkyl, N-($C_{1-3}$ alkyl)-N-($C_{1-3}$ alkanamido), $C_{1-3}$dialkylamino, $CF_3$, N-pyrrolidino, N-piperidino, prop-2-ene-1-oxy or 2,2,2-trihaloethoxy;

or a pharmaceutically acceptable salt thereof, with sulfur in refluxing decalin, tetralin, p-cymene or xylene with oxygen gas for 15 minutes to afford the corresponding compound of Formula (I).

**Claims for the following Contracting State : ES**

**1.** A process for the preparation of compound of the formula

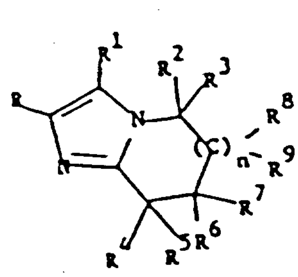

FORMULA (I)

wherein

1) One of R or $R^1$ must be alkyl substituted pyridyl and the other is selected from:

monosubstituted phenyl wherein said substituent is selected from H, halo, hydroxy $C_{1-3}$ alkoxy, $C_{1-3}$ alkylthio, $C_{1-4}$ alkyl,$C_{1-3}$ alkylsulfinyl, $C_{1-3}$ alkylsulfonyl, $C_{1-3}$ alkylamino, $C_{1-3}$ dialkylamino, $CF_3$, N-($C_{1-3}$ alkyl)-N-($C_{1-3}$alkanamido), N-pyrrolidino, N-piperidino, prop-2-ene-1-oxy or 2,2,2-trihaloethoxy;

2) $R^1$ is 4-pyridyl and R selected from:

monosubstituted phenyl wherein said substituent is selected from $C_{1-3}$ alkylthio, $C_{1-3}$ alkylsulfinyl, $C_{1-3}$ alkylsulfonyl, or a branched or unbranched $C_{2-5}$ alkenylthio or $C_{2-5}$ alkenylsulfinyl; or

3) One of $R^1$ or R is pyridyl or alkyl substituted pyridyl and the other is selected from monosubstituted or disubstituted phenyl wherein said substituent is selected from thiol [HS-], acylthio [AC(O)-S-], dithioacyl [AC(S)S-], dialkylthiocarbamyl [$A_2$NC(O)S-], dialkyldithiocarbamyl [$A_2$NC(S)S-], alkylcarbonylalkylthio [AC(O)$CH_2$S-], carbalkoxyalkylthio [AOC(O)$CH_2$S-], or acyloxyalkylthio [AC(O)-O$CH_2$S-] wherein the $CH_2$ is optionally substituted with $C_{1-4}$alkyl, and A is $C_{1-9}$alkyl;

and $R^2$, $R^3$, $R^4$, $R^5$, $R^6$, $R^7$, $R^8$ and $R^9$ are H, or one or two of $R^2$, $R^3$, $R^3$, $R^4$, $R^5$, $R^6$, $R^7$, $R^8$ and $R^9$ are independently selected from H or $C_{1-2}$ alkyl; and n is 0 or 1;

or a pharmaceutically acceptable salt thereof, which comprises

A. when it is required to prepare a compound of Formula (I) wherein $R^1$ is 4-pyridyl, and R is other than pyridyl, reacting a compound of the Formula (E) represented by the structure:

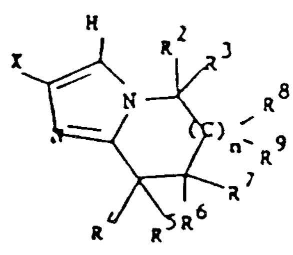

FORMULA (E)

wherein

n is 0 or 1;

$R^2$, $R^3$, $R^4$, $R^5$, $R^6$, $R^7$, $R^8$ and $R^9$ are H, or one or two of $R^2$, $R^3$, $R^4$, $R^5$, $R^6$, $R^7$, $R^8$ and $R^9$ are independently selected from H or $C_{1-2}$alkyl;

X is $C_{1-3}$alkylthiophenyl in pyridine with an aroyl halide, an arylalkyl haloformate ester, or an alkyl haloformate ester, or with the preformed acyl pyridinium salt to yield a compound of formula (F) represented by the structure:

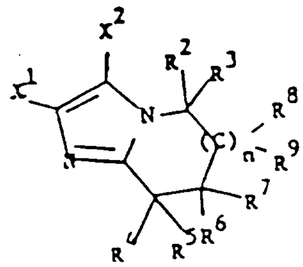

FORMULA (F)

wherein

n is 0 or 1

$R^2$, $R^3$, $R^4$, $R^5$, $R^6$, $R^7$, $R^8$ and $R^9$ are H, or one or two of $R^2$, $R^3$, $R^4$, $R^5$, $R^6$, $R^7$, $R^8$ and $R^9$ are independently selected from H or $C_{1-2}$ alkyl;

$X^1$ is phenyl monosubstituted by $C_{1-3}$alkylthio, and

$X_2$ is 4-(1,4-dihydro)pyridyl substituted with N-($C_{1-8}$alkanoyl), N-($C_{1-8}$alkoxycarbonyl), N-(benzoyl), N-(phenoxycarbonyl), N-(phenylacetyl) or N-(benzyloxycarbonyl); followed by deacylation/oxidation of a compound of Formula (F) to yield a compound of Formula (I); or

B. when it is required to prepare a hydroxy compound of Formulae (I) or (E), demethylating a methoxyphenyl compound of Formulae (E), or (I), wherein Formula (E) is as defined above except that X is methoxyphenyl, Formula (I) is represented by the structure:

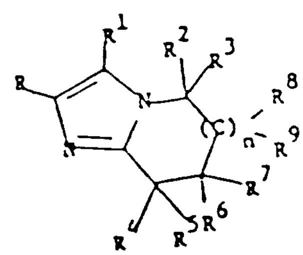

FORMULA (I)

wherein

n is 0 or 1;

$R^2$, $R^3$, $R^4$, $R^5$, $R^6$, $R^7$, $R^8$, and $R^9$ and H or one or two of $R^2$, $R^3$, $R^4$, $R^5$, $R^6$, $R^7$, $R^8$ and $R^9$ are independently selected from H or $C_{1-2}$ alkyl;

One of $R^1$ and R is alkyl substituted pyridyl and the other is methoxyphenyl or a pharmaceutically acceptable salt thereof;

with HBr in acetic acid or $BBr_3$ in methylene chloride to give the corresponding hydroxy compound of Formulae (I), or (E); or

C. O-alkylating the hydroxy compound of Formula (I), or (E), as defined above when one of $R^1$ and R for (I), or X for (E) is a phenyl group substituted with at least one hydroxy, to give the corresponding $C_{1-3}$ alkoxyphenyl, 2,2,2-trihaloethoxyphenyl or prop-2-ene-1-oxy-phenyl substituted compound of Formulae (I) or (E); or

D. acylating an aminophenyl or ($C_{1-3}$ alkylamino)phenyl compound of Formula (I) with the correspondingly substituted acyl halide or anhydride in pyridine to give the corresponding N-($C_{1-3}$ alkyl)-($C_{1-3}$ alkanamido)phenyl compound of Formula (I); or

E. alkylating a ($C_{1-3}$ alkanamido)phenyl compound of Formulae (I) or (E) as previously defined with an alkylating agent in the presence of a base to give the corresponding N-($C_{1-3}$ alkanamido)phenyl or N-($C_{1-3}$ alkyl)-($C_{1-3}$ alkanamido)phenyl compound of Formulae (I), or (E); or

F. hydrolyzing a ($C_{1-3}$ alkanamido)phenyl or N-($C_{1-3}$ alkyl)-($C_{1-3}$ alkanamido)phenyl compound of Formulae (I), or (E) as previously defined or of Formula (G) represented by the structure:

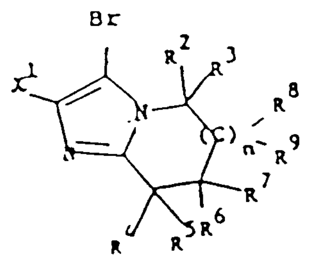

FORMULA (G)

wherein:

n is 0 or 1;

$R^2$, $R^3$, $R^4$, $R^5$, $R^6$, $R^7$, $R^8$, $R^9$ are H; or one or two of $R^2$, $R^3$, $R^4$, $R^5$, $R^6$, $R^7$, $R^8$ and $R^9$ are independently selected from H or $C_{1-2}$ alkyl;

$X^1$ is ($C_{1-3}$alkanamido)phenyl or N-($C_{1-3}$alkyl)-($C_{1-3}$alkanamido)phenyl to give the corresponding aminophenyl or ($C_{1-3}$ alkylamino)phenyl compound of Formulae (I), (E) or (G); or

G. oxidizing a compound of Formula (I) when one of $R^1$ and R is phenyl substituted by one or more $C_{1-3}$ alkylthio, $C_{1-3}$ alkylsulfinyl, $C_{1-3}$ alkenylthio, or acyloxyalkylthio with an oxidizing agent to give the corresponding $C_{1-3}$ alkylsulfinyl, $C_{1-3}$ alkylsulfonyl, acyloxyalkylsulfinyl or $C_{1-3}$ alkenylsulfinyl compound of Formula (I); or

H. reducing of a compound of Formulae (E) or (I) as previously defined, wherein X of Formula (E), or one of R and $R^1$ of Formula (I), is mono- or disubstituted phenyl containing one nitro group, with hydrogen gas on a catalyst to give the corresponding aminophenyl compound of Formulae (I) or (E); or

I. cycloalkylating a compound of Formulae (I), or (E) as previously defined wherein one of $R^1$ and R of Formula (I), or X of Formula (E), is aminophenyl, with dihalobutane or dihalopentane as appropriate in the presence of a base to give the corresponding N-piperidino or N-pyrrolidino compound of Formula (I), or (E); or

J. when it is required to prepare compounds of Formulae (I) or (E) as previously defined, wherein $R^1$ and R of Formula (I), or X of Formula (E), is ($C_{1-3}$ alkylamino)phenyl, reducing the corresponding $C_{1-3}$ alkanamido compounds with borane or borane dimethylsulfide complex in tetrahydrofuran to yield the desired compounds of Formulae (I) or (E); or

K. when it is required to prepare compounds of Formulae (I) or (E) as previously defined wherein one of $R^1$ and R of Formula (I), or X of Formula (E), is ($C_{1-3}$ dialkylamino)phenyl, reducing the corresponding N-($C_{1-3}$ alkyl)($C_{1-3}$alkanamido) compounds with borane or borane dimethylsulfide complex in tetrahydrofuran to yield the desired compounds of Formulae (I) or (E); or

L.

(i) reacting a compound of Formulae (E) or (G) with a $C_{1-5}$ alkyllithium reagent to yield the corresponding lithium reagent by metallation or lithium-halogen interexchange respectively;

(ii) adding excess magnesium halide etherate to the lithium reagent to yield the corresponding Grignard reagent by transmetallation;

(iii) adding the Grignard reagent to an N-acylpyridium salt to yield the corresponding compound of Formula (F) as defined above;

(iv) deacylating/oxidizing the compound of Formula (F) to yield the corresponding compound of Formula (I); or

M. treating the 3-lithio derivative of a compound of Formulae (E) or (G), each as previously defined, with trialkyltin chloride to yield a compound of Formula (J) having the following formula

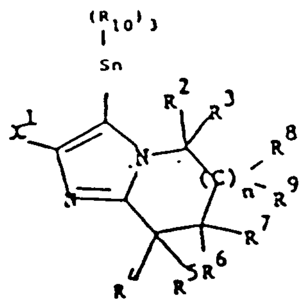

FORMULA J

wherein

n is 0 or 1;

$R^2$, $R^3$, $R^4$, $R^5$, $R^6$, $R^7$, $R^8$ and $R^9$ are H, or one or two of $R^2$, $R^3$, $R^4$, $R^5$, $R^6$, $R^7$, $R^8$ and $R^9$ are independently selected from H or $C_{1-2}$ alkyl;

$R_{10}$ is $C_{1-4}$ alkyl; and $X^1$ is selected from

(a) monosubstituted phenyl wherein said substituent is selected from H, fluoro, chloro, $C_{1-3}$ alkoxy, $C_{1-4}$ alkyl, $C_{1-3}$ dialkylamino, $CF_3$, $C_{1-3}$ alkylamino, N-pyrrolidino, N-piperidino, prop-2-ene-1-oxy or 2,2,2-trihaloethoxy; or

(b) pyridyl or alkyl substituted pyridyl;

and reacting the Formula (J) compound with a mixture of an aryl or heteroaryl halide or triflate and tetrakis(triphosphine)palladium in a mixture of tetrahydrofuran and hexamethylphosphoramide to yield a compound of Formula (I); or

N. when it is required to prepare a compound of Formula (I) wherein one of R or $R^1$ is an substituted phenyl containing one or more alkylthio groups, treating the corresponding fluoro substituted phenyl compound of Formula (I) with a metal salt of the alkylmercaptan in an aprotic polar solvent to yield the desired compound of Formula (I); or

O. when it is required to prepare a compound of Formula (I) wherein one of R or $R^1$ is phenyl substituted with acyloxyalkylthio wherein the alkyl is optionally substituted with $C_{1-4}$ alkyl, treating a compound of Formula (I) wherein $R^1$ is phenyl substituted by at least one alkylsulfinyl with an alkanoic acid anhydride to yield the desired compound of Formula (I); or

P. when it is required to prepare a compound of Formula (I) wherein one of $R^1$ or R is phenyl substituted with at least one sulfhydryl group, hydrolyzing the product of process O as described above to yield the desired compounds of Formula (I); or

Q. when it is required to prepare a compound of Formula (I) wherein one of $R^1$ or R is phenyl substituted with at least one acylthio, dithioacyl, thiocarbamyl or dithiocarbamyl group, treating the product of process P above with an acyl halide, alkanoic acid anhydride, thioacyl halide, dithioalkanoic acid anhydride, dialkylcarbamyl halide, or dialkylthiocarbamyl halide in the presence of a base such as pyridine to yield the desired compounds of Formula (I); or

R. when it is required to prepare a compound of Formula (I), wherein $R^1$ or R is phenyl substituted with an alkenylthio group, alkylating a compound of Formula (I) wherein one of $R^1$ or R is phenyl substituted by at least one sulfhydryl group with an appropriately substituted alkenylhalide, such as allylbromide, to yield compounds of Formula (I) wherein $R^1$ or R is phenyl substituted by at least one alkenylthio group; or

S. when it is required to prepare a compound of Formula (I) wherein $R^1$ or R is phenyl substituted with an alkenylthio group, wherein the sulfur is attached to the carbon bearing the double bond,

i) treating the mercapto product of process P above with a strong base to yield the corresponding metal mercaptide salt compound,

ii) treating the metal mercaptide salt compound with trialkylsilylmethylchloride to yield a compound of Formula (I) wherein the phenyl has at least one trialkylsilylmethylsulfide substituent,

iii) treating the trialkylsilylmethylsulfide substituted compound in an aprotic solvent with a lithiating reagent followed by the appropriate aliphatic aldehyde or ketone to yield a compound of Formula (I) wherein R or $R^1$ is phenyl substituted by at least one alkenylthio group; or

T. when it is required to prepare a compound of Formula (I) wherein R or $R^1$ is alkyl substituted pyridyl, deacylating and aromatizing a compound of Formula (L)

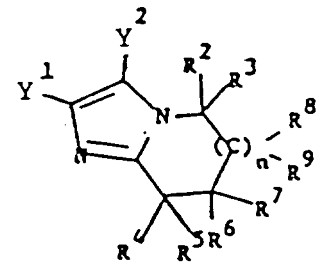

FORMULA (L)

wherein:

n is 0 or 1,

$R^2$, $R^3$, $R^4$, $R^5$, $R^6$, $R^7$, $R^8$, $R^9$ are all H, or one or two of $R^2$, $R^3$, $R^4$, $R^5$, $R^6$, $R^7$, $R^8$ and $R^9$, are independently selected from H or $C_{1-2}$ alkyl;

One of $Y^1$ or $Y^2$ is independently selected from 4-[1,2-dihydro-2-($C_{1-4}$alkyl)]pyridyl substituted with N-($C_{1-8}$ alkanoyl), N-($C_{1-8}$ alkoxycarbonyl), N-(benzoyl), N-(phenoxycarbonyl), N-(phenylacetyl), or N-(benzyloxycarbonyl);

and the other is selected from

(a) monosubstituted phenyl wherein said substituent is selected from H, halo, $C_{1-3}$ alkoxy, $C_{1-3}$ alkylthio, $C_{1-4}$ alkyl, N-($C_{1-3}$ alkyl)-N-($C_{1-3}$ alkanamido), $C_{1-3}$dialkylamino, $CF_3$, N-pyrrolidino, N-piperidino, prop-2-ene-1-oxy or 2,2,2-trihaloethoxy;

or a pharmaceutically acceptable salt thereof, with sulfur in refluxing decalin, tetralin, p-cymene or xylene with oxygen gas for 15 minutes to afford the corresponding compound of Formula (I).

**2.** A process according to Claim 1 wherein the compound of the formula (I) is 2-(4-methylthiophenyl)-3-(4-pyridyl)-6,7-dihydro-[5H]-pyrrolo[1,2-a]imidazole or a pharmaceutically acceptable salt thereof.

**3.** A process according to Claim 1 wherein the compound of the formula (I) is 2-(4-methylsulfinylphenyl)-3-(4-pyridyl)-6,7-dihydro-[5H]-pyrrolo[1,2-a]imidazole or a pharmaceutically acceptable salt thereof.

**4.** The process of Claim 1 which comprises preparation of a compound of Formula (I) which is

2-(4-methylsulfonylphenyl)-3-(4-pyridyl)-6,7-dihydro-[5H]-pyrrolo[1,2-a]imidazole;
2-(4-methoxyphenyl)-3-(2,6-dimethyl-4-pyridyl)-6,7-dihydro-[5H]-pyrrolo[1,2-a]imidazole;
2-(4-ethylthiophenyl)-3-(4-pyridyl)-6,7-dihydro-[5H]-pyrrolo[1,2-a]imidazole;
2-(4-ethylsulfinylphenyl)-3-(4-pyridyl)-6,7-dihydro-[5H]-pyrrolo[1,2-a]imidazole;
2-(4-mercaptophenyl)-3-(4-pyridyl)-6,7-dihyro-[5H]-pyrrolo[1,2-a]imidazole;
2-(4-trimethylacetylthiophenyl)-3-(4-pyridyl)-6,7-dihydro-[5H]-pyrrolo[1,2-a]imidazole;
2-(4-acetylthiophenyl)-3-(4-pyridyl)-6,7-dihydro-[5H]-pyrrolo(1,2-a]imidazole;
2-(4-ethylsulfonylphenyl)-3-(4-pyridyl)-6,7-dihydro-[5H]-pyrrolo[1,2-a]imidazole;
2-(4-fluorophenyl)-3-[4-(2-methyl)pyridyl]-6,7-dihydro-[5H]-pyrrolo[1,2-a]imidazole,
2-(4-methylthiophenyl)-3-[4-(2-methyl)pyridyl]-6,7-dihydro-[5H]-pyrrolo[1,2-a]imidazole,
2-(4-methylsulfinylphenyl)-3-[4-(2-methyl)pyridyl]-6,7-dihydro-[5H]-pyrrolo[1,2-a]imidazole;
2-(4-carbethoxymethylthiophenyl)-3-(4-pyridyl)-6,7-dihydro-[5H]-pyrrolo[1,2-a]imidazole; or
2-(4-acetoxymethylthiophenyl)-3-(4-pyridyl)-6,7-dihydro-[5H]-pyrrolo[1,2-a]imidazole.

**5.** A process for the preparation of a compound of Formula (I) which is 2-(4-methylthiophenyl)-3-(4-pyridyl)-6,7-dihydro-[5H]-pyrrolo[1,2-a]imidazole comprising treating the corresponding fluoro substituted phenyl compund of Formula (I) as defined in Claim 1 with 1.2 equivalents of a metal salt of the alkylmercaptan in an aprotic polar solvent.

**6.** A process for the preparation of a compound of Formula (I) which is 2-(4-methylsulfinylphenyl)-3-(4-pyridyl)-6,7-dihydro-[5H]-pyrrolo[1,2-a]imidazole comprising the oxidation of the corresponding 4-methylthiophenyl compound with an appropriate oxidizing agent.

**7.** A process for the preparation of a pharmaceutical composition containing a compound of the formula (I) as defined in any one of Claims 1 to 4, which process comprises bringing the compound into association with a pharmaceutically acceptable carrier. hydrohalide salt optionally in the presence of 1 to 3 equivalents of an inorganic or organic base to yield a compound of Formula (E).

**8.** A process for the preparation of a compound of Formula (L) having the following structure

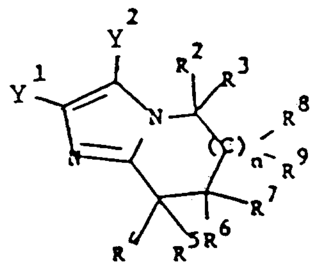

FORMULA (L)

wherein:

n is 0 or 1,

$R^2$, $R^3$, $R^4$, $R^5$, $R^6$, $R^7$, $R^8$, $R^9$ are all H, or one or two of $R^2$, $R^3$, $R^4$, $R^5$, $R^6$, $R^7$, $R^8$ and $R^9$, are independently selected from H or $C_{1-2}$ alkyl;

one of $Y^1$ or $Y^2$ is independently selected from 4-[1,2-dihydro-2-($C_{1-4}$-alkyl)]pyridyl substituted with N-($C_{1-8}$ alkanoyl), N-($C_{1-8}$ alkoxycarbonyl), N-(benzoyl), N-(phenoxycarbonyl), N-(phenylacetyl), or N-(benzyloxycarbonyl);

and the other is selected from

(a) monosubstituted phenyl wherein said substituent is selected from H, halo, $C_{1-3}$ alkoxy, $C_{1-3}$ alkylthio, $C_{1-4}$ alkyl, N-($C_{1-3}$ alkyl)-N-($C_{1-3}$ alkanamido), $C_{1-3}$ dialkylamino, $CF_3$, N-pyrrolidino, N-piperidino, prop-2-ene-1-oxy or 2,2,2-trihaloethoxy;

or a pharmaceutically acceptable salt thereof, which comprises treatment of a compound of Formula (I) of Claim 1 with an acyl halide, aroylhalide, arylalkyl haloformate ester or an alkyl haloformate ester and an $C_{1-4}$ alkyl Grignard reagent.

**9.** A process for the preparation of a compound of Formula (J) having the following structure

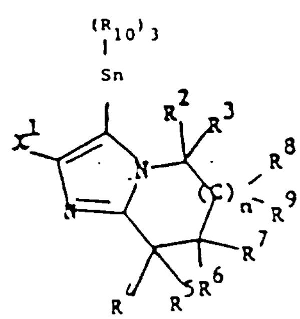

FORMULA (J)

wherein

n is 0 or 1;

$R^2$, $R^3$, $R^4$, $R^5$, $R^6$, $R^7$, $R^8$ and $R^9$ are H, or one or two of $R^2$, $R^3$, $R^4$, $R^5$, $R^6$, $R^7$, $R^8$ and $R^9$ are independently selected from H or $C_{1-2}$ alkyl;

$R_{10}$ is $C_{1-4}$ alkyl;

and $X^1$ is selected from

(a) phenyl or monosubstituted phenyl wherein said substituent is selected from H, fluoro, chloro, $C_{1-3}$ alkoxy, $C_{1-4}$ alkyl, $C_{1-3}$ dialkylamino, $CF_3$, $C_{1-3}$ alkylamino, N-pyrrolidino, N-piperidino, prop-2-ene-1-oxy or 2,2,2-trihaloethoxy;

(e) pyridyl or alkyl substituted pyridyl; or a pharmaceutically acceptable salt thereof, which comprises reaction of a 3-lithio derivative of a compound of Formula (E) as defined in Claim 1 with trialkyltin chloride.

**Claims for the following Contracting State : GR**

**1.** A process for the preparation of compound of the formula

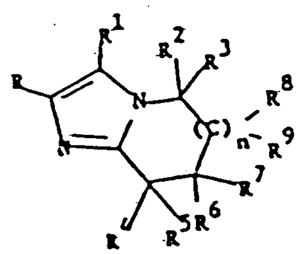

FORMULA (I)

wherein

1) One of R or $R^1$ must be alkyl substituted pyridyl and the other is selected from:

monosubstituted phenyl wherein said substituent is selected from H, halo, hydroxy $C_{1-3}$ alkoxy, $C_{1-3}$ alkylthio, $C_{1-4}$ alkyl,$C_{1-3}$ alkylsulfinyl, $C_{1-3}$ alkylsulfonyl, $C_{1-3}$ alkylamino, $C_{1-3}$ dialkylamino, $CF_3$, N-($C_{1-3}$ alkyl)-N-($C_{1-3}$alkanamido), N-pyrrolidino, N-piperidino, prop-2-ene-1-oxy or 2,2,2-trihaloethoxy;

2) $R^1$ is 4-pyridyl and R selected from:

monosubstituted phenyl wherein said substituent is selected from $C_{1-3}$ alkylthio, $C_{1-3}$ alkylsulfinyl, $C_{1-3}$ alkylsulfonyl, or a branched or unbranched $C_{2-5}$ alkenylthio or $C_{2-5}$ alkenylsulfinyl; or

3) One of $R^1$ or R is pyridyl or alkyl substituted pyridyl and the other is selected from monosubstituted or disubstituted phenyl wherein said substituent is selected from thiol [HS-], acylthio [AC(O)-S-], dithioacyl [AC(S)S-], dialkylthiocarbamyl [$A_2$NC(O)S-], dialkyldithiocarbamyl [$A_2$NC(S)S-], alkylcarbonylalkylthio [AC(O)$CH_2$S-], carbalkoxyalkylthio [AOC(O)$CH_2$S-], or acyloxyalkylthio [AC(O)-O$CH_2$S-] wherein the $CH_2$ is optionally substituted with $C_{1-4}$ alkyl, and A is $C_{1-9}$alkyl;

and $R^2$, $R^3$, $R^4$, $R^5$, $R^6$, $R^7$, $R^8$ and $R^9$ are H, or one or two of $R^2$, $R^3$, $R^3$, $R^4$, $R^5$, $R^6$, $R^7$, $R^8$ and $R^9$ are independently selected from H or $C_{1-2}$ alkyl; and n is 0 or 1;

or a pharmaceutically acceptable salt thereof, which comprises

A. when it is required to prepare a compound of Formula (I) wherein $R^1$ is 4-pyridyl, and R is other than pyridyl, reacting a compound of the Formula (E) represented by the structure:

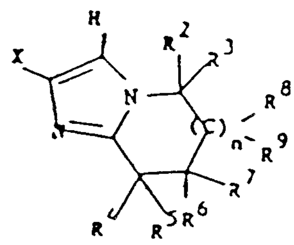

FORMULA (E)

wherein

n is 0 or 1;

$R^2$, $R^3$, $R^4$, $R^5$, $R^6$, $R^7$, $R^8$ and $R^9$ are H, or one or two of $R^2$, $R^3$, $R^4$, $R^5$, $R^6$, $R^7$, $R^8$ and $R^9$ are independently selected from H or $C_{1-2}$alkyl;

X is $C_{1-3}$alkylthiophenyl

in pyridine with an aroyl halide, an arylalkyl haloformate ester, or an alkyl haloformate ester, or with the preformed acyl pyridinium salt to yield a compound of formula (F) represented by the structure:

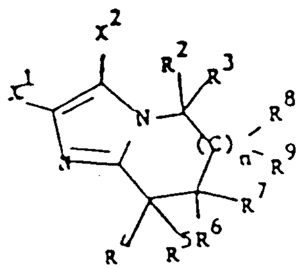

FORMULA (F)

wherein

n is 0 or 1

$R^2$, $R^3$, $R^4$, $R^5$, $R^6$, $R^7$, $R^8$ and $R^9$ are H, or one or two of $R^2$, $R^3$, $R^4$, $R^5$, $R^6$, $R^7$, $R^8$ and $R^9$ are independently selected from H or $C_{1-2}$ alkyl;

$X^1$ is phenyl monosubstituted by $C_{1-3}$alkylthio, and

$X_2$ is 4-(1,4-dihydro)pyridyl substituted with

N-($C_{1-8}$alkanoyl), N-($C_{1-8}$alkoxycarbonyl),

N-(benzoyl), N-(phenoxycarbonyl),

N-(phenylacetyl) or N-(benzyloxycarbonyl); followed by deacylation/oxidation of a compound of Formula (F) to yield a compound of Formula (I); or

B. when it is required to prepare a hydroxy compound of Formulae (I) or (E), demethylating a methoxyphenyl compound of Formulae (E), or (I), wherein Formula (E) is as defined above except that X is methoxyphenyl, Formula (I) is represented by the structure:

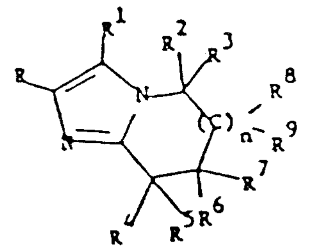

FORMULA (I)

wherein

n is 0 or 1;

$R^2$, $R^3$, $R^4$, $R^5$, $R^6$, $R^7$, $R^8$, and $R^9$ and H or one or two of $R^2$, $R^3$, $R^4$, $R^5$, $R^6$, $R^7$, $R^8$ and $R^9$ are independently selected from H or $C_{1-2}$ alkyl;

One of $R^1$ and R is alkyl substituted pyridyl and the other is selected from methoxyphenyl or a pharmaceutically acceptable salt thereof;

with HBr in acetic acid or $BBr_3$ in methylene chloride to give the corresponding hydroxy compound of Formulae (I), or (E); or

C. O-alkylating the hydroxy compound of Formula (I), or (E), as defined above when one of $R^1$ and R for (I), or X for (E) is a phenyl group substituted with at least one hydroxy, to give the corresponding $C_{1-3}$ alkoxyphenyl, 2,2,2-trihaloethoxyphenyl or prop-2-ene-1-oxy-phenyl substituted impound of Formulae (I) or (E); or

D. acylating an aminophenyl or ($C_{1-3}$ alkylamino)phenyl compound of Formula (I) with the correspondingly substituted acyl halide or anhydride in pyridine to give the corresponding N-($C_{1-3}$ alkyl)-($C_{1-3}$ alkanamido)phenyl compound of Formula (I); or

E. alkylating a ($C_{1-3}$ alkanamido)phenyl compound of Formulae (I) or (E) as previously defined with an alkylating agent in the presence of a base to give the corresponding N-($C_{1-3}$ alkanamido)phenyl or N-($C_{1-3}$ alkyl)-($C_{1-3}$ alkanamido)phenyl compound of Formulae (I), or (E); or

F. hydrolyzing a ($C_{1-3}$ alkanamido)phenyl or N-($C_{1-3}$ alkyl)-($C_{1-3}$ alkanamido)phenyl compound of Formulae (I), or (E) as previously defined or of Formula (G) represented by the structure:

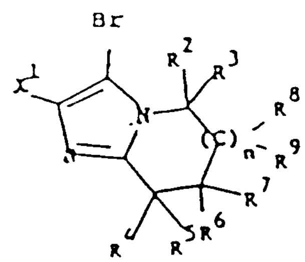

FORMULA (G)

wherein:

n is 0 or 1;

$R^2$, $R^3$, $R^4$, $R^5$, $R^6$, $R^7$, $R^8$, $R^9$ are H; or one or two of $R^2$, $R^3$, $R^4$, $R^5$, $R^6$, $R^7$, $R^8$ and $R^9$ are independently selected from H or $C_{1-2}$ alkyl;

$X^1$ is ($C_{1-3}$alkanamido)phenyl or N-($C_{1-3}$alkyl)-($C_{1-3}$alkanamido)phenyl to give the corresponding aminophenyl or ($C_{1-3}$ alkylamino)phenyl compound of Formulae (I), (E) or (G); or

G. oxidizing a compound of Formula (I) when one of $R^1$ and R is phenyl substituted by one or more $C_{1-3}$ alkylthio, $C_{1-3}$ alkylsulfinyl, $C_{1-3}$ alkenylthio, or acyloxyalkylthio with an oxidizing agent to give the corresponding $C_{1-3}$ alkylsulfinyl, $C_{1-3}$ alkylsulfonyl, acyloxyalkylsulfinyl or $C_{1-3}$ alkenylsulfinyl compound of Formula (I); or

H. reducing of a compound of Formulae (E) or (I) as previously defined, wherein X of Formula (E), or one of R and $R^1$ of Formula (I), is mono- or disubstituted phenyl containing one nitro group, with hydrogen gas on a catalyst to give the corresponding aminophenyl compound of Formulae (I) or (E); or

I. cycloalkylating a compound of Formulae (I), or (E) as previously defined wherein one of $R^1$ and R of Formula (I), or X of Formula (E), is aminophenyl, with dihalobutane or dihalopentane as appropriate in the presence of a base to give the corresponding N-piperidino or N-pyrrolidino compound of Formula (I), or (E); or

J. when it is required to prepare compounds of Formulae (I) or (E) as previously defined, wherein $R^1$ and R of Formula (I), or X of Formula (E), is ($C_{1-3}$ alkylamino)phenyl, reducing the corresponding $C_{1-3}$ alkanamido compounds with borane or borane dimethylsulfide complex in tetrahydrofuran to yield the desired compounds of Formulae (I) or (E); or

K. when it is required to prepare compounds of Formulae (I) or (E) as previously defined wherein one of $R^1$ and R of Formula (I), or X of Formula (E), is ($C_{1-3}$ dialkylamino)phenyl, reducing the corresponding N-($C_{1-3}$ alkyl)($C_{1-3}$alkanamido) compounds with borane or borane dimethylsulfide complex in tetrahydrofuran to yield the desired compounds of Formulae (I) or (E); or

L.

(i) reacting a compound of Formulae (E) or (G) with a $C_{1-5}$ alkyllithium reagent to yield the corresponding lithium reagent by metallation or lithium-halogen interexchange respectively;

(ii) adding excess magnesium halide etherate to the lithium reagent to yield the corresponding Grignard reagent by transmetallation;

(iii) adding the Grignard reagent to an N-acylpyridium salt to yield the corresponding compound of Formula (F) as defined above;

(iv) deacylating/oxidizing the compound of Formula (F) to yield the corresponding compound of Formula (I); or

M. treating the 3-lithio derivative of a compound of Formulae (E) or (G), each as previously defined, with trialkyltin chloride to yield a compound of Formula (J) having the following formula

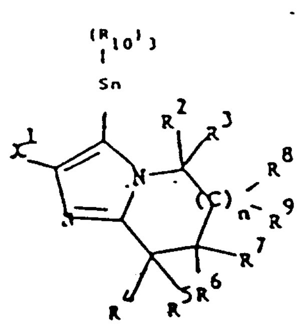

FORMULA J

wherein

n is 0 or 1;

$R^2$, $R^3$, $R^4$, $R^5$, $R^6$, $R^7$, $R^8$ and $R^9$ are H, or one or two of $R^2$, $R^3$, $R^4$, $R^5$, $R^6$, $R^7$, $R^8$ and $R^9$ are independently selected from H or $C_{1-2}$ alkyl;

$R_{10}$ is $C_{1-4}$ alkyl;

and $X^1$ is selected from

(a) monosubstituted phenyl wherein said substituent is selected from H, fluoro, chloro, $C_{1-3}$ alkoxy, $C_{1-4}$ alkyl, $C_{1-3}$ dialkylamino, $CF_3$, $C_{1-3}$ alkylamino, N-pyrrolidino, N-piperidino, prop-2-ene-1-oxy or 2,2,2-trihaloethoxy; or

(b) pyridyl or alkyl substituted pyridyl;

and reacting the Formula (J) compound with a mixture of an aryl or heteroaryl halide or triflate and tetrakis(triphosphine)palladium in a mixture of tetrahydrofuran and hexamethylphosphoramide to yield a compound of Formula (I); or

N. when it is required to prepare a compound of Formula (I) wherein one of R or $R^1$ is an substituted phenyl containing one or more alkylthio groups, treating the corresponding fluoro substituted phenyl

compound of Formula (I) with a metal salt of the alkylmercaptan in an aprotic polar solvent to yield the desired compound of Formula (I); or

O. when it is required to prepare a compound of Formula (I) wherein one of R or $R^1$ is phenyl substituted with acyloxyalkylthio wherein the alkyl is optionally substituted with $C_{1-4}$ alkyl, treating a compound of Formula (I) wherein $R^1$ is phenyl substituted by at least one alkylsulfinyl with an alkanoic acid anhydride to yield the desired compound of Formula (I); or

P. when it is required to prepare a compound of Formula (I) wherein one of $R^1$ or R is phenyl substituted with at least one sulfhydryl group, hydrolyzing the product of process O as described above to yield the desired compounds of Formula (I); or

Q. when it is required to prepare a compound of Formula (I) wherein one of $R^1$ or R is phenyl substituted with at least one acylthio, dithioacyl, thiocarbamyl or dithiocarbamyl group, treating the product of process P above with an acyl halide, alkanoic acid anhydride, thioacyl halide, dithioalkanoic acid anhydride, dialkylcarbamyl halide, or dialkylthiocarbamyl halide in the presence of a base such as pyridine to yield the desired compounds of Formula (I); or

R. when it is required to prepare a compound of Formula (I), wherein $R^1$ or R is phenyl substituted with an alkenylthio group, alkylating a compound of Formula (I) wherein one of $R^1$ or R is phenyl substituted by at least one sulfhydryl group with an appropriately substituted alkenylhalide, such as allylbromide, to yield compounds of Formula (I) wherein $R^1$ or R is phenyl substituted by at least one alkenylthio group; or

S. when it is required to prepare a compound of Formula (I) wherein $R^1$ or R is phenyl substituted with an alkenylthio group, wherein the sulfur is attached to the carbon bearing the double bond,

i) treating the mercapto product of process P above with a strong base to yield the corresponding metal mercaptide salt compound,

ii) treating the metal mercaptide salt compound with trialkylsilylmethylchloride to yield a compound of Formula (I) wherein the phenyl has at least one trialkylsilylmethylsulfide substituent,

iii) treating the trialkylsilylmethylsulfide substituted compound in an aprotic solvent with a lithiating reagent followed by the appropriate aliphatic aldehyde or ketone to yield a compound of Formula (I) wherein R or $R^1$ is phenyl substituted by at least one alkenylthio group; or

T. when it is required to prepare a compound of Formula (I) wherein R or $R^1$ is alkyl substituted pyridyl, deacylating and aromatizing a compound of Formula (L)

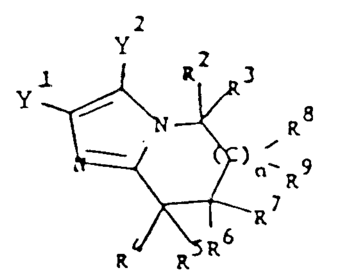

FORMULA (L)

wherein:

n is 0 or 1,

$R^2$, $R^3$, $R^4$, $R^5$, $R^6$, $R^7$, $R^8$, $R^9$ are all H, or one or two of $R^2$, $R^3$, $R^4$, $R^5$, $R^6$, $R^7$, $R^8$ and $R^9$, are independently selected from H or $C_{1-2}$ alkyl;

One of $Y^1$ or $Y^2$ is independently selected from 4-[1,2-dihydro-2-($C_{1-4}$alkyl)]pyridyl substituted with N-($C_{1-8}$ alkanoyl), N-($C_{1-8}$ alkoxycarbonyl), N-(benzoyl), N-(phenoxycarbonyl), N-(phenylacetyl), or N-(benzyloxycarbonyl);

and the other is selected from

(a) monosubstituted phenyl wherein said substituent is selected from H, halo, $C_{1-3}$ alkoxy, $C_{1-3}$ alkylthio, $C_{1-4}$ alkyl, N-($C_{1-3}$ alkyl)-N-($C_{1-3}$ alkanamido), $C_{1-3}$dialkylamino, $CF_3$, N-pyrrolidino, N-piperidino, prop-2-ene-1-oxy or 2,2,2-trihaloethoxy;

or a pharmaceutically acceptable salt thereof, with sulfur in refluxing decalin, tetralin, p-cymene or xylene with oxygen gas for 15 minutes to afford the corresponding compound of Formula (I).

2. A process according to Claim 1 wherein the compound of the formula (I) is 2-(4-methylthiophenyl)-3-(4-pyridyl)-6,7-dihydro-[5H]-pyrrolo[1,2-a]imidazole or a pharmaceutically acceptable salt thereof.

3. A process according to Claim 1 wherein the compound of the formula (I) is 2-(4-methylsulfinylphenyl)-3-(4-pyridyl)-6,7-dihydro-[5H]-pyrrolo[1,2-a]imidazole or a pharmaceutically acceptable salt thereof.

4. The process of Claim 1 which comprises preparation of a compound of Formula (I) which is
2-(4-methylsulfonylphenyl)-3-(4-pyridyl)-6,7-dihydro-[5H]-pyrrolo[1,2-a]imidazole;
2-(4-methoxyphenyl)-3-(2,6-dimethyl-4-pyridyl)-6,7-dihydro-[5H]-pyrrolo[1,2-a]imidazole;
2-(4-ethylthiophenyl)-3-(4-pyridyl)-6,7-dihydro-[5H]-pyrrolo[1,2-a]imidazole;
2-(4-ethylsulfinylphenyl)-3-(4-pyridyl)-6,7-dihydro-[5H]-pyrrolo[1,2-a]imidazole;
2-(4-mercaptophenyl)-3-(4-pyridyl)-6,7-dihyro-[5H]-pyrrolo[1,2-a]imidazole;
2-(4-trimethylacetylthiophenyl)-3-(4-pyridyl)-6,7-dihydro-[5H]-pyrrolo[1,2-a]imidazole;
2-(4-acetylthiophenyl)-3-(4-pyridyl)-6,7-dihydro-[5H]-pyrrolo(1,2-a]imidazole;
2-(4-ethylsulfonylphenyl)-3-(4-pyridyl)-6,7-dihydro-[5H]-pyrrolo[1,2-a]imidazole;
2-(4-fluorophenyl)-3-[4-(2-methyl)pyridyl]-6,7-dihydro-[5H]-pyrrolo[1,2-a]imidazole,
2-(4-methylthiophenyl)-3-[4-(2-methyl)pyridyl]-6,7-dihydro-[5H]-pyrrolo[1,2-a]imidazole,
2-(4-methylsulfinylphenyl)-3-[4-(2-methyl)pyridyl]-6,7-dihydro-[5H]-pyrrolo[1,2-a]imidazole;
2-(4-carbethoxymethylthiophenyl)-3-(4-pyridyl)-6,7-dihydro-[5H]-pyrrolo[1,2-a]imidazole; or
2-(4-acetoxymethylthiophenyl)-3-(4-pyridyl)-6,7-dihydro-[5H]-pyrrolo[1,2-a]imidazole.

5. A process for the preparation of a compound of Formula (I) which is 2-(4-methylthiophenyl)-3-(4-pyridyl)-6,7-dihydro-[5H]-pyrrolo[1,2-a]imidazole comprising treating the corresponding fluoro substituted phenyl compund of Formula (I) as defined in Claim 1 with 1.2 equivalents of a metal salt of the alkylmercaptan in an aprotic polar solvent.

6. A process for the preparation of a compound of Formula (I) which is 2-(4-methylsulfinylphenyl)-3-(4-pyridyl)-6,7-dihydro-[5H]-pyrrolo[1,2-a]imidazole comprising the oxidation of the corresponding 4-methylthiophenyl compound with an appropriate oxidizing agent.

7. A process for the preparation of a pharmaceutical composition containing a compound of the formula (I) as defined in any one of Claims 1 to 4, which process comprises bringing the compound into association with a pharmaceutically acceptable carrier.

8. A compound of the formula

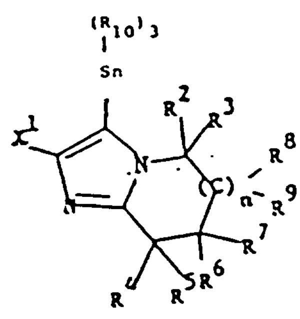

FORMULA (J)

wherein
n is 0 or 1;
$R^2$, $R^3$, $R^4$, $R^5$, $R^6$, $R^7$, $R^8$ and $R^9$ are H, or one or two of $R^2$, $R^3$, $R^4$, $R^5$, $R^6$, $R^7$, $R^8$ and $R^9$ are independently selected from H or $C_{1-2}$ alkyl;
$R_{10}$ is $C_{1-4}$ alkyl;
and $X^1$ is selected from

(a) monosubstituted phenyl wherein said substituent is selected from H, fluoro, chloro, $C_{1-3}$ alkoxy, $C_{1-4}$ alkyl, $C_{1-3}$ dialkylamino, $CF_3$, $C_{1-3}$ alkylamino, N-pyrrolidino, N-piperidino, prop-2-ene-1-oxy or 2,2,2-trihaloethoxy;
(b) pyridyl or alkyl substituted pyridyl; or a pharmaceutically acceptable salt thereof.

**9.** The compound of Claim 8 which is 2-(4-methoxyphenyl)-6,7-dihydro-[5H]-pyrrolo[1,2-a]imidazol-3-yl-tri-n-butyltin.

**10.** A compound of the formula

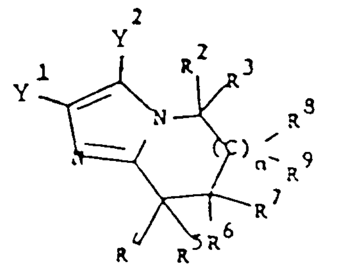

FORMULA (L)

wherein:
n is 0 or 1,
$R^2$, $R^3$, $R^4$, $R^5$, $R^6$, $R^7$, $R^8$, $R^9$ are all H, or one or two of $R^2$, $R^3$, $R^4$, $R^5$, $R^6$, $R^7$, $R^8$ and $R^9$, are independently selected from H or $C_{1-2}$ alkyl;
one of $Y^1$ or $Y^2$ is independently selected from 4-[1,2-dihydro-2-($C_{1-4}$-alkyl]pyridyl substituted with N-($C_{1-8}$ alkanoyl), N-($C_{1-8}$ alkoxycarbonyl), N-(benzoyl), N-(phenoxycarbonyl), N-(phenylacetyl), or N-(benzyloxycarbonyl);
and the other is selected from
(a) monosubstituted phenyl wherein said substituent is selected from H, halo, $C_{1-3}$ alkoxy, $C_{1-3}$ alkylthio, $C_{1-4}$ alkyl, N-($C_{1-3}$ alkyl)-N-($C_{1-3}$ alkanamido), $C_{1-3}$ dialkylamino, $CF_3$, N-pyrrolidino, N-piperidino, prop-2-ene-1-oxy or 2,2,2-trihaloethoxy;
or a pharmaceutically acceptable salt thereof.

**Patentansprüche**
**Patentansprüche für folgende Vertragsstaaten : AT, BE, CH, DE, FR, GB, IT, LI, LU, NL, SE**

**1.** Verbindung der Formel

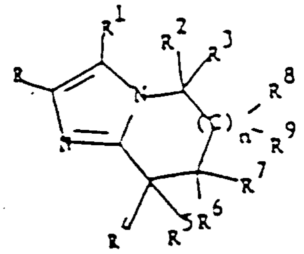

Formel (I)

in der:

1) einer der Reste R oder $R^1$ ein alkyl-substituierter Pyridylrest sein muß und der andere gewählt ist aus:
einem monosubstituierten Phenylrest, wobei der Substituent gewählt ist aus einem H-, Halogenatom, einer Hydroxylgruppe, einem $C_{1-3}$-Alkoxy-, $C_{1-3}$-Alkylthio-, $C_{1-4}$-Alkyl-, $C_{1-3}$-Alkylsulfinyl-, $C_{1-3}$-Alkylsulfonyl-, $C_{1-3}$-Alkylamino-, $C_{1-3}$-Dialkylaminorest, einer $CF_3$-Gruppe, einem N-($C_{1-3}$-Alkyl)-N-($C_{1-3}$-alkanamido)-Rest, einer N-Pyrrolidino-, N-Piperidino-, Prop-2-en-1-oxy- oder 2,2,2-Trihalogenethoxygruppe; oder
2) $R^1$ eine 4-Pyridylgruppe ist und R gewählt ist aus:
einem monosubstituierten Phenylrest, wobei der Substituent gewählt ist aus einem $C_{1-3}$-Alkylthio-, $C_{1-3}$-Alkylsulfinyl-, $C_{1-3}$-Alkylsulfonyl-, oder einem verzweigten oder unverzweigten $C_{2-5}$-Alkenylthio- oder $C_{2-5}$-Alkenylsulfinylrest; oder
3) einer der Reste $R^1$ oder R eine Pyridylgruppe oder ein alkyl-substituierter Pyridylrest ist und der andere aus einem monosubstituierten oder disubstituierten Phenylrest gewählt ist, wobei der Substituent gewählt ist aus: einer Thiolgruppe [HS-], einem Acylthio- [AC(O)S-], Dithioacyl- [AC(S)S-], Dialkylthiocarbamyl- [$A_2$NC(O)S-], Dialkyldithiocarbamyl- [$A_2$NC(S)S-], Alkylcarbonylalkylthio- [AC(O)$CH_2$S-], Carbalkoxyalkylthio- [AOC(O)$CH_2$S-] oder Acyloxyalkylthiorest [AC(O)O$CH_2$S-], wobei die $CH_2$-Gruppe gegebenenfalls mit einem $C_{1-4}$-Alkylrest substituiert ist und A ein $C_{1-9}$-Alkylrest ist;
und $R^2$, $R^3$, $R^4$, $R^5$, $R^6$, $R^7$, $R^8$ und $R^9$ ein H-Atom sind, oder einer oder zwei der Reste $R^2$, $R^3$, $R^3$, $R^4$, $R^5$, $R^6$, $R^7$, $R^8$ und $R^9$ unabhängig aus einem H-Atom oder einem $C_{1-2}$-Alkylrest gewählt sind; n 0 oder 1 ist;
oder ein pharmazeutisch verträgliches Salz davon.

**2.** Verbindung nach Anspruch 1, nämlich 2-(4-Methylthiophenyl)-3-(4-pyridyl)-6,7-dihydro-[5H]-pyrrolo[1,2-a]imidazol.

**3.** Verbindung nach Anspruch 1, nämlich 2-(4-Methylsulfinylphenyl)-3-(4-pyridyl)-6,7-dihydro-[5H]-pyrrolo-[1,2-a]imidazol.

**4.** Verbindung nach Anspruch 1, nämlich: 2-(4-Methylsulfonylphenyl)-3-(4-pyridyl)-6,7-dihydro-[5H]-pyrrolo[1,2-a]imidazol,
2-(4-Methoxyphenyl)-3-(2,6-dimethyl-4-pyridyl)-6,7-dihydro-[5H]-pyrrolo[1,2-a]imidazol,
2-(4-Ethylsulfinylphenyl)-3-(4-pyridyl)-6,7-dihydro-[5H]-pyrrolo[1,2-a]imidazol,
2-(4-Ethylthiophenyl)-3-(4-pyridyl)-6,7-dihydro-[5H]-pyrrolo[1,2-a]imidazol,
2-(4-Mercaptophenyl)-3-(4-pyridyl)-6,7-dihydro-[5H]-pyrrolo[1,2-a]imidazol,
2-(4-Trimethylacetylthiophenyl)-3-(4-pyridyl)-6,7-dihydro-[5H]-pyrrolo[1,2-a]imidazol,
2-(4-Acetylthiophenyl)-3-(4-pyridyl)-6,7-dihydro-[5H]-pyrrolo[1,2-a]imidazol,
2-(4-Ethylsulfonylphenyl)-3-(4-pyridyl)-6,7-dihydro-[5H]-pyrrolo[1,2-a]imidazol,
2-(4-Fluorphenyl)-3-[4-(2-methyl)pyridyl]-6,7-dihydro-[5H]-pyrrolo[1,2-a]imidazol,
2-(4-Methylthiophenyl)-3-[4-(2-methyl)pyridyl]-6,7-dihydro-[5H]-pyrrolo[1,2-a]imidazol,
2-(4-Methylsulfinylphenyl)-3-[4-(2-methyl)pyridyl]-6,7-dihydro-[5H]-pyrrolo[1,2-a]imidazol,
2-(4-Carbethoxymethylthiophenyl)-3-(4-pyridyl)-6,7-dihydro-[5H]-pyrrolo[1,2-a]imidazol, oder
2-(4-Acetoxymethylthiophenyl)-3-(4-pyridyl)-6,7-dihydro-[5H]-pyrrolo[1,2-a]imidazol.

**5.** Arzneimittel, umfassend einen pharmazeutisch verträglichen Träger oder ein Verdünnungsmittel und eine Verbindung nach einem der Ansprüche 1 bis 4.

**6.** Arzneimittel nach Anspruch 5, wobei das Mittel in einer Dosierungseinheitsform vorliegt, die zur parenteralen Verabreichung geeignet ist und die 50 bis 500 mg des Wirkstoffs umfaßt.

**7.** Arzneimittel nach Anspruch 5, wobei das Mittel in einer Dosierungseinheitsform vorliegt, die zur oralen Verabreichung geeignet ist und 100 bis 1000 mg des Wirkstoffs umfaßt.

**8.** Arzneimittel nach Anspruch 5, wobei das Mittel in einer Dosierungseinheitsform vorliegt, die zur Verabreichung durch Inhalation oder örtliche Verabreichung geeignet ist.

**9.** Verbindung der Formel (I) nach einem der Ansprüche 1 bis 4 für die Behandlung einer Erkrankung im Zusammenhang mit dem 5-Lipoxygenase-Weg beim Tier.

**10.** Verbindung der Formel (I) nach einem der Ansprüche 1 bis 4 zur Behandlung oder Prophylaxe einer entzündlichen Erkrankung beim Tier oder von Fieber, Schmerzen oder anderen mit Entzündungen verbundenen Erkrankungen.

**11.** Verbindung der Formel (I) zur Verwendung nach Anspruch 10, wobei die entzündliche Erkrankung rheumatische Arthritis, rheumatische Spondylitis, Osteoarthritis, gichtische Arthritis und andere arthritische Erkrankungen, entzündete Gelenke, Ekzeme, Psoriase oder andere Hautentzündungen, z.B. Sonnenbrand; und Augenentzündungen, z.B. Bindehautentzündung, umfaßt.

**12.** Verbindung der Formel

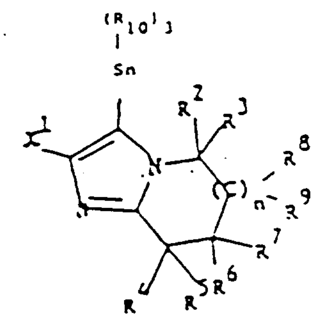

FORMEL (J)

in der
n 0 oder 1 ist;
$R^2$, $R^3$, $R^4$, $R^5$, $R^6$, $R^7$, $R^8$ und $R^9$ jeweils ein H-Atom sind oder einer oder zwei der Reste $R^2$, $R^3$, $R^4$, $R^5$, $R^6$, $R^7$, $R^8$ und $R^9$ unabhängig aus einem H-Atom oder einer $C_{1-2}$-Alkylgruppe gewählt ist;
$R_{10}$ ein $C_{1-4}$-Alkylrest ist;
und $X^1$ gewählt ist aus:

(a) einem monosubstituierten Phenylrest, wobei der Substituent aus einem H-, Fluor-, Chloratom, einem $C_{1-3}$-Alkoxy-, $C_{1-4}$-Alkyl-, $C_{1-3}$-Dialkylaminorest, einer $CF_3$-Gruppe, einem $C_{1-3}$-Alkylaminorest, einer N-Pyrrolidino-, N-Piperidino-, Prop-2-en-1-oxy- oder 2,2,2-Trihalogenethoxygruppe gewählt ist;
(b) einer Pyridylgruppe oder einem alkyl-substituierten Pyridylrest;

oder ein pharmazeutisch verträgliches Salz davon.

**13.** Verbindung nach Anspruch 12, nämlich 2-(4-Methoxyphenyl)-6,7-dihydro-[5H]-pyrrolo[1,2-a]imidazol-3-yl-tri-n-butylzinn.

**14.** Verbindung der Formel

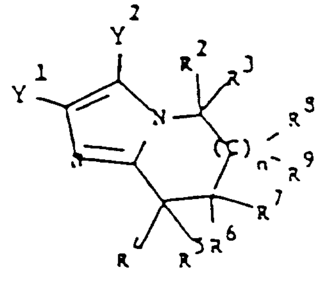

FORMEL (L)

in der:
n 0 oder 1 ist,

$R^2$, $R^3$, $R^4$, $R^5$, $R^6$, $R^7$, $R^8$, $R^9$ alle ein H-Atom sind, oder einer oder zwei der Reste $R^2$, $R^3$, $R^4$, $R^5$, $R^6$, $R^7$, $R^8$ und $R^9$ unabhängig aus einem H-Atom oder einem $C_{1-2}$-Alkylrest gewählt sind;
einer der Reste $Y^1$ oder $Y^2$ unabhängig aus einem 4-[1,2-Dihydro-2-($C_{1-4}$-alkyl)]pyridylrest gewählt ist, der mit einem N-($C_{1-8}$-Alkanoyl)-, N-($C_{1-8}$-Alkoxycarbonyl)-Rest, einer N-(Benzoyl)-, N-(Phenoxycarbonyl)-, N-(Phenylacetyl)- oder N-(Benzyloxycarbonyl)-Gruppe substituiert ist;
und der andere gewählt ist aus:
(a) einem monosubstituierten Phenylrest, wobei der Substituent aus einem H-, einem Halogenatom, einem $C_{1-3}$-Alkoxy-, $C_{1-3}$-Alkylthio-, $C_{1-4}$-Alkyl-, N-($C_{1-3}$-Alkyl)-N-($C_{1-3}$-alkanamido)-, $C_{1-3}$-Dialkylamino-Rest, einer $CF_3$-, N-Pyrrolidino-, N-Piperidino-, Prop-2-en-1-oxy- oder 2,2,2-Trihalogenethoxy-Gruppe gewählt ist; oder ein pharmazeutisch verträgliches Salz davon.

**15.** Verfahren zur Herstellung einer Verbindung der Formel (I) wie in Anspruch 1 definiert oder eines pharmazeutisch verträglichen Salzes davon, umfassend:
A. wenn eine Verbindung der Formel (I) hergestellt werden soll, in der $R^1$ eine 4-Pyridylgruppe ist und R von einer Pyridylgruppe verschieden ist: Umsetzen einer Verbindung der Formel (E) mit der Strukturformel:

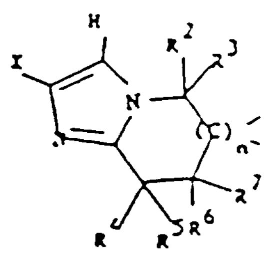

FORMEL (E)

in der n 0 oder 1 ist;
$R^2$, $R^3$, $R^4$, $R^5$, $R^6$, $R^7$, $R^8$ und $R^9$ ein H-Atom sind oder einer oder zwei der Reste $R^2$, $R^3$, $R^4$, $R^5$, $R^6$, $R^7$, $R^8$ und $R^9$ unabhängig aus einem H-Atom oder einem $C_{1-2}$-Alkylrest gewählt sind;
X ein $C_{1-3}$-Alkylthiophenylrest ist;
in Pyridin mit einem Aroylhalogenid oder einem Halogenameisensäurearylalkylester oder einem Halogenameisensäurealkylester oder mit dem vorher hergestellten Acylpyridiniumsalz, wodurch eine Verbindung der Formel (F) mit der Strukturformel

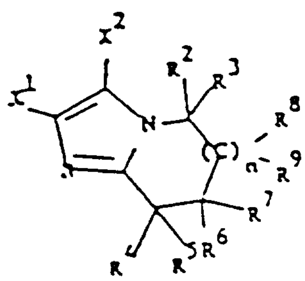

FORMEL (F)

erhalten wird,
in der
n 0 oder 1 ist;
$R^2$, $R^3$, $R^4$, $R^5$, $R^6$, $R^7$, $R^8$ und $R^9$ ein H-Atom sind oder einer oder zwei der Reste $R^2$, $R^3$, $R^4$, $R^5$, $R^6$, $R^7$, $R^8$ und $R^9$ unabhängig aus einem H-Atom oder einem $C_{1-2}$-Alkylrest gewählt sind;
$X^1$ ein mit einem $C_{1-3}$-Alkylthiorest monosubstituierter Phenylrest ist; und
$X^2$ ein 4-(1,4-Dihydro)pyridyl-Rest ist, der substituiert ist mit einem N-($C_{1-8}$-Alkanoyl)-, N-($C_{1-8}$-

Alkoxycarbonyl)-Rest, einer N-(Benzoyl)-, N-(Phenoxycarbonyl)-, N-(Phenylacetyl)- oder N-(Benzyloxycarbonyl)-Gruppe;
gefolgt von Deacylierung/Oxidation der Verbindung der Formel (F), wodurch eine Verbindung der Formel (I) erhalten wird; oder
B. wenn eine Hydroxyverbindung der Formel (I) oder (E) hergestellt werden soll: Demethylieren einer Methoxyphenylverbindung der Formel (E) oder (I), wobei die Formel (E) wie vorstehend definiert ist, außer daß X eine Methoxyphenylgruppe ist und die Formel (I) durch die Strukturformel dargestellt wird:

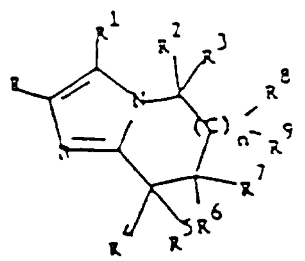

FORMEL (I)

in der
n 0 oder 1 ist;
$R^2$, $R^3$, $R^4$, $R^5$, $R^6$, $R^7$, $R^8$ und $R^9$ ein H-Atom sind oder einer oder zwei der Reste $R^2$, $R^3$, $R^4$, $R^5$, $R^6$, $R^7$, $R^8$ und $R^9$ unabhängig aus einem H-Atom oder einem $C_{1-2}$-Alkylrest gewählt sind;
einer der Reste $R^1$ und R ein alkyl-substituierter Pyridylrest und der andere eine Methoxyphenylgruppe ist;

oder ein pharmazeutisch verträgliches Salz davon; mit HBr in Essigsäure oder $BBr_3$ in Methylenchlorid, wodurch die entsprechende Hydroxyverbindung der Formel (I) oder (E) erhalten werden; oder

C. O-Alkylieren der Hydroxyverbindung der Formel (I) oder (E), wie vorstehend definiert, wenn einer der Reste $R^1$ und R für (I), oder X für (E) eine Phenylgruppe ist, die mit mindestens einer Hydroxylgruppe substituiert ist, wodurch die entsprechende $C_{1-3}$-alkoxyphenyl-, 2,2,2-trihalogenethoxyphenyl-, oder prop-2-en-1-oxy-phenyl-substituierte Verbindung der Formel (I) oder (E) erhalten wird; oder

D. Acylieren einer Aminophenyl- oder ($C_{1-3}$-Alkylamino)phenyl-Verbindung der Formel (I) mit dem entsprechend substituierten Acylhalogenid oder -anhydrid in Pyridin, wodurch die entsprechende N-($C_{1-3}$-Alkyl)-($C_{1-3}$-alkanamido)phenyl-Verbindung der Formel (I) erhalten wird; oder

E. Alkylieren einer ($C_{1-3}$-Alkanamido)phenyl-Verbindung der Formel (I) oder (E), wie bereits definiert, mit einem Alkylierungsmittel in Gegenwart einer Base, wodurch die entsprechende N-($C_{1-3}$-Alkanamido)phenyl- oder N-($C_{1-3}$-Alkyl)-($C_{1-3}$-alkanamido)phenyl-Verbindung der Formel (I) oder (E) erhalten wird; oder

F. Hydrolysieren einer ($C_{1-3}$-Alkanamido)phenyl- oder N-($C_{1-3}$-Alkyl)-($C_{1-3}$-alkanamido)phenyl-Verbindung der Formel (I) oder (E), wie bereits definiert, oder der Formel (G) mit der Strukturformel:

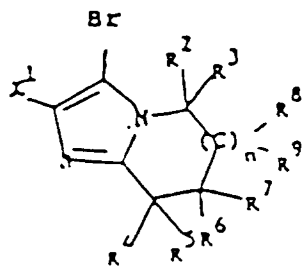

FORMEL (G)

in der
n 0 oder 1 ist;
$R^2$, $R^3$, $R^4$, $R^5$, $R^6$, $R^7$, $R^8$ und $R^9$ ein H-Atom sind oder einer oder zwei der Reste $R^2$, $R^3$, $R^4$, $R^5$, $R^6$, $R^7$, $R^8$ und $R^9$ unabhängig aus einem H-Atom oder einem $C_{1-2}$-Alkylrest gewählt sind;
$X^1$ ein ($C_{1-3}$-Alkanamido)phenyl- oder N-($C_{1-3}$-Alkyl)-($C_{1-3}$-alkanamido)phenyl-Rest ist,
wodurch die entsprechende Aminophenyl- oder ($C_{1-3}$-Alkylamino)phenyl-Verbindung der Formel (I), (E) oder (G) erhalten wird; oder

G. Oxidieren einer Verbindung der Formel (I), wenn einer der Reste $R^1$ oder R ein Phenylrest ist, der mit einem oder mehreren der Reste aus $C_{1-3}$-Alkylthio-, $C_{1-3}$-Alkylsulfinyl-, $C_{1-3}$-Alkenylthio-, oder Acyloxyalkylthio-Rest substituiert ist, mit einem Oxidationsmittel, wodurch die entsprechende $C_{1-3}$-Alkylsulfinyl-, $C_{1-3}$-Alkylsulfonyl-, Acyloxyalkylsulfinyl- oder $C_{1-3}$-Alkenylsulfinyl-Verbindung der Formel (I) erhalten wird; oder

H. Reduzieren einer Verbindung der Formel (E) oder (I), wie bereits definiert, wobei X der Formel (E) oder einer der Reste R und $R^1$ der Formel (I) ein mono- oder disubstituierter Phenylrest ist, der eine Nitrogruppe enthält, mit Wasserstoffgas über einem Katalysator, wodurch die entsprechende Aminophenylverbindung der Formel (I) oder (E) erhalten wird; oder

I. Cycloalkylieren einer Verbindung der Formel (I) oder (E), wie bereits definiert, wobei einer der Reste $R^1$ und R der Formel (I) oder X der Formel (E) eine Aminophenylgruppe ist, mit einem geeigneten Dihalogenbutan oder Dihalogenpentan in Gegenwart einer Base, wodurch die entsprechende N-Piperidino- oder N-Pyrrolidino-Verbindung der Formel (I) oder (E) erhalten wird; oder

J. wenn Verbindungen der Formel (I) oder (E), wie bereits definiert, hergestellt werden sollen, wobei $R^1$ und R der Formel (I) oder X der Formel (E) ein ($C_{1-3}$-Alkylamino)phenylrest ist: Reduzieren der entsprechenden $C_{1-3}$-Alkanamido-Verbindungen mit Boran oder einem Borandimethylsulfid-Komplex in Tetrahydrofuran, wodurch die gewünschten Verbindungen der Formel (I) oder (E) erhalten werden; oder

K. wenn Verbindungen der Formel (I) oder (E), wie bereits definiert, hergestellt werden sollen, wobei einer der Reste $R^1$ und R der Formel (I) oder X der Formel (E) ein ($C_{1-3}$-Dialkylamino)phenyl-Rest ist: Reduzieren der entsprechenden N-($C_{1-3}$-Alkyl)-($C_{1-3}$-alkanamido)-Verbindungen mit Boran oder einem Borandimethylsulfid-Komplex in Tetrahydrofuran, wodurch die gewünschten Verbindungen der Formel (I) oder (E) hergestellt werden; oder

L.

(i) Umsetzen einer Verbindung der Formel (E) oder (G) mit einem $C_{1-5}$-Alkyllithium-Reagenz, wodurch durch Metallierung bzw. Lithium/Halogen-Austausch das entsprechende Lithium-Reagenz erhalten wird;

(ii) Zugabe eines Überschusses von Magnesiumhalogenidetherat zum Lithium-Reagenz, wodurch durch Transmetallierung das entsprechende Grignard-Reagenz erhalten wird;

(iii) Zugabe des Grignard-Reagenz zu einem N-Acylpyridiumsalz, wodurch die entsprechende Verbindung der Formel (F), wie vorstehend definiert, erhalten wird;

(iv) Deacylieren/Oxidieren der Verbindung der Formel (F), wodurch die entsprechende Verbindung der Formel (I) erhalten wird; oder

M. Behandeln des 3-Lithio-Derivats der Verbindung der Formel (E) oder (G), jeweils wie bereits definiert, mit Trialkylzinnchlorid, wodurch eine Verbindung der Formel (J) mit der nachstehenden Formel erhalten wird:

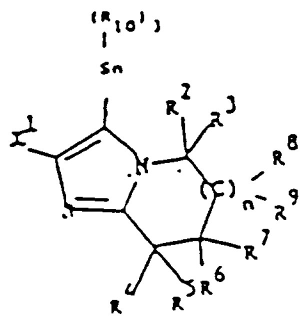

**FORMEL J**

in der
n 0 oder 1 ist;
$R^2$, $R^3$, $R^4$, $R^5$, $R^6$, $R^7$, $R^8$ und $R^9$ ein H-Atom sind oder einer oder zwei der Reste $R^2$, $R^3$, $R^4$, $R^5$, $R^6$, $R^7$, $R^8$ und $R^9$ unabhängig aus einem H-Atom oder einem $C_{1-2}$-Alkylrest gewählt sind;
$R_{10}$ ein $C_{1-4}$-Alkylrest ist;
und $X^1$ gewählt ist aus:

(a) einem monosubstituierten Phenylrest, wobei der Substituent gewählt ist aus einem H-, Fluor-, Chloratom, einem $C_{1-3}$-Alkoxy-, $C_{1-4}$-Alkyl-, $C_{1-3}$-Dialkylamino-Rest, einer $CF_3$-Gruppe, einem $C_{1-3}$-Alkylamino-Rest, einer N-Pyrrolidino-, N-Piperidino-, Prop-2-en-1-oxy- oder 2,2,2-Trihalogenethoxygruppe; oder

(b) einer Pyridylgruppe oder einem mit einem Alkylrest substituierten Pyridylrest; und Umsetzen der Verbindung der Formel (J) mit einem Gemisch aus einem Aryl- oder Heteroarylhalogenid oder -triflat und Tetrakis(triphosphin)palladium in einem Gemisch aus Tetrahydrofuran und Hexamethylphosphoramid, wodurch eine Verbindung der Formel (I) erhalten wird; oder

N. wenn eine Verbindung der Formel (I) hergestellt werden soll, in der einer der Reste R oder $R^1$ eine substituierter Phenylrest ist, der eine oder mehrere Alkylthiogruppen enthält: Behandeln der entsprechenden fluor-substituierten Phenylverbindung der Formel (I) mit einem Metallsalz des Alkylmercaptans in einem aprotischen polaren Lösungsmittel, wodurch die gewünschte Verbindung der Formel (I) erhalten wird; oder

O. wenn eine Verbindung der Formel (I) hergestellt werden soll, in der einer der Reste R oder $R^1$ ein mit einem Acyloxyalkylthiorest substituierter Phenylrest ist, wobei der Alkylrest gegebenenfalls mit einem $C_{1-4}$-Alkylrest substituiert ist: Behandeln einer Verbindung der Formel (I), wobei $R^1$ ein mit mindestens einem Alkylsulfinylrest substituierter Phenylrest ist, mit einem Alkansäureanhydrid, wodurch die gewünschte Verbindung der Formel (I) erhalten wird; oder

P. wenn eine Verbindung der Formel (I) hergestellt werden soll, wobei einer der Reste $R^1$ oder R eine mit mindestens einer Sulfhydrylgruppe substituierte Phenylgruppe ist: Hydrolyse des Produktes des vorstehend beschriebenen Verfahrens O, wodurch die gewünschten Verbindungen der Formel (I) erhalten werden; oder

Q. wenn eine Verbindung der Formel (I) hergestellt werden soll, wobei einer der Reste $R^1$ oder R eine Phenylgruppe ist, die mit mindestens einer Acylthio-, Dithioacyl-, Thiocarbamyl- oder Dithiocarbamylgruppe substituiert ist: Behandeln des Produktes des vorstehenden Verfahrens P mit einem Acylhalogenid, einem Alkansäureanhydrid, einem Thioacylhalogenid, einem Dithioalkansäureanhydrid, einem Dialkylcarbamylhalogenid oder einem Dialkylthiocarbamylhalogenid in Gegenwart einer Base, wie Pyridin, wodurch die gewünschten Verbindungen der Formel (I) erhalten werden; oder

R. wenn eine Verbindung der Formel (I) hergestellt werden soll, in der $R^1$ oder R ein mit einer Alkenylthiogruppe substituierter Phenylrest ist: Alkylieren einer Verbindung der Formel (I), worin einer der Reste $R^1$ oder R eine mit mindestens einer Sulfhydrylgruppe substituierte Phenylgruppe ist, mit einem geeignet substituierten Alkenylhalogenid, wie Allylbromid, wodurch Verbindungen der Formel (I) erhalten werden, in denen $R^1$ oder R ein mit mindestens einem Alkenylthiorest substituierter Phenylrest ist; oder

S. wenn eine Verbindung der Formel (I) hergestellt werden soll, wobei $R^1$ oder R ein mit einem Alkenylthiorest substituierter Phenylrest ist, wobei der Schwefel an das die Doppelbindung tragende

Kohlenstoffatom gebunden ist:

i) Behandeln des Mercaptoprodukts des vorstehenden Verfahrens P mit einer starken Base, wodurch die entsprechende Metallmercaptidsalzverbindung erhalten wird,

ii) Behandeln der Metallmercaptidsalzverbindung mit Trialkylsilylmethylchlorid, wodurch eine Verbindung der Formel (I) erhalten wird, in der der Phenylrest mindestens einen Trialkylsilylmethylsulfid-Substituenten aufweist,

iii) Behandeln der trialkylsilylmethylsulfid-substituierten Verbindung in einem aprotischen Lösungsmittel mit einem Reagenz zur Übertragung von Lithium gefolgt vom geeigneten aliphatischen Aldehyd oder Keton, wodurch eine Verbindung der Formel (I) erhalten wird, in der R oder $R^1$ ein mit mindestens einem Alkenylthiorest substituierter Phenylrest ist; oder

T. wenn eine Verbindung der Formel (I) hergestellt werden soll, wobei R oder $R^1$ ein alkylsubstituierter Pyridylrest ist: Deacylieren und Aromatisieren einer Verbindung der Formel (L)

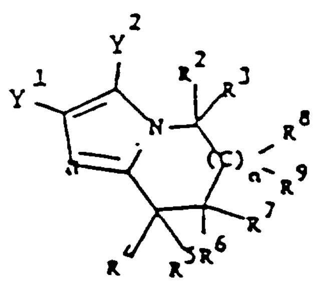

FORMEL (L)

in der

n 0 oder 1 ist;

$R^2$, $R^3$, $R^4$, $R^5$, $R^6$, $R^7$, $R^8$ und $R^9$ jeweils ein H-Atom sind oder einer oder zwei der Reste $R^2$, $R^3$, $R^4$, $R^5$, $R^6$, $R^7$, $R^8$ und $R^9$ unabhängig aus einem H-Atom oder einem $C_{1-2}$-Alkylrest gewählt sind;

einer der Reste $Y^1$ oder $Y^2$ unabhängig gewählt ist aus einem 4-[1,2-Dihydro-2-($C_{1-4}$-alkyl)]pyridyl-Rest, der substituiert ist mit einem N-($C_{1-8}$-Alkanoyl)-, N-($C_{1-8}$-Alkoxycarbonyl)-Rest, einer N-(Benzoyl)-, N-(Phenoxycarbonyl)-, N-(Phenylacetyl)- oder N-(Benzyloxycarbonyl)-Gruppe;

und der andere Rest gewählt ist aus:

(a) einem monosubstituierten Phenylrest, wobei der Substituent gewählt ist aus einem H-, Halogenatom, $C_{1-3}$-Alkoxy-, $C_{1-3}$-Alkylthio-, $C_{1-4}$-Alkyl-, N-($C_{1-3}$-Alkyl)-N-($C_{1-3}$-alkanamido)-, $C_{1-3}$-Dialkylamino-Rest, einer $CF_3$-, N-Pyrrolidino-, N-Piperidino-, Prop-2-en-1-oxy- oder 2,2,2-Trihalogenethoxy-Gruppe; oder

eines pharmazeutisch verträglichen Salzes davon mit Schwefel 15 Minuten unter Rückfluß von Decalin, Tetralin, p-Cymol oder Xylol mit Sauerstoffgas, wodurch die entsprechende Verbindung der Formel (I) erhalten wird.

**Patentansprüche für folgenden Vertragsstaat : ES**

1. Verfahren zur Herstellung einer Verbindung der Formel

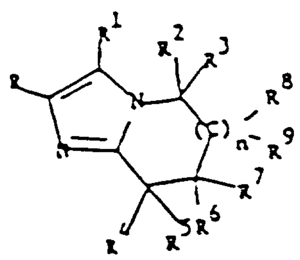

Formel (I)

in der:

1) einer der Reste R oder $R^1$ ein alkyl-substituierter Pyridylrest sein muß und der andere gewählt ist aus:
einem monosubstituierten Phenylrest, wobei der Substituent gewählt ist aus einem H-, Halogenatom, einer Hydroxylgruppe, einem $C_{1-3}$-Alkoxy-, $C_{1-3}$-Alkylthio-, $C_{1-4}$-Alkyl-, $C_{1-3}$-Alkylsulfinyl-, $C_{1-3}$-Alkylsulfonyl-, $C_{1-3}$-Alkylamino-, $C_{1-3}$-Dialkylaminorest, einer $CF_3$-Gruppe, einem N-($C_{1-3}$-Alkyl)-N-($C_{1-3}$-alkanamido)-Rest, einer N-Pyrrolidino-, N-Piperidino-, Prop-2-en-1-oxy- oder 2,2,2-Trihalogenethoxygruppe;

2) $R^1$ eine 4-Pyridylgruppe ist und R gewählt ist aus:
einem monosubstituierten Phenylrest, wobei der Substituent gewählt ist aus einem $C_{1-3}$-Alkylthio-, $C_{1-3}$-Alkylsulfinyl-, $C_{1-3}$-Alkylsulfonyl- oder einem verzweigten oder unverzweigten $C_{2-5}$-Alkenylthio- oder $C_{2-5}$-Alkenylsulfinylrest; oder

3) einer der Reste $R^1$ oder R eine Pyridylgruppe oder ein alkyl-substituierter Pyridylrest ist und der andere aus einem monosubstituierten oder disubstituierten Phenylrest gewählt ist, wobei der Substituent gewählt ist aus: einer Thiolgruppe [HS-], einem Acylthio- [AC(O)S-], Dithioacyl- [AC(S)S-], Dialkylthiocarbamyl- [$A_2$NC(O)S-], Dialkyldithiocarbamyl- [$A_2$NC(S)S-], Alkylcarbonylalkylthio- [AC(O)$CH_2$S-], Carbalkoxyalkylthio- [AOC(O)$CH_2$S-] oder Acyloxyalkylthiorest [AC(O)O$CH_2$S-], wobei die $CH_2$-Gruppe gegebenenfalls mit einem $C_{1-4}$-Alkylrest substituiert ist und A ein $C_{1-9}$-Alkylrest ist;

und $R^2$, $R^3$, $R^4$, $R^5$, $R^6$, $R^7$, $R^8$ und $R^9$ ein H-Atom sind, oder einer oder zwei der Reste $R^2$, $R^3$, $R^3$, $R^4$, $R^5$, $R^6$, $R^7$, $R^8$ und $R^9$ unabhängig aus einem H-Atom oder einem $C_{1-2}$-Alkylrest gewählt sind; und n 0 oder 1 ist;

oder eines pharmazeutisch verträglichen Salzes davon, umfassend:

A. wenn eine Verbindung der Formel (I) hergestellt werden soll, in der $R^1$ eine 4-Pyridylgruppe ist und R von einer Pyridylgruppe verschieden ist: Umsetzen einer Verbindung der Formel (E) mit der Strukturformel:

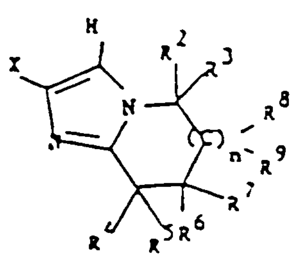

FORMEL (E)

in der

n 0 oder 1 ist;
$R^2$, $R^3$, $R^4$, $R^5$, $R^6$, $R^7$, $R^8$ und $R^9$ ein H-Atom sind oder einer oder zwei der Reste $R^2$, $R^3$, $R^4$, $R^5$, $R^6$, $R^7$, $R^8$ und $R^9$ unabhängig aus einem H-Atom oder einem $C_{1-2}$-Alkylrest gewählt sind;
X ein $C_{1-3}$-Alkylthiophenylrest ist;
in Pyridin mit einem Aroylhalogenid oder einem Halogenameisensäurearylalkylester oder einem Halogenameisensäurealkylester oder mit dem vorher hergestellten Acylpyridiniumsalz, wodurch eine Verbindung der Formel (F) mit der Strukturformel

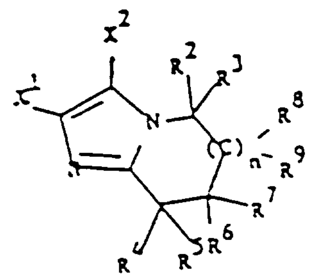

FORMEL (F)

erhalten wird,
in der
n 0 oder 1 ist;
$R^2$, $R^3$, $R^4$, $R^5$, $R^6$, $R^7$, $R^8$ und $R^9$ ein H-Atom sind oder einer oder zwei der Reste $R^2$, $R^3$, $R^4$, $R^5$, $R^6$, $R^7$, $R^8$ und $R^9$ unabhängig aus einem H-Atom oder einem $C_{1-2}$-Alkylrest gewählt sind;
$X^1$ ein mit einem $C_{1-3}$-Alkylthiorest monosubstituierter Phenylrest ist; und
$X^2$ ein 4-(1,4-Dihydro)pyridyl-Rest ist, der substituiert ist mit einem N-($C_{1-8}$-Alkanoyl)-, N-($C_{1-8}$-Alkoxycarbonyl)-Rest, einer N-(Benzoyl)-, N-(Phenoxycarbonyl)-, N-(Phenylacetyl)- oder N-(Benzyloxycarbonyl)-Gruppe;
gefolgt von Deacylierung/Oxidation der Verbindung der Formel (F), wodurch eine Verbindung der Formel (I) erhalten wird; oder
B. wenn eine Hydroxyverbindung der Formel (I) oder (E) hergestellt werden soll: Demethylieren einer Methoxyphenylverbindung der Formel (E) oder (I), wobei die Formel (E) wie vorstehend definiert ist, außer daß X eine Methoxyphenylgruppe ist und die Formel (I) durch die Strukturformel dargestellt wird:

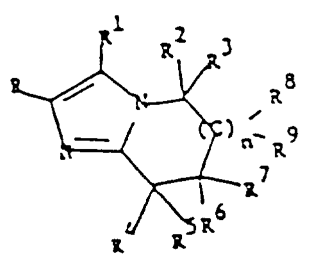

FORMEL (I)

in der
n 0 oder 1 ist;
$R^2$, $R^3$, $R^4$, $R^5$, $R^6$, $R^7$, $R^8$ und $R^9$ ein H-Atom sind oder einer oder zwei der Reste $R^2$, $R^3$, $R^4$, $R^5$, $R^6$, $R^7$, $R^8$ und $R^9$ unabhängig aus einem H-Atom oder einem $C_{1-2}$-Alkylrest gewählt sind;
einer der Reste $R^1$ und R ein alkyl-substituierter Pyridylrest und der andere eine Methoxyphenylgruppe ist;

oder eines pharmazeutisch verträglichen Salzes davon; mit HBr in Essigsäure oder $BBr_3$ in Methylenchlorid, wodurch die entsprechende Hydroxyverbindung der Formel (I) oder (E) erhalten

wird; oder

C. O-Alkylieren der Hydroxyverbindung der Formel (I) oder (E), wie vorstehend definiert, wenn einer der Reste $R^1$ und R für (I), oder X für (E) eine Phenylgruppe ist, die mit mindestens einer Hydroxylgruppe substituiert ist, wodurch die entsprechende $C_{1-3}$-alkoxyphenyl-, 2,2,2-trihalogenethoxyphenyl-, oder prop-2-en-1-oxy-phenyl-substituierte Verbindung der Formel (I) oder (E) erhalten wird; oder

D. Acylieren einer Aminophenyl- oder ($C_{1-3}$-Alkylamino)phenyl-Verbindung der Formel (I) mit dem entsprechend substituierten Acylhalogenid oder -anhydrid in Pyridin, wodurch die entsprechende N-($C_{1-3}$-Alkyl)-($C_{1-3}$-alkanamido)phenyl-Verbindung der Formel (I) erhalten wird; oder

E. Alkylieren einer ($C_{1-3}$-Alkanamido)phenyl-Verbindung der Formel (I) oder (E), wie bereits definiert, mit einem Alkylierungsmittel in Gegenwart einer Base, wodurch die entsprechende N-($C_{1-3}$-Alkanamido)phenyl- oder N-($C_{1-3}$-Alkyl)-($C_{1-3}$-alkanamido)phenyl-Verbindung der Formel (I) oder (E) erhalten wird; oder

F. Hydrolysieren einer ($C_{1-3}$-Alkanamido)phenyl- oder N-($C_{1-3}$-Alkyl)-($C_{1-3}$-alkanamido)phenyl-Verbindung der Formel (I) oder (E), wie bereits definiert, oder der Formel (G) mit der Strukturformel:

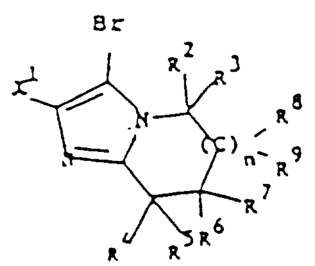

FORMEL (G)

in der

n 0 oder 1 ist;

$R^2$, $R^3$, $R^4$, $R^5$, $R^6$, $R^7$, $R^8$ und $R^9$ ein H-Atom sind oder einer oder zwei der Reste $R^2$, $R^3$, $R^4$, $R^5$, $R^6$, $R^7$, $R^8$ und $R^9$ unabhängig aus einem H-Atom oder einem $C_{1-2}$-Alkylrest gewählt sind;

$X^1$ ein ($C_{1-3}$-Alkanamido)phenyl- oder N-($C_{1-3}$-Alkyl)-($C_{1-3}$-alkanamido)phenyl-Rest ist,

wodurch die entsprechende Aminophenyl- oder ($C_{1-3}$-Alkylamino)phenyl-Verbindung der Formel (I), (E) oder (G) erhalten wird; oder

G. Oxidieren einer Verbindung der Formel (I), wenn einer der Reste $R^1$ oder R ein Phenylrest ist, der mit einem oder mehreren der Reste aus $C_{1-3}$-Alkylthio-, $C_{1-3}$-Alkylsulfinyl-, $C_{1-3}$-Alkenylthio-, oder Acyloxyalkylthio-Rest substituiert ist, mit einem Oxidationsmittel, wodurch die entsprechende $C_{1-3}$-Alkylsulfinyl-, $C_{1-3}$-Alkylsulfonyl-, Acyloxyalkylsulfinyl- oder $C_{1-3}$-Alkenylsulfinyl-Verbindung der Formel (I) erhalten wird; oder

H. Reduzieren einer Verbindung der Formel (E) oder (I), wie bereits definiert, wobei X der Formel (E) oder einer der Reste R und $R^1$ der Formel (I) ein mono- oder disubstituierter Phenylrest ist, der eine Nitrogruppe enthält, mit Wasserstoffgas über einem Katalysator, wodurch die entsprechende Aminophenylverbindung der Formel (I) oder (E) erhalten wird; oder

I. Cycloalkylieren einer Verbindung der Formel (I) oder (E), wie bereits definiert, wobei einer der Reste $R^1$ und R der Formel (I) oder X der Formel (E) eine Aminophenylgruppe ist, mit einem geeigneten Dihalogenbutan oder Dihalogenpentan in Gegenwart einer Base, wodurch die entsprechende N-Piperidino- oder N-Pyrrolidino-Verbindung der Formel (I) oder (E) erhalten wird; oder

J. wenn Verbindungen der Formel (I) oder (E), wie bereits definiert, hergestellt werden sollen, wobei $R^1$ und R der Formel (I) oder X der Formel (E) ein ($C_{1-3}$-Alkylamino)phenylrest ist: Reduzieren der entsprechenden $C_{1-3}$-Alkanamido-Verbindungen mit Boran oder einem Borandimethylsulfid-Komplex in Tetrahydrofuran, wodurch die gewünschten Verbindungen der Formel (I) oder (E) erhalten werden; oder

K. wenn Verbindungen der Formel (I) oder (E), wie bereits definiert, hergestellt werden sollen, wobei einer der Reste $R^1$ und R der Formel (I) oder X der Formel (E) ein ($C_{1-3}$-Dialkylamino)phenyl-Rest ist: Reduzieren der entsprechenden N-($C_{1-3}$-Alkyl)-($C_{1-3}$-alkanamido)-Verbindungen mit Boran oder einem Borandimethylsulfid-Komplex in Tetrahydrofuran, wodurch die gewünschten Verbindungen der

Formel (I) oder (E) hergestellt werden; oder

L.

(i) Umsetzen einer Verbindung der Formel (E) oder (G) mit einem $C_{1-5}$-Alkyllithium-Reagenz, wodurch durch Metallierung bzw. Lithium/Halogen-Austausch das entsprechende Lithium-Reagenz erhalten wird;

(ii) Zugeben eines Überschusses von Magnesiumhalogenidetherat zum Lithium-Reagenz, wodurch durch Transmetallierung das entsprechende Grignard-Reagenz erhalten wird;

(iii) Zugeben des Grignard-Reagenz zu einem N-Acylpyridiumsalz, wodurch die entsprechende Verbindung der Formel (F), wie vorstehend definiert, erhalten wird;

(iv) Deacylieren/Oxidieren der Verbindung der Formel (F), wodurch die entsprechende Verbindung der Formel (I) erhalten wird; oder

M. Behandeln des 3-Lithio-Derivats der Verbindung der Formel (E) oder (G), jeweils wie bereits definiert, mit Trialkylzinnchlorid, wodurch eine Verbindung der Formel (J) mit der nachstehenden Formel erhalten wird:

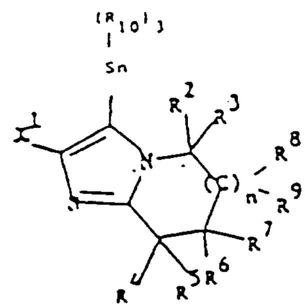

FORMEL J

in der

n 0 oder 1 ist;

$R^2$, $R^3$, $R^4$, $R^5$, $R^6$, $R^7$, $R^8$ und $R^9$ ein H-Atom sind oder einer oder zwei der Reste $R^2$, $R^3$, $R^4$, $R^5$, $R^6$, $R^7$, $R^8$ und $R^9$ unabhängig aus einem H-Atom oder einem $C_{1-2}$-Alkylrest gewählt sind;

$R_{10}$ ein $C_{1-4}$-Alkylrest ist;

und $X^1$ gewählt ist aus:

(a) einem monosubstituierten Phenylrest, wobei der Substituent gewählt ist aus einem H-, Fluor-, Chloratom, einem $C_{1-3}$-Alkoxy-, $C_{1-4}$-Alkyl-, $C_{1-3}$-Dialkylamino-Rest, einer $CF_3$-Gruppe, einem $C_{1-3}$-Alkylamino-Rest, einer N-Pyrrolidino-, N-Piperidino-, Prop-2-en-1-oxy- oder 2,2,2-Trihalogenethoxygruppe; oder

(b) einer Pyridylgruppe oder einem mit einem Alkylrest substituierten Pyridylrest;

und Umsetzen der Verbindung der Formel (J) mit einem Gemisch aus einem Aryl- oder Heteroarylhalogenid oder -triflat und Tetrakis(triphosphin)palladium in einem Gemisch aus Tetrahydrofuran und Hexamethylphosphoramid, wodurch eine Verbindung der Formel (I) erhalten wird; oder

N. wenn eine Verbindung der Formel (I) hergestellt werden soll, in der einer der Reste R oder $R^1$ eine substituierter Phenylrest ist, der eine oder mehrere Alkylthiogruppen enthält: Behandeln der entsprechenden fluor-substituierten Phenylverbindung der Formel (I) mit einem Metallsalz des Alkylmercaptans in einem aprotischen polaren Lösungsmittel, wodurch die gewünschte Verbindung der Formel (I) erhalten wird; oder

O. wenn eine Verbindung der Formel (I) hergestellt werden soll, in der einer der Reste R oder $R^1$ ein mit einem Acyloxyalkylthiorest substituierter Phenylrest ist, wobei der Alkylrest gegebenenfalls mit einem $C_{1-4}$-Alkylrest substituiert ist: Behandeln einer Verbindung der Formel (I), wobei $R^1$ ein mit mindestens einem Alkylsulfinylrest substituierter Phenylrest ist, mit einem Alkansäureanhydrid, wodurch die gewünschte Verbindung der Formel (I) erhalten wird; oder

P. wenn eine Verbindung der Formel (I) hergestellt werden soll, wobei einer der Reste $R^1$ oder R eine mit mindestens einer Sulfhydrylgruppe substituierte Phenylgruppe ist: Hydrolyse des Produktes des vorstehend beschriebenen Verfahrens O, wodurch die gewünschten Verbindungen der Formel (I)

erhalten werden; oder

Q. wenn eine Verbindung der Formel (I) hergestellt werden soll, wobei einer der Reste $R^1$ oder R eine Phenylgruppe ist, die mit mindestens einer Acylthio-, Dithioacyl-, Thiocarbamyl- oder Dithiocarbamylgruppe substituiert ist: Behandeln des Produktes des vorstehenden Verfahrens P mit einem Acylhalogenid, einem Alkansäureanhydrid, einem Thioacylhalogenid, einem Dithioalkansäureanhydrid, einem Dialkylcarbamylhalogenid oder einem Dialkylthiocarbamylhalogenid in Gegenwart einer Base, wie Pyridin, wodurch die gewünschten Verbindungen der Formel (I) erhalten werden; oder

R. wenn eine Verbindung der Formel (I) hergestellt werden soll, in der $R^1$ oder R ein mit einer Alkenylthiogruppe substituierter Phenylrest ist: Alkylieren einer Verbindung der Formel (I), worin einer der Reste $R^1$ oder R eine mit mindestens einer Sulfhydrylgruppe substituierte Phenylgruppe ist, mit einem geeignet substituierten Alkenylhalogenid, wie Allylbromid, wodurch Verbindungen der Formel (I) erhalten werden, in denen $R^1$ oder R ein mit mindestens einem Alkenylthiorest substituierter Phenylrest ist; oder

S. wenn eine Verbindung der Formel (I) hergestellt werden soll, wobei $R^1$ oder R ein mit einem Alkenylthiorest substituierter Phenylrest ist, wobei der Schwefel an das die Doppelbindung tragende Kohlenstoffatom gebunden ist:

i) Behandeln des Mercaptoprodukts des vorstehenden Verfahrens P mit einer starken Base, wodurch die entsprechende Metallmercaptidsalzverbindung erhalten wird,

ii) Behandeln der Metallmercaptidsalzverbindung mit Trialkylsilylmethylchlorid, wodurch eine Verbindung der Formel (I) erhalten wird, in der der Phenylrest mindestens einen Trialkylsilylmethylsulfid-Substituenten aufweist,

iii) Behandeln der trialkylsilylmethylsulfid-substituierten Verbindung in einem aprotischen Lösungsmittel mit einem Reagenz zur Übertragung von Lithium gefolgt vom geeigneten aliphatischen Aldehyd oder Keton, wodurch eine Verbindung der Formel (I) erhalten wird, in der R oder $R^1$ ein mit mindestens einem Alkenylthiorest substituierter Phenylrest ist; oder

T. wenn eine Verbindung der Formel (I) hergestellt werden soll, wobei R oder $R^1$ ein alkylsubstituierter Pyridylrest ist: Deacylieren und Aromatisieren einer Verbindung der Formel (L)

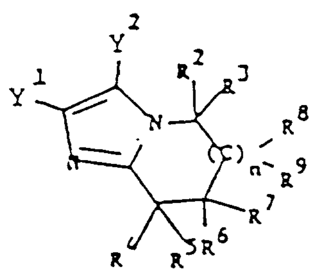

**FORMEL (L)**

in der

n 0 oder 1 ist;

$R^2$, $R^3$, $R^4$, $R^5$, $R^6$, $R^7$, $R^8$ und $R^9$ jeweils ein H-Atom sind oder einer oder zwei der Reste $R^2$, $R^3$, $R^4$, $R^5$, $R^6$, $R^7$, $R^8$ und $R^9$ unabhängig aus einem H-Atom oder einem $C_{1-2}$-Alkylrest gewählt sind;

einer der Reste $Y^1$ oder $Y^2$ unabhängig gewählt ist aus einem 4-[1,2-Dihydro-2-($C_{1-4}$-alkyl)]pyridyl-Rest, der substituiert ist mit einem N-($C_{1-8}$-Alkanoyl)-, N-($C_{1-8}$-Alkoxycarbonyl)-Rest, einer N-(Benzoyl)-, N-(Phenoxycarbonyl)-, N-(Phenylacetyl)- oder N-(Benzyloxycarbonyl)-Gruppe;

und der andere Rest gewählt ist aus:

(a) einem monosubstituierten Phenylrest, wobei der Substituent gewählt ist aus einem H-, Halogenatom, $C_{1-3}$-Alkoxy-, $C_{1-3}$-Alkylthio-, $C_{1-4}$-Alkyl-, N-($C_{1-3}$-Alkyl)-N-($C_{1-3}$-alkanamido)-, $C_{1-3}$-Dialkylamino-Rest, einer $CF_3$-, N-Pyrrolidino-, N-Piperidino-, Prop-2-en-1-oxy- oder 2,2,2-Trihalogenethoxy-Gruppe; oder

eines pharmazeutisch verträglichen Salzes davon mit Schwefel 15 Minuten unter Rückfluß von Decalin, Tetralin, p-Cymol oder Xylol mit Sauerstoffgas, wodurch die entsprechende Verbindung der Formel (I) erhalten wird.

2. Verfahren nach Anspruch 1, wobei die Verbindung der Formel (I) 2-(4-Methylthiophenyl)-3-(4-pyridyl)-6,7-dihydro-[5H]-pyrrolo[1,2-a]imidazol oder ein pharmazeutisch verträgliches Salz davon ist.

3. Verfahren nach Anspruch 1, wobei die Verbindung der Formel (I) 2-(4-Methylsulfinylphenyl)-3-(4-pyridyl)-6,7-dihydro-[5H]-pyrrolo[1,2-a]imidazol oder ein pharmazeutisch verträgliches Salz davon ist.

4. Verfahren nach Anspruch 1, das die Herstellung einer Verbindung der Formel (I) umfaßt, nämlich:
2-(4-Methylsulfonylphenyl)-3-(4-pyridyl)-6,7-dihydro-[5H]-pyrrolo[1,2-a]imidazol,
2-(4-Methoxyphenyl)-3-(2,6-dimethyl-4-pyridyl)-6,7-dihydro-[5H]-pyrrolo[1,2-a]imidazol,
2-(4-Ethylthiophenyl)-3-(4-pyridyl)-6,7-dihydro-[5H]-pyrrolo[1,2-a]imidazol,
2-(4-Ethylsulfinylphenyl)-3-(4-pyridyl)-6,7-dihydro-[5H]-pyrrolo[1,2-a]imidazol,
2-(4-Mercaptophenyl)-3-(4-pyridyl)-6,7-dihydro-[5H]-pyrrolo[1,2-a]imidazol,
2-(4-Trimethylacetylthiophenyl)-3-(4-pyridyl)-6,7-dihydro-[5H]-pyrrolo[1,2-a]imidazol,
2-(4-Acetylthiophenyl)-3-(4-pyridyl)-6,7-dihydro-[5H]-pyrrolo[1,2-a]imidazol,
2-(4-Ethylsulfonylphenyl)-3-(4-pyridyl)-6,7-dihydro-[5H]-pyrrolo[1,2-a]imidazol,
2-(4-Fluorphenyl)-3-[4-(2-methyl)pyridyl]-6,7-dihydro-[5H]-pyrrolo[1,2-a]imidazol,
2-(4-Methylthiophenyl)-3-[4-(2-methyl)pyridyl]-6,7-dihydro-[5H]-pyrrolo[1,2-a]imidazol,
2-(4-Methylsulfinylphenyl)-3-[4-(2-methyl)pyridyl]-6,7-dihydro-[5H]-pyrrolo[1,2-a]imidazol,
2-(4-Carbethoxymethylthiophenyl)-3-(4-pyridyl)-6,7-dihydro-[5H]-pyrrolo[1,2-a]imidazol, oder
2-(4-Acetoxymethylthiophenyl)-3-(4-pyridyl)-6,7-dihydro-[5H]-pyrrolo[1,2-a]imidazol.

5. Verfahren zur Herstellung einer Verbindung der Formel (I), nämlich 2-(4-Methylthiophenyl)-3-(4-pyridyl)-6,7-dihydro-[5H]-pyrrolo[1,2-a]imidazol, umfassend die Behandlung der entsprechenden fluor-substituierten Phenylverbindung der Formel (I) nach Anspruch 1 mit 1,2 Äquivalenten eines Metallsalzes von Alkylmercaptan in einem aprotischen polaren Lösungsmittel.

6. Verfahren zur Herstellung einer Verbindung der Formel (I), nämlich 2-(4-Methylsulfinylphenyl)-3-(4-pyridyl)-6,7-dihydro-[5H]-pyrrolo[1,2-a]imidazol, umfassend die Oxidation der entsprechenden 4-Methylthiophenylverbindung mit einem geeigneten Oxidationsmittel.

7. Verfahren zur Herstellung eines Arzneimittels, das eine Verbindung der Formel (I) nach einem der Ansprüche 1 bis 4 enthält, wobei das Verfahren das Verbinden der Verbindung mit einem pharmazeutisch verträglichen Träger umfaßt.

8. Verfahren zur Herstellung einer Verbindung der Formel (L) mit nachstehender Strukturformel

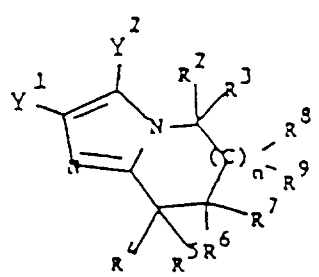

FORMEL (L)

in der
n 0 oder 1 ist,
$R^2$, $R^3$, $R^4$, $R^5$, $R^6$, $R^7$, $R^8$, $R^9$ alle ein H-Atom sind, oder einer oder zwei der Reste $R^2$, $R^3$, $R^4$, $R^5$, $R^6$, $R^7$, $R^8$ und $R^9$ unabhängig aus einem H-Atom oder einem $C_{1-2}$-Alkylrest gewählt sind;
einer der Reste $Y^1$ oder $Y^2$ unabhängig aus einem 4-[1,2-Dihydro-2-($C_{1-4}$-alkyl]pyridylrest gewählt ist, der mit einem N-($C_{1-8}$-Alkanoyl)-, N-($C_{1-8}$-Alkoxycarbonyl)-Rest, einer N-(Benzoyl)-, N-(Phenoxycarbonyl)-, N-(Phenylacetyl)- oder N-(Benzyloxycarbonyl)-Gruppe substituiert ist;
und der andere gewählt ist aus:

(a) einem monosubstituierten Phenylrest, wobei der Substituent aus einem H-, einem Halogenatom, einem $C_{1-3}$-Alkoxy-, $C_{1-3}$-Alkylthio-, $C_{1-4}$-Alkyl-, N-($C_{1-3}$-Alkyl)-N-($C_{1-3}$-alkanamido)-, $C_{1-3}$-Dialkylamino-Rest, einer $CF_3$-, N-Pyrrolidino-, N-Piperidino-, Prop-2-en-1-oxy- oder 2,2,2-Trihalogenethoxy-Gruppe gewählt ist; oder eines pharmazeutisch verträglichen Salzes davon, umfassend die Behandlung einer Verbindung der Formel (I) nach Anspruch 1 mit einem Acylhalogenid, Aroylhalogenid, Arylalkylhalogenformiatester oder Alkylhalogenformiatester und einem $C_{1-4}$-Alkyl-Grignard-Reagenz.

**9.** Verfahren zur Herstellung einer Verbindung der Formel (J) mit nachstehender Strukturformel

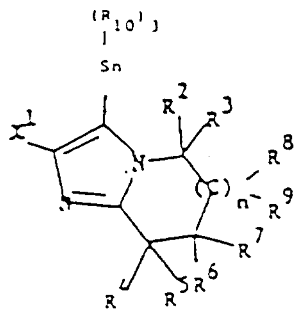

FORMEL (J)

in der
n 0 oder 1 ist;
$R^2$, $R^3$, $R^4$, $R^5$, $R^6$, $R^7$, $R^8$ und $R^9$ jeweils ein H-Atom sind oder einer oder zwei der Reste $R^2$, $R^3$, $R^4$, $R^5$, $R^6$, $R^7$, $R^8$ und $R^9$ unabhängig aus einem H-Atom oder einer $C_{1-2}$-Alkylgruppe gewählt ist;
$R_{10}$ ein $C_{1-4}$-Alkylrest ist;
und $X^1$ gewählt ist aus:

(a) einem monosubstituierten Phenylrest, wobei der Substituent aus einem H-, Fluor-, Chloratom, einem $C_{1-3}$-Alkoxy-, $C_{1-4}$-Alkyl-, $C_{1-3}$-Dialkylaminorest, einer $CF_3$-Gruppe, einem $C_{1-3}$-Alkylaminorest, einer N-Pyrrolidino-, N-Piperidino-, Prop-2-en-1-oxy- oder 2,2,2-Trihalogenethoxygruppe gewählt ist;
(b) einer Pyridylgruppe oder einem alkyl-substituierten Pyridylrest;
oder eines pharmazeutisch verträglichen Salzes davon, umfassend die Umsetzung eines 3-Lithio-Derivats einer Verbindung der Formel (E) nach Anspruch 1 mit Trialkylzinnchlorid.

**Patentansprüche für folgenden Vertragsstaat : GR**

**1.** Verfahren zur Herstellung einer Verbindung der Formel

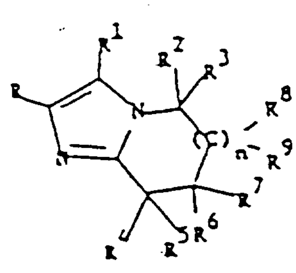

Formel (I)

in der:
1) einer der Reste R oder $R^1$ ein alkyl-substituierter Pyridylrest sein muß und der andere gewählt ist aus:
einem monosubstituierten Phenylrest, wobei der Substituent gewählt ist aus einem H-, Halogenatom,

einer Hydroxylgruppe, einem $C_{1-3}$-Alkoxy-, $C_{1-3}$-Alkylthio-, $C_{1-4}$-Alkyl-, $C_{1-3}$-Alkylsulfinyl-, $C_{1-3}$-Alkylsulfonyl-, $C_{1-3}$-Alkylamino-, $C_{1-3}$-Dialkylaminorest, einer $CF_3$-Gruppe, einem N-($C_{1-3}$-Alkyl)-N-($C_{1-3}$-alkanamido)-Rest, einer N-Pyrrolidino-, N-Piperidino-, Prop-2-en-1-oxy- oder 2,2,2-Trihalogenethoxygruppe;

2) $R^1$ eine 4-Pyridylgruppe ist und R gewählt ist aus:

einem monosubstituierten Phenylrest, wobei der Substituent gewählt ist aus einem $C_{1-3}$-Alkylthio-, $C_{1-3}$-Alkylsulfinyl-, $C_{1-3}$-Alkylsulfonyl- oder einem verzweigten oder unverzweigten $C_{2-5}$-Alkenylthio- oder $C_{2-5}$-Alkenylsulfinylrest; oder

3) einer der Reste $R^1$ oder R eine Pyridylgruppe oder ein alkyl-substituierter Pyridylrest ist und der andere aus einem monosubstituierten oder disubstituierten Phenylrest gewählt ist, wobei der Substituent gewählt ist aus: einer Thiolgruppe [HS-], einem Acylthio- [AC(O)S-], Dithioacyl- [AC(S)S-], Dialkylthiocarbamyl- [$A_2$NC(O)S-], Dialkyldithiocarbamyl- [$A_2$NC(S)S-], Alkylcarbonylalkylthio- [AC(O)$CH_2$S-], Carbalkoxyalkylthio- [AOC(O)$CH_2$S-] oder Acyloxyalkylthiorest [AC(O)O$CH_2$S-], wobei die $CH_2$-Gruppe gegebenenfalls mit einem $C_{1-4}$-Alkylrest substituiert ist und A ein $C_{1-9}$-Alkylrest ist;

und $R^2$, $R^3$, $R^4$, $R^5$, $R^6$, $R^7$, $R^8$ und $R^9$ ein H-Atom sind, oder einer oder zwei der Reste $R^2$, $R^3$, $R^3$, $R^4$, $R^5$, $R^6$, $R^7$, $R^8$ und $R^9$ unabhängig aus einem H-Atom oder einem $C_{1-2}$-Alkylrest gewählt sind; und n 0 oder 1 ist;

oder eines pharmazeutisch verträglichen Salzes davon, umfassend:

A. wenn eine Verbindung der Formel (I) hergestellt werden soll, in der $R^1$ eine 4-Pyridylgruppe ist und R von einer Pyridylgruppe verschieden ist: Umsetzen einer Verbindung der Formel (E) mit der Strukturformel:

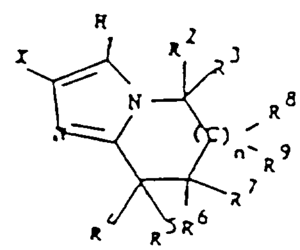

FORMEL (E)

in der

n 0 oder 1 ist;

$R^2$, $R^3$, $R^4$, $R^5$, $R^6$, $R^7$, $R^8$ und $R^9$ ein H-Atom sind oder einer oder zwei der Reste $R^2$, $R^3$, $R^4$, $R^5$, $R^6$, $R^7$, $R^8$ und $R^9$ unabhängig aus einem H-Atom oder einem $C_{1-2}$-Alkylrest gewählt sind;

X ein $C_{1-3}$-Alkylthiophenylrest ist;

in Pyridin mit einem Aroylhalogenid oder einem Halogenameisensäurearylalkylester oder einem Halogenameisensäurealkylester oder mit dem vorher hergestellten Acylpyridiniumsalz, wodurch eine Verbindung der Formel (F) mit der Strukturformel

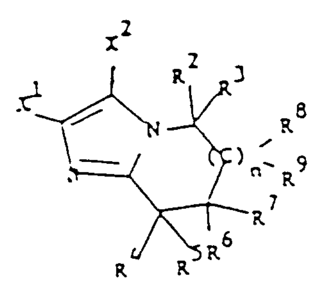

FORMEL (F)

erhalten wird,
in der
n 0 oder 1 ist;
$R^2$, $R^3$, $R^4$, $R^5$, $R^6$, $R^7$, $R^8$ und $R^9$ ein H-Atom sind oder einer oder zwei der Reste $R^2$, $R^3$, $R^4$, $R^5$, $R^6$, $R^7$, $R^8$ und $R^9$ unabhängig aus einem H-Atom oder einem $C_{1-2}$-Alkylrest gewählt sind;
$X^1$ ein mit einem $C_{1-3}$-Alkylthiorest monosubstituierter Phenylrest ist; und
$X^2$ ein 4-(1,4-Dihydro)pyridyl-Rest ist, der substituiert ist mit einem N-($C_{1-8}$-Alkanoyl)-, N-($C_{1-8}$-Alkoxycarbonyl)-Rest, einer N-(Benzoyl)-, N-(Phenoxycarbonyl)-, N-(Phenylacetyl)- oder N-(Benzyloxycarbonyl)-Gruppe;
gefolgt von Deacylierung/Oxidation der Verbindung der Formel (F), wodurch eine Verbindung der Formel (I) erhalten wird; oder
B. wenn eine Hydroxyverbindung der Formel (I) oder (E) hergestellt werden soll: Demethylieren einer Methoxyphenylverbindung der Formel (E) oder (I), wobei die Formel (E) wie vorstehend definiert ist, außer daß X eine Methoxyphenylgruppe ist und die Formel (I) durch die Strukturformel dargestellt wird:

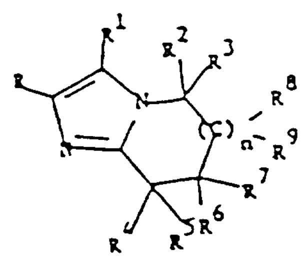

FORMEL (I)

in der
n 0 oder 1 ist;
$R^2$, $R^3$, $R^4$, $R^5$, $R^6$, $R^7$, $R^8$ und $R^9$ ein H-Atom sind oder einer oder zwei der Reste $R^2$, $R^3$, $R^4$, $R^5$, $R^6$, $R^7$, $R^8$ und $R^9$ unabhängig aus einem H-Atom oder einem $C_{1-2}$-Alkylrest gewählt sind;
einer der Reste $R^1$ und R ein alkyl-substituierter Pyridylrest und der andere eine Methoxyphenylgruppe ist;
oder eines pharmazeutisch verträglichen Salzes davon; mit HBr in Essigsäure oder $BBr_3$ in Methylenchlorid, wodurch die entsprechende Hydroxyverbindung der Formel (I) oder (E) erhalten wird; oder
C. O-Alkylieren der Hydroxyverbindung der Formel (I) oder (E), wie vorstehend definiert, wenn einer der Reste $R^1$ und R für (I), oder X für (E) eine Phenylgruppe ist, die mit mindestens einer Hydroxylgruppe substituiert ist, wodurch die entsprechende $C_{1-3}$-alkoxyphenyl-, 2,2,2-trihalogenethoxyphenyl-, oder prop-2-en-1-oxy-phenyl-substituierte Verbindung der Formel (I) oder (E) erhalten wird; oder
D. Acylieren einer Aminophenyl- oder ($C_{1-3}$-Alkylamino)phenyl-Verbindung der Formel (I) mit dem entsprechend substituierten Acylhalogenid oder -anhydrid in Pyridin, wodurch die entsprechende N-($C_{1-3}$-Alkyl)-($C_{1-3}$-alkanamido)phenyl-Verbindung der Formel (I) erhalten wird; oder
E. Alkylieren einer ($C_{1-3}$-Alkanamido)phenyl-Verbindung der Formel (I) oder (E), wie bereits definiert, mit einem Alkylierungsmittel in Gegenwart einer Base, wodurch die entsprechende N-($C_{1-3}$-Alkanamido)phenyl- oder N-($C_{1-3}$-Alkyl)-($C_{1-3}$-alkanamido)phenyl-Verbindung der Formel (I) oder (E) erhalten wird; oder
F. Hydrolysieren einer ($C_{1-3}$-Alkanamido)phenyl- oder N-($C_{1-3}$-Alkyl)-($C_{1-3}$-alkanamido)phenyl-Verbindung der Formel (I) oder (E), wie bereits definiert, oder der Formel (G) mit der Strukturformel:

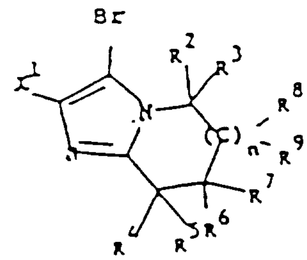

FORMEL (G)

in der
n 0 oder 1 ist;
$R^2$, $R^3$, $R^4$, $R^5$, $R^6$, $R^7$, $R^8$ und $R^9$ ein H-Atom sind oder einer oder zwei der Reste $R^2$, $R^3$, $R^4$, $R^5$, $R^6$, $R^7$, $R^8$ und $R^9$ unabhängig aus einem H-Atom oder einem $C_{1-2}$-Alkylrest gewählt sind;
$X^1$ ein ($C_{1-3}$-Alkanamido)phenyl- oder N-($C_{1-3}$-Alkyl)-($C_{1-3}$-alkanamido)phenyl-Rest ist,
wodurch die entsprechende Aminophenyl- oder ($C_{1-3}$-Alkylamino)phenyl-Verbindung der Formel (I), (E) oder (G) erhalten wird; oder

G. Oxidieren einer Verbindung der Formel (I), wenn einer der Reste $R^1$ oder R ein Phenylrest ist, der mit einem oder mehreren der Reste aus $C_{1-3}$-Alkylthio-, $C_{1-3}$-Alkylsulfinyl-, $C_{1-3}$-Alkenylthio-, oder Acyloxyalkylthio-Rest substituiert ist, mit einem Oxidationsmittel, wodurch die entsprechende $C_{1-3}$-Alkylsulfinyl-, $C_{1-3}$-Alkylsulfonyl-, Acyloxyalkylsulfinyl- oder $C_{1-3}$-Alkenylsulfinyl-Verbindung der Formel (I) erhalten wird; oder

H. Reduzieren einer Verbindung der Formel (E) oder (I), wie bereits definiert, wobei X der Formel (E) oder einer der Reste R und $R^1$ der Formel (I) ein mono- oder disubstituierter Phenylrest ist, der eine Nitrogruppe enthält, mit Wasserstoffgas über einem Katalysator, wodurch die entsprechende Aminophenylverbindung der Formel (I) oder (E) erhalten wird; oder

I. Cycloalkylieren einer Verbindung der Formel (I) oder (E), wie bereits definiert, wobei einer der Reste $R^1$ und R der Formel (I) oder X der Formel (E) eine Aminophenylgruppe ist, mit einem geeigneten Dihalogenbutan oder Dihalogenpentan in Gegenwart einer Base, wodurch die entsprechende N-Piperidino- oder N-Pyrrolidino-Verbindung der Formel (I) oder (E) erhalten wird; oder

J. wenn Verbindungen der Formel (I) oder (E), wie bereits definiert, hergestellt werden sollen, wobei $R^1$ und R der Formel (I) oder X der Formel (E) ein ($C_{1-3}$-Alkylamino)phenylrest ist: Reduzieren der entsprechenden $C_{1-3}$-Alkanamido-Verbindungen mit Boran oder einem Borandimethylsulfid-Komplex in Tetrahydrofuran, wodurch die gewünschten Verbindungen der Formel (I) oder (E) erhalten werden; oder

K. wenn Verbindungen der Formel (I) oder (E), wie bereits definiert, hergestellt werden sollen, wobei einer der Reste $R^1$ und R der Formel (I) oder X der Formel (E) ein ($C_{1-3}$-Dialkylamino)phenyl-Rest ist: Reduzieren der entsprechenden N-($C_{1-3}$-Alkyl)-($C_{1-3}$-alkanamido)-Verbindungen mit Boran oder einem Borandimethylsulfid-Komplex in Tetrahydrofuran, wodurch die gewünschten Verbindungen der Formel (I) oder (E) hergestellt werden; oder

L.

(i) Umsetzen einer Verbindung der Formel (E) oder (G) mit einem $C_{1-5}$-Alkyllithium-Reagenz, wodurch durch Metallierung bzw. Lithium/Halogen-Austausch das entsprechende Lithium-Reagenz erhalten wird;

(ii) Zugeben eines Überschusses von Magnesiumhalogenidetherat zum Lithium-Reagenz, wodurch durch Transmetallierung das entsprechende Grignard-Reagenz erhalten wird;

(iii) Zugeben des Grignard-Reagenz zu einem N-Acylpyridiumsalz, wodurch die entsprechende Verbindung der Formel (F), wie vorstehend definiert, erhalten wird;

(iv) Deacylieren/Oxidieren der Verbindung der Formel (F), wodurch die entsprechende Verbindung der Formel erhalten wird; oder

M. Behandeln des 3-Lithio-Derivats der Verbindung der Formel (E) oder (G), jeweils wie bereits definiert, mit Trialkylzinnchlorid, wodurch eine Verbindung der Formel (J) mit der nachstehenden Formel erhalten wird:

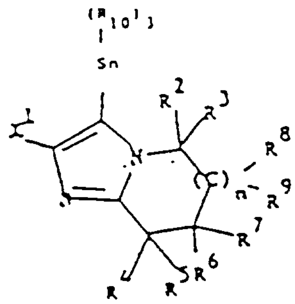

FORMEL J

in der

n 0 oder 1 ist;

$R^2$, $R^3$, $R^4$, $R^5$, $R^6$, $R^7$, $R^8$ und $R^9$ ein H-Atom sind oder einer oder zwei der Reste $R^2$, $R^3$, $R^4$, $R^5$, $R^6$, $R^7$, $R^8$ und $R^9$ unabhängig aus einem H-Atom oder einem $C_{1-2}$-Alkylrest gewählt sind;

$R_{10}$ ein $C_{1-4}$-Alkylrest ist;

und $X^1$ gewählt ist aus:

(a) einem monosubstituierten Phenylrest, wobei der Substituent gewählt ist aus einem H-, Fluor-, Chloratom, einem $C_{1-3}$-Alkoxy-, $C_{1-4}$-Alkyl-, $C_{1-3}$-Dialkylamino-Rest, einer $CF_3$-Gruppe, einem $C_{1-3}$-Alkylamino-Rest, einer N-Pyrrolidino-, N-Piperidino-, Prop-2-en-1-oxy- oder 2,2,2-Trihalogenethoxygruppe; oder

(b) einer Pyridylgruppe oder einem mit einem Alkylrest substituierten Pyridylrest;

und Umsetzen der Verbindung der Formel (J) mit einem Gemisch aus einem Aryl- oder Heteroarylhalogenid oder -triflat und Tetrakis(triphosphin)palladium in einem Gemisch aus Tetrahydrofuran und Hexamethylphosphoramid, wodurch eine Verbindung der Formel (I) erhalten wird; oder

N. wenn eine Verbindung der Formel (I) hergestellt werden soll, in der einer der Reste R oder $R^1$ eine substituierter Phenylrest ist, der eine oder mehrere Alkylthiogruppen enthält: Behandeln der entsprechenden fluor-substituierten Phenylverbindung der Formel (I) mit einem Metallsalz des Alkylmercaptans in einem aprotischen polaren Lösungsmittel, wodurch die gewünschte Verbindung der Formel (I) erhalten wird; oder

O. wenn eine Verbindung der Formel (I) hergestellt werden soll, in der einer der Reste R oder $R^1$ ein mit einem Acyloxyalkylthiorest substituierter Phenylrest ist, wobei der Alkylrest gegebenenfalls mit einem $C_{1-4}$-Alkylrest substituiert ist: Behandeln einer Verbindung der Formel (I), wobei $R^1$ ein mit mindestens einem Alkylsulfinylrest substituierter Phenylrest ist, mit einem Alkansäureanhydrid, wodurch die gewünschte Verbindung der Formel (I) erhalten wird; oder

P. wenn eine Verbindung der Formel (I) hergestellt werden soll, wobei einer der Reste $R^1$ oder R eine mit mindestens einer Sulfhydrylgruppe substituierte Phenylgruppe ist: Hydrolyse des Produktes des vorstehend beschriebenen Verfahrens O, wodurch die gewünschten Verbindungen der Formel (I) erhalten werden; oder

Q. wenn eine Verbindung der Formel (I) hergestellt werden soll, wobei einer der Reste $R^1$ oder R eine Phenylgruppe ist, die mit mindestens einer Acylthio-, Dithioacyl-, Thiocarbamyl- oder Dithiocarbamylgruppe substituiert ist: Behandeln des Produktes des vorstehenden Verfahrens P mit einem Acylhalogenid, einem Alkansäureanhydrid, einem Thioacylhalogenid, einem Dithioalkansäureanhydrid, einem Dialkylcarbamylhalogenid oder einem Dialkylthiocarbamylhalogenid in Gegenwart einer Base, wie Pyridin, wodurch die gewünschten Verbindungen der Formel (I) erhalten werden; oder

R. wenn eine Verbindung der Formel (I) hergestellt werden soll, in der $R^1$ oder R ein mit einer Alkenylthiogruppe substituierter Phenylrest ist: Alkylieren einer Verbindung der Formel (I), worin einer der Reste $R^1$ oder R eine mit mindestens einer Sulfhydrylgruppe substituierte Phenylgruppe ist, mit einem geeignet substituierten Alkenylhalogenid, wie Allylbromid, wodurch Verbindungen der Formel (I) erhalten werden, in denen $R^1$ oder R ein mit mindestens einem Alkenylthiorest substituierter Phenylrest ist; oder

S. wenn eine Verbindung der Formel (I) hergestellt werden soll, wobei $R^1$ oder R ein mit einem Alkenylthiorest substituierter Phenylrest ist, wobei der Schwefel an das die Doppelbindung tragende Kohlenstoffatom gebunden ist:

i) Behandeln des Mercaptoprodukts des vorstehenden Verfahrens P mit einer starken Base, wodurch die entsprechende Metallmercaptidsalzverbindung erhalten wird,

ii) Behandeln der Metallmercaptidsalzverbindung mit Trialkylsilylmethylchlorid, wodurch eine Verbindung der Formel (I) erhalten wird, in der der Phenylrest mindestens einen Trialkylsilylmethylsulfid-Substituenten aufweist,

iii) Behandeln der trialkylsilylmethylsulfid-substituierten Verbindung in einem aprotischen Lösungsmittel mit einem Reagenz zur Übertragung von Lithium gefolgt vom geeigneten aliphatischen Aldehyd oder Keton, wodurch eine Verbindung der Formel (I) erhalten wird, in der R oder $R^1$ ein mit mindestens einem Alkenylthiorest substituierter Phenylrest ist; oder

T. wenn eine Verbindung der Formel (I) hergestellt werden soll, wobei R oder $R^1$ ein alkylsubstituierter Pyridylrest ist: Deacylieren und Aromatisieren einer Verbindung der Formel (L)

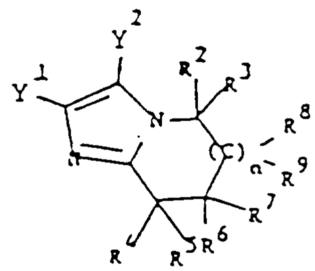

FORMEL (L)

in der

n 0 oder 1 ist;

$R^2$, $R^3$, $R^4$, $R^5$, $R^6$, $R^7$, $R^8$ und $R^9$ jeweils ein H-Atom sind oder einer oder zwei der Reste $R^2$, $R^3$, $R^4$, $R^5$, $R^6$, $R^7$, $R^8$ und $R^9$ unabhängig aus einem H-Atom oder einem $C_{1-2}$-Alkylrest gewählt sind;

einer der Reste $Y^1$ oder $Y^2$ unabhängig gewählt ist aus einem 4-[1,2-Dihydro-2-($C_{1-4}$-alkyl)]pyridyl-Rest, der substituiert ist mit einem N-($C_{1-8}$-Alkanoyl)-, N-($C_{1-8}$-Alkoxycarbonyl)-Rest, einer N-(Benzoyl)-, N-(Phenoxycarbonyl)-, N-(Phenylacetyl)- oder N-(Benzyloxycarbonyl)-Gruppe;

und der andere Rest gewählt ist aus:

(a) einem monosubstituierten Phenylrest, wobei der Substituent gewählt ist aus einem H-, Halogenatom, $C_{1-3}$-Alkoxy-, $C_{1-3}$-Alkylthio-, $C_{1-4}$-Alkyl-, N-($C_{1-3}$-Alkyl)-N-($C_{1-3}$-alkanamido)-, $C_{1-3}$-Dialkylamino-Rest, einer $CF_3$-, N-Pyrrolidino-, N-Piperidino-, Prop-2-en-1-oxy- oder 2,2,2-Trihalogenethoxy-Gruppe; oder

ein pharmazeutisch verträgliches Salz davon mit Schwefel 15 Minuten unter Rückfluß von Decalin, Tetralin, p-Cymol oder Xylol mit Sauerstoffgas, wodurch die entsprechende Verbindung der Formel (I) erhalten wird.

**2.** Verfahren nach Anspruch 1, wobei die Verbindung der Formel (I) 2-(4-Methylthiophenyl)-3-(4-pyridyl)-6,7-dihydro-[5H]-pyrrolo[1,2-a]imidazol oder ein pharmazeutisch verträgliches Salz davon ist.

**3.** Verfahren nach Anspruch 1, wobei die Verbindung der Formel (I) 2-(4-Methylsulfinylphenyl)-3-(4-pyridyl)-6,7-dihydro-[5H]-pyrrolo[1,2-a]imidazol oder ein pharmazeutisch verträgliches Salz davon ist.

**4.** Verfahren nach Anspruch 1, das die Herstellung einer Verbindung der Formel (I) umfaßt, nämlich:
2-(4-Methylsulfonylphenyl)-3-(4-pyridyl)-6,7-dihydro-[5H]-pyrrolo[1,2-a]imidazol,
2-(4-Methoxyphenyl)-3-(2,6-dimethyl-4-pyridyl)-6,7-dihydro-[5H]-pyrrolo[1,2-a]imidazol,
2-(4-Ethylthiophenyl)-3-(4-pyridyl)-6,7-dihydro-[5H]-pyrrolo[1,2-a]imidazol,
2-(4-Ethylsulfinylphenyl)-3-(4-pyridyl)-6,7-dihydro-[5H]-pyrrolo[1,2-a]imidazol,
2-(4-Mercaptophenyl)-3-(4-pyridyl)-6,7-dihydro-[5H]-pyrrolo[1,2-a]imidazol,
2-(4-Trimethylacetylthiophenyl)-3-(4-pyridyl)-6,7-dihydro-[5H]-pyrrolo[1,2-a]imidazol,

2-(4-Acetylthiophenyl)-3-(4-pyridyl)-6,7-dihydro-[5H]-pyrrolo[1,2-a]imidazol,
2-(4-Ethylsulfonylphenyl)-3-(4-pyridyl)-6,7-dihydro-[5H]-pyrrolo[1,2-a]imidazol,
2-(4-Fluorphenyl)-3-[4-(2-methyl)pyridyl]-6,7-dihydro-[5H]-pyrrolo[1,2-a]imidazol,
2-(4-Methylthiophenyl)-3-[4-(2-methyl)pyridyl]-6,7-dihydro-[5H]-pyrrolo[1,2-a]imidazol,
2-(4-Methylsulfinylphenyl)-3-[4-(2-methyl)pyridyl]-6,7-dihydro-[5H]-pyrrolo[1,2-a]imidazol,
2-(4-Carbethoxymethylthiophenyl)-3-(4-pyridyl)-6,7-dihydro-[5H]-pyrrolo[1,2-a]imidazol, oder
2-(4-Acetoxymethylthiophenyl)-3-(4-pyridyl)-6,7-dihydro-[5H]-pyrrolo[1,2-a]imidazol.

5. Verfahren zur Herstellung einer Verbindung der Formel (I), die 2-(4-Methylthiophenyl)-3-(4-pyridyl)-6,7-dihydro-[5H]-pyrrolo[1,2-a]imidazol ist, umfassend die Behandlung der entsprechenden fluor-substituierten Phenylverbindung der Formel (I) nach Anspruch 1 mit 1,2 Äquivalenten eines Metallsalzes von Alkylmercaptan in einem aprotischen polaren Lösungsmittel.

6. Verfahren zur Herstellung einer Verbindung der Formel (I), die 2-(4-Methylsulfinylphenyl)-3-(4-pyridyl)-6,7-dihydro-[5H]-pyrrolo[1,2-a]imidazol ist, umfassend die Oxidation der entsprechenden 4-Methylthiophenylverbindung mit einem geeigneten Oxidationsmittel.

7. Verfahren zur Herstellung eines Arzneimittels, das eine Verbindung der Formel (I) nach einem der Ansprüche 1 bis 4 enthält, wobei das Verfahren dA Verbinden der Verbindung mit einem pharmazeutisch verträglichen Träger umfaßt.

8. Verbindung der Formel

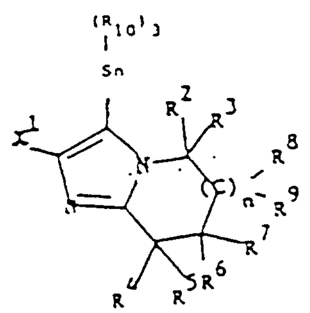

FORMEL (J)

in der
n 0 oder 1 ist;
$R^2$, $R^3$, $R^4$, $R^5$, $R^6$, $R^7$, $R^8$ und $R^9$ jeweils ein H-Atom sind oder einer oder zwei der Reste $R^2$, $R^3$, $R^4$, $R^5$, $R^6$, $R^7$, $R^8$ und $R^9$ unabhängig aus einem H-Atom oder einer $C_{1-2}$-Alkylgruppe gewählt ist;
$R_{10}$ ein $C_{1-4}$-Alkylrest ist;
und $X^1$ gewählt ist aus:
(a) einem monosubstituierten Phenylrest, wobei der Substituent aus einem H-, Fluor-, Chloratom, einem $C_{1-3}$-Alkoxy-, $C_{1-4}$-Alkyl-, $C_{1-3}$-Dialkylaminorest, einer $CF_3$-Gruppe, einem $C_{1-3}$-Alkylaminorest, einer N-Pyrrolidino-, N-Piperidino-, Prop-2-en-1-oxy- oder 2,2,2-Trihalogenethoxygruppe gewählt ist;
(b) einer Pyridylgruppe oder einem alkyl-substituierten Pyridylrest;
oder ein pharmazeutisch verträgliches Salz davon.

9. Verbindung nach Anspruch 8, die 2-(4-Methoxyphenyl)-6,7-dihydro-[5H]-pyrrolo[1,2-a]imidazol-3-yl-tri-n-butylzinn ist.

**10.** Verbindung der Formel

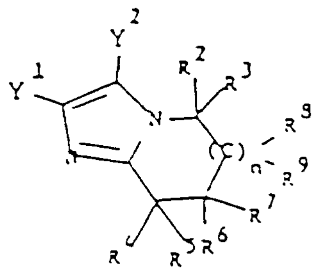

FORMEL (L)

in der
n 0 oder 1 ist,
$R^2$, $R^3$, $R^4$, $R^5$, $R^6$, $R^7$, $R^8$, $R^9$ alle ein H-Atom sind, oder einer oder zwei der Reste $R^2$, $R^3$, $R^4$, $R^5$, $R^6$, $R^7$, $R^8$ und $R^9$ unabhängig aus einem H-Atom oder einem $C_{1-2}$-Alkylrest gewählt sind;
einer der Reste $Y^1$ oder $Y^2$ unabhängig aus einem 4-[1,2-Dihydro-2-($C_{1-4}$-alkyl]pyridylrest gewählt ist, der mit einem N-($C_{1-8}$-Alkanoyl)-, N-($C_{1-8}$-Alkoxycarbonyl)-Rest, einer N-(Benzoyl)-, N-(Phenoxycarbonyl)-, N-(Phenylacetyl)- oder N-(Benzyloxycarbonyl)-Gruppe substituiert ist;
und der andere gewählt ist aus:
(a) einem monosubstituierten Phenylrest, wobei der Substituent aus einem H-, einem Halogenatom, einem $C_{1-3}$-Alkoxy-, $C_{1-3}$-Alkylthio-, $C_{1-4}$-Alkyl-, N-($C_{1-3}$-Alkyl)-N-($C_{1-3}$-alkanamido)-, $C_{1-3}$-Dialkylamino-Rest, einer $CF_3$-, N-Pyrrolidino-, N-Piperidino-, Prop-2-en-1-oxy- oder 2,2,2-Trihalogenethoxy-Gruppe gewählt ist; oder ein pharmazeutisch verträgliches Salz davon.

**Revendications**
**Revendications pour les Etats contractants suivants : AT, BE, CH, DE, FR, GB, IT, LI, LU, NL, SE**

**1.** Composé de formule :

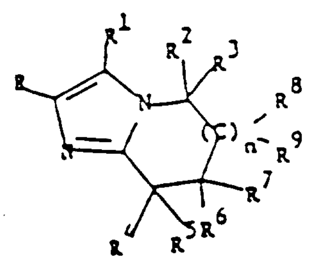

FORMULE (I)

dans lequel :
1) un des groupes R ou $R^1$ doit être un groupe pyridyle alkyle-substitué, et l'autre est choisi parmi les groupes phényle monosubstitués dans lesquels ledit substituant est choisi parmi H, les halogènes, les groupes hydroxy, alcoxy $C_{1-3}$, alkyl $C_{1-3}$ thio, alkyle $C_{1-4}$, alkyl $C_{1-3}$ sulfinyle, alkyl $C_{1-3}$ sulfonyle, alkyl $C_{1-3}$ amino, dialkyl $C_{1-3}$ amino, $CF_3$, N-(alkyl $C_{1-3}$)-N-(alcan $C_{1-3}$ amido), N-pyrrolidino, N-pipéridino, prop-2-èn-1-oxy, ou 2,2,2-trihaloéthoxy; ou
2) $R^1$ est un groupe 4-pyridyle et R est choisi parmi les groupes phényle monosubstitués dans lesquels ledit substituant est choisi parmi les groupes alkyl $C_{1-3}$ thio, alkyl $C_{1-3}$ sulfinyle, alkyl $C_{1-3}$ sulfonyle, ou un groupe alkényl $C_{2-5}$ thio ou alkényl $C_{2-5}$ sulfinyle ramifié ou non ramifié; ou

3) un des groupes $R^1$ ou R est un groupe pyridyle ou pyridyle alkyle-substitué, et l'autre est choisi parmi les groupes phényle monosubstitués ou disubstitués dans lesquels ledit substituant est choisi parmi les groupes thiol [HS-], acylthio [AC(O)S-], dithioacyle [AC(S)S-], dialkylthiocarbamyle [$A_2$NC-(O)S-], dialkyldithiocarbamyle [$A_2$NC(S)S-], alkylcarbonylalkylthio [AC(O)$CH_2$S-], carbalcoxyalkylthio [AOC(O)$CH_2$S-], ou acyloxyalkylthio [AC(O)O$CH_2$S-], dans lesquels le groupe $CH_2$ est éventuellement substitue par un groupe alkyle $C_{1-4}$, et A est un groupe alkyle $C_{1-9}$;
et $R^2$, $R^3$, $R^4$, $R^5$, $R^6$, $R^7$, $R^8$, et $R^9$ sont un atome d'hydrogène, ou un ou deux parmi $R^2$, $R^3$, $R^4$, $R^5$, $R^6$, $R^7$, $R^8$, et $R^9$ sont choisis indépendamment entre un atome d'hydrogène et un groupe alkyle $C_{1-2}$; n est égal à 0 ou à 1;
ou un sel de celui-ci, pharmaceutiquement acceptable.

**2.** Composé selon la revendication 1, qui est le 2-(4-méthylthiophényl)-3-(4-pyridyl)-6,7-dihydro-[5H]-pyrrolo[1,2-a]imidazole.

**3.** Composé selon la revendication 1, qui est le 2-(4-méthylsulfinylphényl)-3-(4-pyridyl)-6,7-dihydro-[5H]-pyrrolo[1,2-a]imidazole.

**4.** Composé selon la revendication 1, qui est :
- le 2-(4-méthylsulfonylphényl)-3-(4-pyridyl)-6,7-dihydro-[5H]-pyrrolo-[1,2-a]imidazole,
- le 2-(4-méthoxyphényl)-3-(2,6-diméthyl-4-pyridyl)-6,7-dihydro-[5H]-pyrrolo[1,2-a]imidazole,
- le 2-(4-éthylsulfinylphényl)-3-(4-pyridyl)-6,7-dihydro-[5H]-pyrrolo-[1,2-a]imidazole,
- le 2-(4-éthylthiophényl)-3-(4-pyridyl)-6,7-dihydro-[5H]-pyrrolo[1,2-a]imidazole,
- le 2-(4-mercaptophényl)-3-(4-pyridyl)-6,7-dihydro-[5H]-pyrrolo[1,2-a]imidazole,
- le 2-(4-triméthylacétylthiophényl)-3-(4-pyridyl)-6,7-dihydro-[5H]-pyrrolo[1,2-a]imidazole,
- le 2-(4-acétylthiophényl)-3-(4-pyridyl)-6,7-dihydro-[5H]-pyrrolo-[1,2-a]imidazole,
- le 2-(4-éthylsulfonylphényl)-3-(4-pyridyl)-6,7-dihydro-[5H]-pyrrolo-[1,2-a]imidazole,
- le 2-(4-fluorophényl)-3-[4-(2-méthyl)pyridyl]-6,7-dihydro-[5H]-pyrrolo-[1,2-a]imidazole,
- le 2-(4-méthylthiophényl)-3-[4-(2-méthyl)pyridyl]-6,7-dihydro-[5H]-pyrrolo-[1,2-a]imidazole,
- le 2-(4-méthylsulfinylphényl)-3-[4-(2-méthyl)pyridyl]-6,7-dihydro-[5H]-pyrrolo-[1,2-a]imidazole,
- le 2-(4-carbéthoxyméthylthiophényl)-3-(4-pyridyl)-6,7-dihydro-[5H]-pyrrolo[1,2-a]imidazole, ou
- le 2-(4-acétoxyméthylthiophényl)-3-(4-pyridyl)-6,7-dihydro-[5H]-pyrrolo[1,2-a]imidazole.

**5.** Composition pharmaceutique comprenant un véhicule ou diluant pharmaceutiquement acceptable, et un composé selon l'une quelconque des revendications 1 à 4.

**6.** Composition selon la revendication 5, dans laquelle la composition est sous forme de dose unitaire adaptée à l'administration parentérale, et qui contient 50 mg à 500 mg du composé actif.

**7.** Composition selon la revendication 5, dans laquelle la composition est sous forme de dose unitaire adaptée à l'administration orale, et qui contient 100 mg à 1.000 mg du composé actif.

**8.** Composition selon la revendication 5, dans laquelle la composition est sous forme de dose unitaire adaptée à l'administration par inhalation, ou à l'administration topique.

**9.** Composé de Formule (I) selon l'une quelconque des revendications 1 à 4, pour l'utilisation dans le traitement d'une maladie impliquant la voie de la 5-lipoxygénase chez un animal.

**10.** Composé de Formule (I) selon l'une quelconque des revendications 1 à 4, pour l'utilisation dans le traitement ou la prophylaxie d'un état inflammatoire chez un animal, ou de la pyrèse, de la douleur, et d'autres états associés à l'inflammation.

**11.** Composé de Formule (I) en vue de l'utilisation selon la revendication 10, pour laquelle l'état inflammatoire est choisi parmi l'arthrite rhumatoïde, la spondylite rhumatoïde, l'ostéoarthrite, l'arthrite goutteuse, et parmi d'autres états arthritiques, inflammation des articulations, eczéma, psoriasis ou autres états inflammatoires de la peau, tels que le coup de soleil; et parmi les états inflammatoires de l'oeil, tels que la conjonctivite.

**12.** Composé de formule :

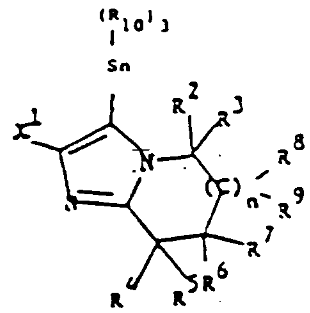

FORMULE (J)

dans lequel

- n est égal à 0 ou à 1;
- $R^2$, $R^3$, $R^4$, $R^5$, $R^6$, $R^7$, $R^8$, et $R^9$ sont des atomes d'hydrogène ou un ou deux de $R^2$, $R^3$, $R^4$, $R^5$, $R^6$, $R^7$, $R^8$, et $R^9$ sont choisis indépendamment parmi l'hydrogène ou les groupes alkyle $C_{1-2}$;
- $R_{10}$ est un groupe alkyle $C_{1-4}$; et
- $X^1$ est choisi parmi

(a) les groupes phényle monosubstitués, ledit substituant étant choisi parmi l'hydrogène, les groupes fluoro, chloro, alcoxy $C_{1-3}$, alkyle $C_{1-4}$, dialkyl $C_{1-3}$ amino, $CF_3$, alkyl $C_{1-3}$ amino, N-pyrrolidino, N-pipéridino, prop-2-èn-1-oxy, ou 2,2,2-trihaloéthoxy;

(b) les groupes pyridyle ou pyridyle alkyle-substitués; ou un sel de celui-ci pharmaceutiquement acceptable.

**13.** Composé selon la revendication 12, qui est le 2-(4-méthoxyphényl)-6,7-dihydro-[5H]-pyrrolo[1,2-a]-imidazol-3-yl-tri-n-butyl étain.

**14.** Composé de formule :

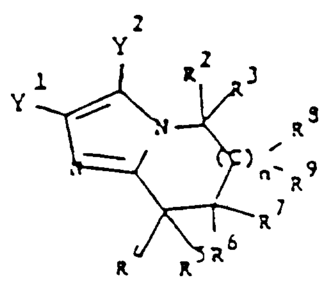

FORMULE (L)

dans lequel

- n est égal à 0 ou à 1;
- $R^2$, $R^3$, $R^4$, $R^5$, $R^6$, $R^7$, $R^8$, et $R^9$ sont tous des atomes d'hydrogène, ou un ou deux de $R^2$, $R^3$, $R^4$, $R^5$, $R^6$, $R^7$, $R^8$, et $R^9$ sont choisis indépendamment parmi l'hydrogène ou les groupes alkyle $C_{1-2}$;
- un des groupes $Y^1$ ou $Y^2$ est choisi indépendamment parmi les groupes 4-[1,2-dihydro-2-(alkyl $C_{1-4}$)]pyridyle substitués par des groupes N-(alcanoyle $C_{1-8}$), N-(alcoxy $C_{1-8}$ carbonyle), N-(benzoyle), N-(phénoxycarbonyle), N-(phénylacétyle), ou N-(benzyloxycarbonyle);
- et l'autre est choisi parmi les groupes phényle monosubstitués, ledit substituant étant choisi parmi l'hydrogène, les groupes halo, alcoxy $C_{1-3}$, alkyl $C_{1-3}$ thio, alkyle $C_{1-4}$, N-(alkyl $C_{1-3}$)-N-

(alcan $C_{1-3}$ amido), dialkyl $C_{1-3}$ amino, $CF_3$, N-pyrrolidino, N-pipéridino, prop-2-èn-1-oxy, ou 2,2,2-trihaloéthoxy;

ou un sel de celui-ci, pharmaceutiquement acceptable.

**15.** Procédé de préparation d'un composé de Formule (I) selon la revendication 1, ou d'un sel de celui-ci, pharmaceutiquement acceptable, qui comprend :

A. lorsque le produit à préparer est un composé de Formule (I) dans lequel $R^1$ est un groupe 4-pyridyle, et R est un groupe autre que pyridyle, la réaction d'un composé de Formule (E) représenté par la structure :

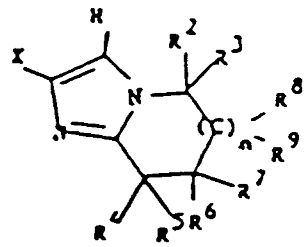

FORMULE (E)

dans lequel

- n est égal à 0 ou à 1;
- $R^2$, $R^3$, $R^4$, $R^5$, $R^6$, $R^7$, $R^8$, et $R^9$ sont des atomes d'hydrogène, ou un ou deux de $R^2$, $R^3$, $R^4$, $R^5$, $R^6$, $R^7$, $R^8$, et $R^9$ sont choisis indépendamment parmi l'hydrogène ou les groupes alkyle $C_{1-2}$;
- X est un groupe alkyl $C_{1-3}$ thiophényle;

dans la pyridine, avec un halogénure d'aroyle, un ester arylalkyle haloformiate, ou un ester alkyle haloformiate, ou avec le sel acyl pyridinium préformé, pour donner un composé de Formule (F) représenté par la structure :

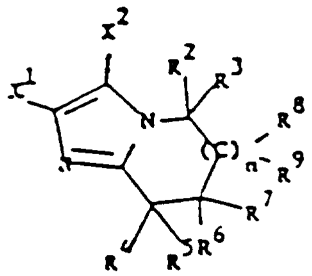

FORMULE (F)

dans lequel

- n est égal à 0 ou à 1;
- $R^2$, $R^3$, $R^4$, $R^5$, $R^6$, $R^7$, $R^8$, et $R^9$ sont des atomes d'hydrogène, ou un ou deux de $R^2$, $R^3$, $R^4$, $R^5$, $R^6$, $R^7$, $R^8$, et $R^9$ sont choisis indépendamment parmi l'hydrogène ou les groupes alkyle $C_{1-2}$;
- $X^1$ est un groupe phényle monosubstitué par un groupe alkyl $C_{1-3}$ thio; et
- $X^2$ est un groupe 4-(1,4-dihydro)pyridyle substitué par un groupe N-(alcanoyle $C_{1-8}$), N-(alcoxy $C_{1-8}$ carbonyle), N-(benzoyle), N-(phénoxycarbonyle), N-(phénylacétyle), ou N-(benzyloxycarbonyle); suivie d'une désacylation/oxydation d'un composé de Formule (F) pour donner un composé de Formule (I); ou

B. lorsque le produit à préparer est un composé hydroxy de Formules (I) ou (E), la déméthylation d'un composé méthoxyphényle de Formule (E) ou (I), dans lequel la Formule (E) est telle que

définie ci-dessus, excepté que X est un groupe méthoxyphényle, et la Formule (I) est représentée par la structure :

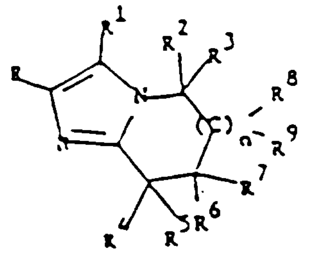

FORMULE (I )

dans lequel

- n est égal à 0 ou à 1;
- $R^2$, $R^3$, $R^4$, $R^5$, $R^6$, $R^7$, $R^8$, et $R^9$ sont des atomes d'hydrogène, ou un ou deux de $R^2$, $R^3$, $R^4$, $R^5$, $R^6$, $R^7$, $R^8$, et $R^9$ sont choisis indépendamment parmi l'hydrogène ou les groupes alkyle $C_{1-2}$;
- un des groupes $R^1$ et R est un groupe pyridyle alkyle-substitué, et l'autre est un groupe méthoxyphényle;

ou un sel de celui-ci, pharmaceutiquement acceptable;

avec HBr dans l'acide acétique, ou avec $BBr_3$ dans le dichlorométhane, pour donner le composé hydroxy correspondant de Formules (I), ou (E); ou

C. l'O-alkylation du composé hydroxy de Formule (I), ou (E), tel que défini plus haut, lorsque l'un des groupes $R^1$ ou R de (I), et le groupe X de (E) est un groupe phényle substitué par au moins un groupe hydroxy, pour donner le composé correspondant, de Formules (I), ou (E) substitué par les groupes alcoxy $C_{1-3}$ phényle, 2,2,2-trihaloéthoxyphényle, ou prop-2-èn-1-oxyphényle; ou

D. l'acylation d'un composé aminophényle ou (alkyl $C_{1-3}$ amino)phényle de Formule (I) avec l'halogénure ou l'anhydride d'acyle substitué de façon correspondante, dans la pyridine, pour donner le composé N-(alkyl $C_{1-3}$ )-(alcan $C_{1-3}$ amido)phényle de Formule (I); ou

E. l'alkylation d'un composé (alcan $C_{1-3}$ amido)phényle de Formules (I), ou (E), tel que défini précédemment, avec un agent alkylant en présence d'un base, pour donner le composé N-(alcan $C_{1-3}$ amido)phényle ou N-(alkyl $C_{1-3}$)-(alcan $C_{1-3}$ amido)phényle correspondant de Formules (I) ou (E); ou

F. l'hydrolyse d'un composé (alcan $C_{1-3}$ amido)phényle ou N-(alkyl $C_{1-3}$)-(alcan $C_{1-3}$ amido)-phényle de Formules (I), ou (E), tel que défini précédemment, ou d'un composé de Formule (G) représenté par la structure :

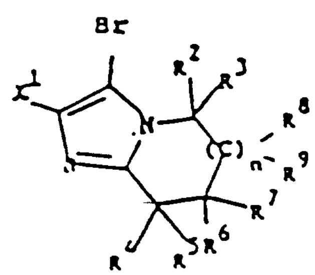

FORMULE (G)

dans lequel

- n est égal à 0 ou à 1;
- $R^2$, $R^3$, $R^4$, $R^5$, $R^6$, $R^7$, $R^8$, et $R^9$ sont des atomes d'hydrogène; ou un ou deux de $R^2$, $R^3$, $R^4$, $R^5$, $R^6$, $R^7$, $R^8$, et $R^9$ sont choisis indépendamment parmi l'hydrogène ou les groupes alkyle $C_{1-2}$;

- $X^1$ est un groupe (alcan $C_{1-3}$ amido)phényle, ou N-(alkyl $C_{1-3}$)-(alcan $C_{1-3}$ amido)phényle;

pour donner le composé aminophényle ou (alkyl $C_{1-3}$ amino)phényle correspondant, de Formules (I), (E), ou (G); ou

G. l'oxydation d'un composé de Formule (I), lorsque l'un des groupes $R^1$ et R est un groupe phényle substitué par un ou plusieurs groupes alkyl $C_{1-3}$ thio, alkyl $C_{1-3}$ sulfinyle, alkényl $C_{1-3}$ thio, ou acyloxyalkylthio, avec un agent oxydant pour donner le composé alkyl $C_{1-3}$ sulfinyle, alkyl $C_{1-3}$ sulfonyle, acyloxyalkylsulfinyle, ou alkényl $C_{1-3}$ sulfinyle correspondant, de Formule (I); ou

H. la réduction d'un composé de Formules (E), ou (I), tel que défini précédemment, dans lequel le groupe X de la Formule (E), ou un des groupes R et $R^1$ de la Formule (I), est un groupe phényle mono- ou di-substitué contenant un groupe nitro, avec l'hydrogène gazeux en présence d'un catalyseur, pour donner le composé aminophényle correspondant, de Formules (I), ou (E); ou

I. la cycloalkylation d'un composé de Formules (I), ou (E), tel que défini précédemment, dans lequel un des groupes $R^1$ et R de la Formule (I), ou le groupe X de la Formule (E), est un groupe aminophényle, avec selon les cas, le dihalobutane ou le dihalopentane, en présence d'une base, pour donner le composé N-pipéridino, ou N-pyrrolidino correspondant, de Formules (I), ou (E); ou

J. lorsque les produits à préparer sont des composés de Formules (I) ou (E), tels que définis précédemment, dans lesquels les groupes $R^1$ et R de la Formule (I), ou le groupe X de la Formule (E), est un groupe (alkyl $C_{1-3}$ amino)phényle, la réduction des composés alcan $C_{1-3}$ amido correspondants par le borane ou le complexe borane diméthylsulfure dans le tétrahydrofurane, pour donner les composés désirés de Formules (I), ou (E); ou

K. lorsque les produits à préparer sont des composés de Formules (I) ou (E), tels que définis précédemment, dans lesquels l'un des groupes $R^1$ et R de la Formule (I), ou le groupe X de la Formule (E), est un groupe (dialkyl $C_{1-3}$ amino)phényle, la réduction des composés N-(alkyl $C_{1-3}$)-(alcan $C_{1-3}$ amido) correspondants par le borane ou le complexe borane diméthylsulfure dans le tétrahydrofurane, pour donner les composés désirés de Formules (I), ou (E); ou

L.

(i) la réaction d'un composé de Formule (E), ou (G) avec un réactif alkyl $C_{1-5}$ lithium pour donner le réactif lithium correspondant, par métallation ou inter-échange lithium-halogène, respectivement;

(ii) l'addition d'étherate d'halogénure de magnésium en excès, au réactif lithium pour donner par transmétallation, le réactif de Grignard correspondant;

(iii) l'addition du réactif de Grignard à un sel de N-acylpyridinium, pour donner le composé correspondant de Formule (F) tel que défini plus haut; ou

(iv) la désacylation/oxydation du composé de Formule (F) pour donner le composé correspondant de Formule (I); ou

M. le traitement du dérivé 3-lithio d'un composé de Formules (E), ou (G), chacun tel que défini précédemment, avec un chlorure de trialkyl étain, pour donner un composé de Formule (J) ayant la formule suivante :

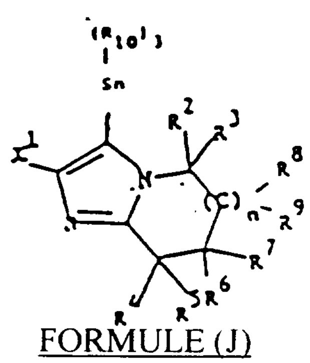

FORMULE (J)

dans lequel :

- n est égal à 0 ou à 1;
- $R^2$, $R^3$, $R^4$, $R^5$, $R^6$, $R^7$, $R^8$, et $R^9$ sont des atomes d'hydrogène, ou un ou deux de $R^2$, $R^3$, $R^4$, $R^5$, $R^6$, $R^7$, $R^8$, et $R^9$ sont choisis indépendamment parmi l'hydrogène ou les groupes alkyle $C_{1-2}$;
- $R_{10}$ est un groupe alkyle $C_{1-4}$; et
- $X^1$ est choisi parmi

(a) les groupes phényle monosubstitués, ledit substituant étant choisi parmi les groupes H, fluoro, chloro, alcoxy $C_{1-3}$, alkyle $C_{1-4}$, dialkyl $C_{1-3}$ amino, $CF_3$, alkyl $C_{1-3}$ amino, N-pyrrolidino, N-pipéridino, prop-2-èn-1-oxy, ou 2,2,2-trihaloéthoxy; ou

(b) les groupes pyridyle ou pyridyle alkyle-substitués; et la réaction du composé de Formule (J) avec un mélange d'halogénure ou triflate d'aryle ou d'hétéroaryle, et de tétrakis(triphosphine)-palladium dans un mélange de tétrahydrofurane et d'hexaméthylphosphoramide, pour donner un composé de Formule (I); ou

N. lorsque le produit à préparer est un composé de Formule (I) dans lequel un des groupes R ou $R^1$ est un groupe phényle substitué contenant un ou plusieurs groupes alkylthio, le traitement du composé phényle fluoro-substitué correspondant, de Formule (I), avec un sel métallique de l'alkylmercaptan dans un solvant polaire aprotique, pour donner le composé désiré de Formule (I); ou

O. lorsque le produit à préparer est un composé de Formule (I) dans lequel un des groupes R ou $R^1$ est un groupe phényle substitué par un groupe acyloxyalkylthio dans lequel le groupe alkyle est éventuellement substitué par un groupe alkyle $C_{1-4}$, le traitement d'un composé de Formule (I) dans lequel $R^1$ est un groupe phényle substitué par au moins un groupe alkylsulfinyle, avec un anhydride d'acide alcanoïque, pour donner le composé désiré de Formule (I); ou

P. lorsque le produit à préparer est un composé de Formule (I) dans lequel un des groupes $R^1$ ou R est un groupe phényle substitué par au moins un groupe sulfhydryle, l'hydrolyse du produit issu du procédé O décrit ci-dessus, pour donner les composés désirés de Formule (I); ou

Q. lorsque le produit à préparer est un composé de Formule (I) dans lequel un des groupes $R^1$ ou R est un groupe phényle substitué par au moins un groupe acylthio, dithioacyle, thiocarbamyle, ou dithiocarbamyle, le traitement du produit issu du procédé O décrit ci-dessus, avec un halogénure d'acyle, un anhydride d'acide alcanoïque, un halogénure de thioacyle, un anhydride d'acide dithioalcanoïque, un halogénure de dialkylcarbamyle, ou un halogénure de dialkylthiocarbamyle, en présence d'une base telle que la pyridine, pour donner les composés désirés de Formule (I); ou

R. lorsque le produit à préparer est un composé de Formule (I) dans lequel le groupe $R^1$ ou le groupe R est un groupe phényle substitué par un groupe alkénylthio, l'alkylation d'un composé de Formule (I) dans lequel un des groupes $R^1$ ou R est un groupe phényle substitué par au moins un groupe sulfhydryle, avec un halogénure d'alkényle substitué de façon appropriée, tel que le bromure d'allyle, pour donner des composés de Formule (I) dans lesquels $R^1$ ou R est un groupe phényle substitué par au moins un groupe alkénylthio; ou

S. lorsque le produit à préparer est un composé de Formule (I) dans lequel le groupe $R^1$ ou le groupe R est un groupe phényle substitué par un groupe alkénylthio dans lequel le soufre est attaché à l'atome de carbone portant la double liaison,

i) le traitement du produit mercapto issu du procédé P décrit ci-dessus, avec une base forte, pour donner le composé sel de métal du mercaptide correspondant,

ii) le traitement du composé sel de métal du mercaptide avec un trialkylsilylméthylchlorure, pour donner un composé de Formule (I) dans lequel le groupe phényle a au moins un substituant trialkylsilylméthylsulfure,

iii) le traitement du composé trialkylsilylméthylsulfure-substitué, dans un solvant aprotique, avec un réactif lithiant, suivi de l'aldéhyde ou cétone aliphatique approprié, pour donner un composé de Formule (I) dans lequel R ou $R^1$ est un groupe phényle substitué par au moins un groupe alkénylthio: ou

T. lorsque le produit à préparer est un composé de Formule (I) dans lequel le groupe R ou le groupe $R^1$ est un groupe pyridyle substitué par un groupe alkyle; la désacylation et l'aromatisation d'un composé de Formule (L) :

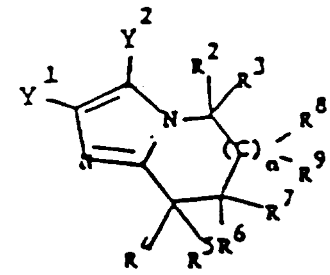

FORMULE (L)

dans lequel

- n est égal à 0 ou à 1;
- $R^2$, $R^3$, $R^4$, $R^5$, $R^6$, $R^7$, $R^8$, et $R^9$ sont tous des atomes d'hydrogène, ou un ou deux de $R^2$, $R^3$, $R^4$, $R^5$, $R^6$, $R^7$, $R^8$, et $R^9$ sont choisis indépendamment parmi l'hydrogène ou les groupes alkyle $C_{1-2}$;
- un des groupes $Y^1$ ou $Y^2$ est choisi indépendamment parmi les groupes 4-[1,2-dihydro-2-(alkyl $C_{1-4}$)]pyridyle substitués par des groupes N-(alcanoyle $C_{1-8}$), N-(alcoxy $C_{1-8}$ carbonyle), N-(benzoyle), N-(phénoxycarbonyle), N-(phénylacétyle), ou N-(benzyloxycarbonyle);
- et l'autre est choisi parmi les groupes phényle monosubstitués, ledit substituant étant choisi parmi l'hydrogène, les groupes halo, alcoxy $C_{1-3}$, alkyl $C_{1-3}$ thio, alkyle $C_{1-4}$, N-(alkyl $C_{1-3}$)-N-(alcan $C_{1-3}$ amido), dialkyl $C_{1-3}$ amino, $CF_3$, N-pyrrolidino, N-pipéridino, prop-2-èn-1-oxy, ou 2,2,2-trihaloéthoxy;

ou d'un sel de celui-ci, pharmaceutiquement acceptable, par le soufre, dans la décaline, la tétraline, le p-cymène, ou le xylène au reflux, avec l'oxygène gazeux pendant 15 minutes, pour donner le composé de Formule (I) correspondant.

**Revendications pour l'Etat contractant suivant : ES**

**1.** Procédé pour la préparation d'un composé de formule :

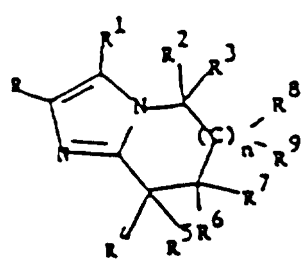

FORMULE (I)

dans lequel :

1) un des groupes R ou $R^1$ doit être un groupe pyridyle alkyle-substitué, et l'autre est choisi parmi les groupes phényle monosubstitués dans lesquels ledit substituant est choisi parmi l'hydrogène, les halogènes. les groupes hydroxy, alcoxy $C_{1-3}$, alkyl $C_{1-3}$ thio, alkyle $C_{1-4}$, alkyl $C_{1-3}$ sulfinyle, alkyl $C_{1-3}$ sulfonyle, alkyl $C_{1-3}$ amino, dialkyl $C_{1-3}$ amino, $CF_3$, N-(alkyl $C_{1-3}$)-N-(alcan $C_{1-3}$ amido), N-pyrrolidino, N-pipéridino, prop-2-èn-1-oxy, ou 2,2,2-trihaloéthoxy; ou

2) $R^1$ est un groupe 4-pyridyle et R est choisi parmi les groupes phényle monosubstitués dans lesquels ledit substituant est choisi parmi les groupes alkyl $C_{1-3}$ thio, alkyl $C_{1-3}$ sulfinyle, alkyl $C_{1-3}$ sulfonyle, ou un groupe alkényl $C_{2-5}$ thio ou alkényl $C_{2-5}$ sulfinyle ramifié ou non ramifié; ou

3) un des groupes $R^1$ ou R est un groupe pyridyle ou pyridyle alkyle-substitué, et l'autre est choisi parmi les groupes phényle monosubstitués ou disubstitués dans lesquels ledit substituant est choisi parmi les groupes thiol [HS-], acylthio [AC(O)S-], dithioacyle [AC(S)S-], dialkylthiocarbamyle [$A_2$NC-

(O)S-], dialkyldithiocarbamyle [$A_2NC(S)S$-], alkylcarbonylalkylthio [$AC(O)CH_2S$-], carbalcoxyalkylthio [$AOC(O)CH_2S$-], ou acyloxyalkylthio [$AC(O)OCH_2S$-], dans lesquels le groupe $CH_2$ est éventuellement substitué par un groupe alkyle $C_{1-4}$, et A est un groupe alkyle $C_{1-9}$;

et $R^2$, $R^3$, $R^4$, $R^5$, $R^6$, $R^7$, $R^8$, et $R^9$ sont un atome d'hydrogène, ou un ou deux parmi $R^2$, $R^3$, $R^4$, $R^5$, $R^6$, $R^7$, $R^8$, et $R^9$ sont choisis indépendamment entre un atome d'hydrogène et un groupe alkyle $C_{1-2}$; et n est égal à 0 ou à 1;

ou d'un sel de celui-ci, pharmaceutiquement acceptable, qui comprend :

A. lorsque le produit à préparer est un composé de Formule (I) dans lequel $R^1$ est un groupe 4-pyridyle, et R est un groupe autre que pyridyle, la réaction d'un composé de Formule (E) représenté par la structure :

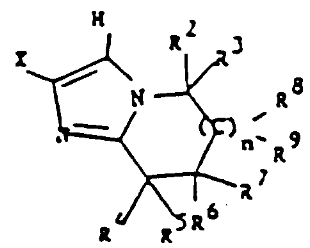

FORMULE (E)

dans lequel

- n est égal à 0 ou à 1;
- $R^2$, $R^3$, $R^4$, $R^5$, $R^6$, $R^7$, $R^8$, et $R^9$ sont des atomes d'hydrogène, ou un ou deux de $R^2$, $R^3$, $R^4$, $R^5$, $R^6$, $R^7$, $R^8$, et $R^9$ sont choisis indépendamment parmi l'hydrogène ou les groupes alkyle $C_{1-2}$;
- X est un groupe alkyl $C_{1-3}$ thiophényle;

dans la pyridine, avec un halogénure d'aroyle, un ester arylalkyle haloformiate, ou un ester alkyle haloformiate, ou avec le sel acyl pyridinium préformé, pour donner un composé de Formule (F) représenté par la structure :

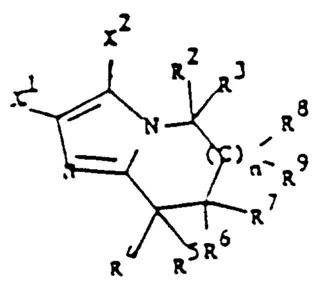

FORMULE (F)

dans lequel

- n est égal à 0 ou à 1;
- $R^2$, $R^3$, $R^4$, $R^5$, $R^6$, $R^7$, $R^8$, et $R^9$ sont des atomes d'hydrogène, ou un ou deux de $R^2$, $R^3$, $R^4$, $R^5$, $R^6$, $R^7$, $R^8$, et $R^9$ sont choisis indépendamment parmi l'hydrogène ou les groupes alkyle $C_{1-2}$;
- $X^1$ est un groupe phényle monosubstitué par un groupe alkyl $C_{1-3}$ thio; et
- $X^2$ est un groupe 4-(1,4-dihydro)pyridyle substitué par un groupe N-(alcanoyle $C_{1-8}$), N-(alcoxy $C_{1-8}$ carbonyle), N-(benzoyle), N-(phénoxycarbonyle), N-(phénylacétyle), ou N-(benzyloxycarbonyle); suivie d'une désacylation/oxydation d'un composé de Formule (F) pour donner un composé de Formule (I); ou

B. lorsque le produit à préparer est un composé hydroxy de Formules (I), ou (E), la déméthylation d'un composé méthoxyphényle de Formule (E) ou (I), dans lequel la Formule (E) est telle que définie ci-dessus, excepté que X est un groupe méthoxyphényle, et la Formule (I) est représentée par la structure :

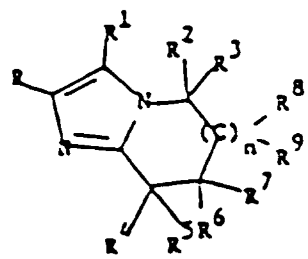

FORMULE (I)

dans lequel

- n est égal à 0 ou à 1;
- $R^2$, $R^3$, $R^4$, $R^5$, $R^6$, $R^7$, $R^8$, et $R^9$ sont des atomes d'hydrogène, ou un ou deux de $R^2$, $R^3$, $R^4$, $R^5$, $R^6$, $R^7$, $R^8$, et $R^9$ sont choisis indépendamment parmi l'hydrogène ou les groupes alkyle $C_{1-2}$;
- un des groupes $R^1$ et R est un groupe pyridyle alkyle-substitué, et l'autre est un groupe méthoxyphényle;

ou d'un sel de celui-ci, pharmaceutiquement acceptable;

avec HBr dans l'acide acétique, ou avec $BBr_3$ dans le dichlorométhane, pour donner le composé hydroxy correspondant, de Formules (I), ou (E); ou

C. l'O-alkylation du composé hydroxy de Formule (I), ou (E), tel que défini plus haut, lorsque l'un des groupes $R^1$ ou R de (I), et le groupe X de (E) est un groupe phényle substitué par au moins un groupe hydroxy, pour donner le composé correspondant, de Formules (I), ou (E) substitué par les groupes alcoxy $C_{1-3}$ phényle, 2,2,2-trihaloéthoxyphényle, ou prop-2-èn-1-oxyphényle; ou

D. l'acylation d'un composé aminophényle ou (alkyl $C_{1-3}$ amino)phényle de Formule (I) avec l'halogénure ou l'anhydride d'acyle substitué de façon correspondante, dans la pyridine, pour donner le composé N-(alkyl $C_{1-3}$ )-(alcan $C_{1-3}$ amido)phényle de Formule (I); ou

E. l'alkylation d'un composé (alcan $C_{1-3}$ amido)phényle de Formules (I), ou (E), tel que défini précédemment, avec un agent alkylant en présence d'un base, pour donner le composé N-(alcan $C_{1-3}$ amido)phényle ou N-(alkyl $C_{1-3}$)-(alcan $C_{1-3}$ amido)phényle correspondant de Formules (I) ou (E); ou

F. l'hydrolyse d'un composé (alcan $C_{1-3}$ amido)phényle ou N-(alkyl $C_{1-3}$)-(alcan $C_{1-3}$ amido)-phényle de Formules (I), ou (E), tel que défini précédemment, ou d'un composé de Formule (G) représenté par la structure :

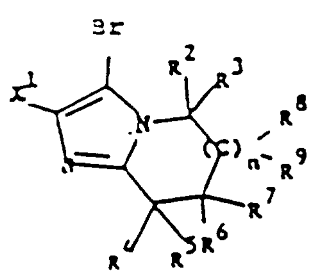

FORMULE (G)

dans lequel

- n est égal à 0 ou à 1;
- $R^2$, $R^3$, $R^4$, $R^5$, $R^6$, $R^7$, $R^8$, et $R^9$ sont des atomes d'hydrogène; ou un ou deux de $R^2$, $R^3$, $R^4$, $R^5$, $R^6$, $R^7$, $R^8$, et $R^9$ sont choisis indépendamment parmi l'hydrogène ou les groupes alkyle $C_{1-2}$;
- $X^1$ est un groupe (alcan $C_{1-3}$ amido)phényle, ou N-(alkyl $C_{1-3}$)-(alcan $C_{1-3}$ amido)phényle;

pour donner le composé aminophényle ou (alkyl $C_{1-3}$ amino)phényle correspondant, de Formules (I), (E), ou (G); ou

G. l'oxydation d'un composé de Formule (I), lorsque l'un des groupes $R^1$ et R est un groupe phényle substitué par un ou plusieurs groupes alkyl $C_{1-3}$ thio, alkyl $C_{1-3}$ sulfinyle, alkényl $C_{1-3}$ thio, ou acyloxyalkylthio, avec un agent oxydant, pour donner le composé alkyl $C_{1-3}$ sulfinyle, alkyl $C_{1-3}$ sulfonyle, acyloxyalkylsulfinyle, ou alkényl $C_{1-3}$ sulfinyle correspondant, de Formule (I); ou

H. la réduction d'un composé de Formules (E), ou (I), tel que défini précédemment, dans lequel le groupe X de la Formule (E), ou un des groupes R et $R^1$ de la Formule (I), est un groupe phényle mono- ou di-substitué contenant un groupe nitro, avec l'hydrogène gazeux en présence d'un catalyseur, pour donner le composé aminophényle correspondant, de Formules (I), ou (E); ou

I. la cycloalkylation d'un composé de Formules (I), ou (E), tel que défini précédemment, dans lequel un des groupes $R^1$ et R de la Formule (I), ou le groupe X de la Formule (E), est un groupe aminophényle, avec selon les cas, le dihalobutane ou le dihalopentane, en présence d'une base, pour donner le composé N-pipéridino, ou N-pyrrolidino correspondant, de Formules (I), ou (E); ou

J. lorsque les produits à préparer sont des composés de Formules (I) ou (E), tels que définis précédemment, dans lesquels les groupes $R^1$ et R de la Formule (I), ou le groupe X de la Formule (E), est un groupe (alkyl $C_{1-3}$ amino)phényle, la réduction des composés alcan $C_{1-3}$ amido correspondants, par le borane ou le complexe borane diméthylsulfure dans le tétrahydrofurane, pour donner les composés désirés de Formules (I), ou (E); ou

K. lorsque les produits à préparer sont des composés de Formules (I) ou (E), tels que définis précédemment, dans lesquels l'un des groupes $R^1$ et R de la Formule (I), ou le groupe X de la Formule (E), est un groupe (dialkyl $C_{1-3}$ amino)phényle, la réduction des composés N-(alkyl $C_{1-3}$)-(alcan $C_{1-3}$ amido) correspondants par le borane ou le complexe borane diméthylsulfure dans le tétrahydrofurane, pour donner les composés désirés de Formules (I), ou (E); ou

L.

(i) la réaction d'un composé de Formule (E), ou (G) avec un réactif alkyl $C_{1-5}$ lithium pour donner le réactif lithium correspondant, par métallation ou inter-échange lithium-halogène, respectivement;

(ii) l'addition d'étherate d'halogénure de magnésium en excès, au réactif lithium, pour donner par transmétallation, le réactif de Grignard correspondant;

(iii) l'addition du réactif de Grignard à un sel de N-acylpyridinium, pour donner le composé correspondant de Formule (F) tel que défini plus haut; ou

(iv) la désacylation/oxydation du composé de Formule (F) pour donner le composé correspondant de Formule (I); ou

M. le traitement du dérivé 3-lithio d'un composé de Formules (E), ou (G), chacun tel que défini précédemment, avec un chlorure de trialkyl étain, pour donner un composé de Formule (J) ayant la formule suivante :

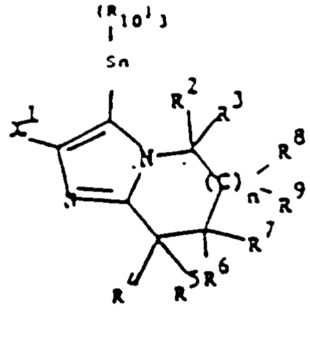

FORMULE (J)

dans lequel :

- n est égal à 0 ou à 1;
- $R^2$, $R^3$, $R^4$, $R^5$, $R^6$, $R^7$, $R^8$, et $R^9$ sont des atomes d'hydrogène, ou un ou deux de $R^2$, $R^3$, $R^4$, $R^5$, $R^6$, $R^7$, $R^8$, et $R^9$ sont choisis indépendamment parmi l'hydrogène ou les groupes alkyle $C_{1-2}$;
- $R_{10}$ est un groupe alkyle $C_{1-4}$; et
- $X^1$ est choisi parmi

(a) les groupes phényle monosubstitués, ledit substituant étant choisi parmi l'hydrogène, les groupes fluoro, chloro, alcoxy $C_{1-3}$, alkyle $C_{1-4}$, dialkyl $C_{1-3}$ amino, $CF_3$, alkyl $C_{1-3}$ amino, N-pyrrolidino, N-pipéridino, prop-2-èn-1-oxy, ou 2,2,2-trihaloéthoxy; ou

(b) les groupes pyridyle ou pyridyle alkyle-substitués;

et la réaction du composé de Formule (J) avec un mélange d'halogénure ou triflate d'aryle ou d'hétéroaryle, et de tétrakis(triphosphine)palladium dans un mélange de tétrahydrofurane et d'hexaméthylphosphoramide, pour donner un composé de Formule (I); ou

N. lorsque le produit à préparer est un composé de Formule (I) dans lequel un des groupes R ou $R^1$ est un groupe phényle substitué contenant un ou plusieurs groupes alkylthio, le traitement du composé phényle fluoro-substitué correspondant, de Formule (I), avec un sel métallique de l'alkylmercaptan, dans un solvant polaire aprotique, pour donner le composé désiré de Formule (I); ou

O. lorsque le produit à préparer est un composé de Formule (I) dans lequel un des groupes R ou $R^1$ est un groupe phényle substitué par un groupe acyloxyalkylthio dans lequel le groupe alkyle est éventuellement substitué par un groupe alkyle $C_{1-4}$, le traitement d'un composé de Formule (I) dans lequel $R^1$ est un groupe phényle substitué par au moins un groupe alkylsulfinyle, avec un anhydride d'acide alcanoïque, pour donner le composé désiré de Formule (I); ou

P. lorsque le produit à préparer est un composé de Formule (I) dans lequel un des groupes $R^1$ ou R est un groupe phényle substitué par au moins un groupe sulfhydryle, l'hydrolyse du produit issu du procédé O décrit ci-dessus, pour donner les composés désirés de Formule (I); ou

Q. lorsque le produit à préparer est un composé de Formule (I) dans lequel un des groupes $R^1$ ou R est un groupe phényle substitué par au moins un groupe acylthio, dithioacyle, thiocarbamyle, ou dithiocarbamyle, le traitement du produit issu du procédé O décrit ci-dessus, avec un halogénure d'acyle, un anhydride d'acide alcanoïque, un halogénure de thioacyle, un anhydride d'acide dithioalcanoïque, un halogénure de dialkylcarbamyle, ou un halogénure de dialkylthiocarbamyle, en présence d'une base telle que la pyridine, pour donner les composés désirés de Formule (I); ou

R. lorsque le produit à préparer est un composé de Formule (I) dans lequel le groupe $R^1$ ou le groupe R est un groupe phényle substitué par un groupe alkénylthio, l'alkylation d'un composé de Formule (I) dans lequel un des groupes $R^1$ ou R est un groupe phényle substitué par au moins un groupe sulfhydryle, avec un halogénure d'alkényle substitué de façon appropriée, tel que le bromure d'allyle, pour donner des composés de Formule (I) dans lesquels $R^1$ ou R est un groupe phényle substitué par au moins un groupe alkénylthio; ou

S. lorsque le produit à préparer est un composé de Formule (I) dans lequel le groupe $R^1$ ou le groupe R est un groupe phényle substitué par un groupe alkénylthio dans lequel le soufre est attaché à l'atome de carbone portant la double liaison,

i) le traitement du produit mercapto issu du procédé P décrit ci-dessus, avec une base forte, pour donner le composé sel de métal du mercaptide correspondant,

ii) le traitement du composé sel de métal du mercaptide avec un trialkylsilylméthylchlorure, pour donner un composé de Formule (I) dans lequel le groupe phényle a au moins un substituant trialkylsilylméthylsulfure,

iii) le traitement du composé trialkysilylméthylsulfure-substitué, dans un solvant aprotique, avec un réactif lithiant, suivi de l'aldéhyde ou cétone aliphatique approprié, pour donner un composé de Formule (I) dans lequel R ou $R^1$ est un groupe phényle substitué par au moins un groupe alkénylthio; ou

T. lorsque le produit à préparer est un composé de Formule (I) dans lequel le groupe R ou le groupe $R^1$ est un groupe pyridyle substitué par un groupe alkyle, la désacylation et l'aromatisation d'un composé de Formule (L) :

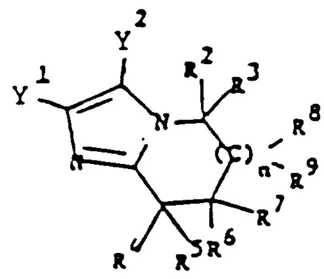

FORMULE (L)

dans lequel

- n est égal à 0 ou à 1;
- $R^2$, $R^3$, $R^4$, $R^5$, $R^6$, $R^7$, $R^8$, et $R^9$ sont tous des atomes d'hydrogène, ou un ou deux de $R^2$, $R^3$, $R^4$, $R^5$, $R^6$, $R^7$, $R^8$, et $R^9$ sont choisis indépendamment parmi l'hydrogène ou les groupes alkyle $C_{1-2}$;
- un des groupes $Y^1$ ou $Y^2$ est choisi indépendamment parmi les groupes 4-[1,2-dihydro-2-(alkyl $C_{1-4}$)]pyridyle substitués par des groupes N-(alcanoyle $C_{1-8}$), N-(alcoxy $C_{1-8}$ carbonyle), N-(benzoyle), N-(phénoxycarbonyle), N-(phénylacétyle), ou N-(benzyloxycarbonyle);
- et l'autre est choisi parmi les groupes phényle monosubstitués, ledit substituant étant choisi parmi l'hydrogène, les groupes halo, alcoxy $C_{1-3}$, alkyl $C_{1-3}$ thio, alkyle $C_{1-4}$, N-(alkyl $C_{1-3}$)-N-(alcan $C_{1-3}$ amido), dialkyl $C_{1-3}$ amino, $CF_3$, N-pyrrolidino, N-pipéridino, prop-2-èn-1-oxy, ou 2,2,2-trihaloéthoxy;

ou d'un sel de celui-ci, pharmaceutiquement acceptable, par le soufre, dans la décaline, la tétraline, le p-cymène, ou le xylène au reflux, avec l'oxygène gazeux pendant 15 minutes pour donner le composé de Formule (I) correspondant.

**2.** Procédé selon la revendication 1, dans lequel le composé de Formule (I) est le 2-(4-méthylthiophényl)-3-(4-pyridyl)-6,7-dihydro-[5H]-pyrrolo[1,2-a]imidazole, ou un sel de celui-ci, pharmaceutiquement acceptable.

**3.** Procédé selon la revendication 1, dans lequel le composé de Formule (I) est le 2-(4-méthylsulfinylphényl)-3-(4-pyridyl)-6,7-dihydro-[5H]-pyrrolo[1,2-a]imidazole, ou un sel de celui-ci, pharmaceutiquement acceptable.

**4.** Procédé selon la revendication 1, qui comprend la préparation d'un composé de Formule (I) qui est :

- le 2-(4-méthylsulfonylphényl)-3-(4-pyridyl)-6,7-dihydro-[5H]-pyrrolo-[1,2-a]imidazole;
- le 2-(4-méthoxyphényl)-3-(2,6-diméthyl-4-pyridyl)-6,7-dihydro-[5H]-pyrrolo[1,2-a]imidazole;
- le 2-(4-éthylthiophényl)-3-(4-pyridyl)-6,7-dihydro-[5H]-pyrrolo[1,2-a]-imidazole;
- le 2-(4-éthylsulfinylphényl)-3-(4-pyridyl)-6,7-dihydro-[5H]-pyrrolo-[1,2-a]imidazole;
- le 2-(4-mercaptophényl)-3-(4-pyridyl)-6,7-dihydro-[5H]-pyrrolo[1,2-a]imidazole;
- le 2-(4-triméthylacétylthiophényl)-3-(4-pyridyl)-6,7-dihydro-[5H]-pyrrolo[1,2-a]imidazole;
- le 2-(4-acétylthiophényl)-3-(4-pyridyl)-6,7-dihydro-[5H]-pyrrolo-[1,2-a]imidazole;
- le 2-(4-éthylsulfonylphényl)-3-(4-pyridyl)-6,7-dihydro-[5H]-pyrrolo-[1,2-a]imidazole,
- le 2-(4-fluorophényl)-3-[4-(2-méthyl)pyridyl]-6,7-dihydro-[5H]-pyrrolo-[1,2-a]imidazole;
- le 2-(4-méthylthiophényl)-3-[4-(2-méthyl)pyridyl]-6,7-dihydro-[5H]-pyrrolo-[1,2-a]imidazole;
- le 2-(4-méthylsulfinylphényl)-3-[4-(2-méthyl)pyridyl]-6,7-dihydro-[5H]-pyrrolo-[1,2-a]imidazole;
- le 2-(4-carbéthoxyméthylthiophényl)-3-(4-pyridyl)-6,7-dihydro-[5H]-pyrrolo[1,2-a]imidazole; ou
- le 2-(4-acétoxyméthylthiophényl)-3-(4-pyridyl)-6,7-dihydro-[5H]-pyrrolo[1,2-a]imidazole.

**5.** Procédé pour la préparation d'un composé de Formule (I) qui est le 2-(4-méthylthiophényl)-3-(4-pyridyl)-6,7-dihydro-[5H]-pyrrolo[1,2-a]imidazole, comprenant le traitement du composé phényle fluorosubstitué correspondant de Formule (I) selon la revendication 1, avec 1,2 équivalent d'un sel métallique de l'alkylmercaptan dans un solvant polaire arotique.

**6.** Procédé pour la préparation d'un composé de Formule (I) qui est le 2-(4-méthylsulfinylphényl)-3-(4-pyridyl)-6,7-dihydro-[5H]-pyrrolo-[1,2-a]imidazole, comprenant l'oxydation du composé 4-méthylthiophényl correspondant, par un agent oxydant approprié.

**7.** Procédé pour la préparation d'une composition pharmaceutique contenant un composé de Formule (I) selon l'une quelconque des revendications 1 à 4, lequel procédé comprend la mise en association du composé avec un véhicule pharmaceutiquement acceptable.

**8.** Procédé pour la préparation d'un composé de Formule (L) ayant la structure suivante : dans lequel

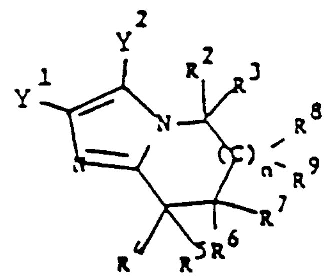

FORMULE (L)

- n est égal à 0 ou à 1;
- $R^2$, $R^3$, $R^4$, $R^5$, $R^6$, $R^7$, $R^8$, et $R^9$ sont tous des atomes d'hydrogène, ou un ou deux de $R^2$, $R^3$, $R^4$, $R^5$, $R^6$, $R^7$, $R^8$, et $R^9$ sont choisis indépendamment parmi l'hydrogène ou les groupes alkyle $C_{1-2}$;
- un des groupes $Y^1$ ou $Y^2$ est choisi indépendamment parmi les groupes 4-[1,2-dihydro-2-(alkyl $C_{1-4}$)]pyridyle substitués par des groupes N-(alcanoyle $C_{1-8}$), N-(alcoxy $C_{1-8}$ carbonyle), N-(benzoyle), N-(phénoxycarbonyle), N-(phénylacétyle), ou N-(benzyloxycarbonyle);
- et l'autre est choisi parmi les groupes phényle monosubstitués, ledit substituant étant choisi parmi l'hydrogène, les groupes halo, alcoxy $C_{1-3}$, alkyl $C_{1-3}$ thio, alkyle $C_{1-4}$, N-(alkyl $C_{1-3}$)-N-(alcan $C_{1-3}$ amido), dialkyl $C_{1-3}$ amino, $CF_3$, N-pyrrolidino, N-pipéridino, prop-2-èn-1-oxy, ou 2,2,2-trihaloéthoxy;

ou d'un sel de celui-ci, pharmaceutiquement acceptable,
qui comprend le traitement d'un composé de Formule (I) selon la revendication 1, avec un halogénure d'acyle, un halogénure d'aroyle, un ester arylalkyle haloformiate, ou un ester alkyle haloformiate, et un réactif de Grignard alkyle $C_{1-4}$.

**9.** Procédé pour la préparation d'un composé de Formule (J), ayant la structure suivante :

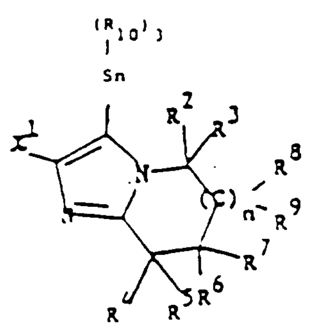

FORMULE (J)

dans lequel

- n est égal à 0 ou à 1;
- $R^2$, $R^3$, $R^4$, $R^5$, $R^6$, $R^7$, $R^8$, et $R^9$ sont des atomes d'hydrogène, ou un ou deux de $R^2$, $R^3$, $R^4$, $R^5$, $R^6$, $R^7$, $R^8$, et $R^9$ sont choisis indépendamment parmi l'hydrogène ou les groupes alkyle $C_{1-2}$;
- $R_{10}$ est un groupe alkyle $C_{1-4}$; et
- $X^1$ est choisi parmi
  (a) les groupes phényle ou phényle monosubstitués, ledit substituant étant choisi parmi l'hydrogène, les groupes fluoro, chloro, alcoxy $C_{1-3}$, alkyle $C_{1-4}$, dialkyl $C_{1-3}$ amino, $CF_3$, alkyl $C_{1-3}$ amino, N-pyrrolidino, N-pipéridino, prop-2-èn-1-oxy, ou 2,2,2-trihaloéthoxy;
  (b) les groupes pyridyle ou pyridyle alkyle-substitués;
  ou d'un sel de celui-ci, pharmaceutiquement acceptable, qui comprend la réaction d'un dérivé 3-lithio d'un composé de Formule (E) selon la revendication 1, avec un chlorure de trialkyl étain.

**Revendications pour l'Etat contractant suivant : GR**

**1.** Procédé pour la préparation d'un composé de formule :

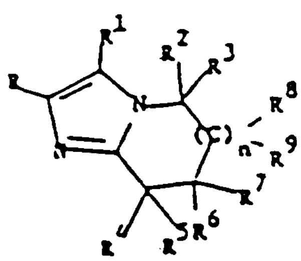

FORMULE (I)

dans lequel :

1) un des groupes R ou $R^1$ doit être un groupe pyridyle alkyle-substitué, et l'autre est choisi parmi les groupes phényle monosubstitués dans lesquels ledit substituant est choisi parmi l'hydrogène, les halogènes, les groupes hydroxy, alcoxy $C_{1-3}$, alkyl $C_{1-3}$ thio, alkyle $C_{1-4}$, alkyl $C_{1-3}$ sulfinyle, alkyl $C_{1-3}$ sulfonyle, alkyl $C_{1-3}$ amino, dialkyl $C_{1-3}$ amino, $CF_3$, N-(alkyl $C_{1-3}$)-N-(alcan $C_{1-3}$ amido), N-pyrrolidino, N-pipéridino, prop-2-èn-1-oxy, ou 2,2,2-trihaloéthoxy; ou

2) $R^1$ est un groupe 4-pyridyle et R est choisi parmi les groupes phényle monosubstitués dans lesquels ledit substituant est choisi parmi les groupes alkyl $C_{1-3}$ thio, alkyl $C_{1-3}$ sulfinyle, alkyl $C_{1-3}$ sulfonyle, ou un groupe alkényl $C_{2-5}$ thio ou alkényl $C_{2-5}$ sulfinyle ramifié ou non ramifié; ou

3) un des groupes $R^1$ ou R est un groupe pyridyle ou pyridyle alkyle-substitué, et l'autre est choisi parmi les groupes phényle monosubstitués ou disubstitués dans lesquels ledit substituant est choisi parmi les groupes thiol [HS-], acylthio [AC(O)S-], dithioacyle [AC(S)S-], dialkylthiocarbamyle [$A_2$NC-(O)S-], dialkyldithiocarbamyle [$A_2$NC(S)S-], alkylcarbonylalkylthio [AC(O)$CH_2$S-], carbalcoxyalkylthio [AOC(O)$CH_2$S-], ou acyloxyalkylthio [AC(O)O$CH_2$S-], dans lesquels le groupe $CH_2$ est éventuellement substitué par un groupe alkyle $C_{1-4}$, et A est un groupe alkyle $C_{1-9}$;

et $R^2$, $R^3$, $R^4$, $R^5$, $R^6$, $R^7$, $R^8$, et $R^9$ sont un atome d'hydrogène, ou un ou deux parmi $R^2$, $R^3$, $R^4$, $R^5$, $R^6$, $R^7$, $R^8$, et $R^9$ sont choisis indépendamment entre un atome d'hydrogène et un groupe alkyle $C_{1-2}$; et n est égal à 0 ou à 1;

ou d'un sel de celui-ci, pharmaceutiquement acceptable, qui comprend :

A. lorsque le produit à préparer est un composé de Formule (I) dans lequel $R^1$ est un groupe 4-pyridyle, et R est un groupe autre que pyridyle, la réaction d'un composé de Formule (E) représenté par la structure :

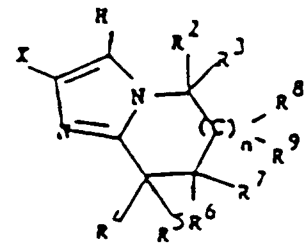

FORMULE (E)

dans lequel

- n est égal à 0 ou à 1;
- $R^2$, $R^3$, $R^4$, $R^5$, $R^6$, $R^7$, $R^8$, et $R^9$ sont des atomes d'hydrogène, ou un ou deux de $R^2$, $R^3$, $R^4$, $R^5$, $R^6$, $R^7$, $R^8$, et $R^9$ sont choisis indépendamment parmi l'hydrogène ou les groupes alkyle $C_{1-2}$;
- X est un groupe alkyl $C_{1-3}$ thiophényle;

dans la pyridine, avec un halogénure d'aroyle, un ester arylalkyle haloformiate, ou un ester alkyle haloformiate, ou avec le sel acyl pyridinium préformé, pour donner un composé de Formule (F) représenté par la structure :

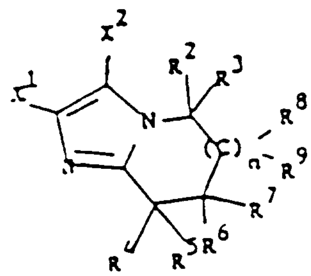

FORMULE (F)

dans lequel

- n est égal à 0 ou à 1;
- $R^2$, $R^3$, $R^4$, $R^5$, $R^6$, $R^7$, $R^8$, et $R^9$ sont des atomes d'hydrogène, ou un ou deux de $R^2$, $R^3$, $R^4$, $R^5$, $R^6$, $R^7$, $R^8$, et $R^9$ sont choisis indépendamment parmi l'hydrogène ou les groupes alkyle $C_{1-2}$;
- $X^1$ est un groupe phényle monosubstitué par un groupe alkyl $C_{1-3}$ thio; et
- $X^2$ est un groupe 4-(1,4-dihydro)pyridyle substitué par un groupe N-(alcanoyle $C_{1-8}$), N-(alcoxy $C_{1-8}$ carbonyle), N-(benzoyle), N-(phénoxycarbonyle), N-(phénylacétyle), ou N-(benzyloxycarbonyle); suivie d'une désacylation/oxydation d'un composé de Formule (F) pour donner un composé de Formule (I); ou

B. lorsque le produit à préparer est un composé hydroxy de Formules (I), ou (E), la déméthylation d'un composé méthoxyphényle de Formule (E) ou (I), dans lequel la Formule (E) est telle que définie ci-dessus. excepté que X est un groupe méthoxyphényle, et la Formule (I) est représentée par la structure :

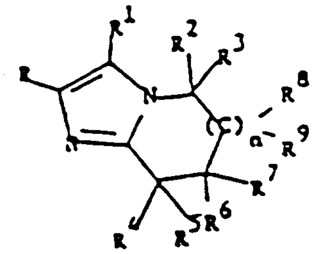

FORMULE (I)

dans lequel

- n est égal à 0 ou à 1;
- $R^2$, $R^3$, $R^4$, $R^5$, $R^6$, $R^7$, $R^8$, et $R^9$ sont des atomes d'hydrogène, ou un ou deux de $R^2$, $R^3$, $R^4$, $R^5$, $R^6$, $R^7$, $R^8$, et $R^9$ sont choisis indépendamment parmi l'hydrogène ou les groupes alkyle $C_{1-2}$;
- un des groupes $R^1$ et R est un groupe pyridyle alkyle-substitué, et l'autre est un groupe méthoxyphényle;

ou d'un sel de celui-ci, pharmaceutiquement acceptable;

avec HBr dans l'acide acétique, ou avec $BBr_3$ dans le dichlorométhane, pour donner le composé hydroxy correspondant, de Formules (I), ou (E); ou

C. l'O-alkylation du composé hydroxy de Formule (I), ou (E), tel que défini plus haut, lorsque l'un des groupes $R^1$ ou R de (I), et le groupe X de (E) est un groupe phényle substitué par au moins un groupe hydroxy, pour donner le composé correspondant, de Formules (I), ou (E) substitué par les groupes alcoxy $C_{1-3}$ phényle, 2,2,2-trihaloéthoxyphényle, ou prop-2-èn-1-oxyphényle; ou

D. l'acylation d'un composé aminophényle ou (alkyl $C_{1-3}$ amino)phényle de Formule (I) avec l'halogénure ou l'anhydride d'acyle substitué de façon correspondante, dans la pyridine, pour donner le composé N-(alkyl $C_{1-3}$ )-(alcan $C_{1-3}$ amido)phényle de Formule (I); ou

E. l'alkylation d'un composé (alcan $C_{1-3}$ amido)phényle de Formules (I), ou (E), tel que défini précédemment, avec un agent alkylant en présence d'un base, pour donner le composé N-(alcan $C_{1-3}$ amido)phényle ou N-(alkyl $C_{1-3}$)-(alcan $C_{1-3}$ amido)phényle correspondant de Formules (I) ou (E); ou

F. l'hydrolyse d'un composé (alcan $C_{1-3}$ amido)phényle ou N-(alkyl $C_{1-3}$)-(alcan $C_{1-3}$ amido)-phényle de Formules (I), ou (E), tel que défini précédemment, ou d'un composé de Formule (G) représenté par la structure :

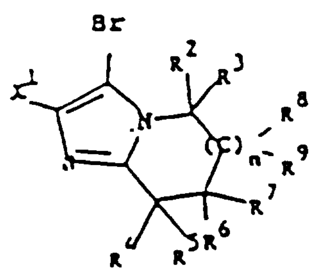

FORMULE (G)

dans lequel

- n est égal à 0 ou à 1;
- $R^2$, $R^3$, $R^4$, $R^5$, $R^6$, $R^7$, $R^8$, et $R^9$ sont des atomes d'hydrogène; ou un ou deux de $R^2$, $R^3$, $R^4$, $R^5$, $R^6$, $R^7$, $R^8$, et $R^9$ sont choisis indépendamment parmi l'hydrogène ou les groupes alkyle $C_{1-2}$;

- $X^1$ est un groupe (alcan $C_{1-3}$ amido)phényle, ou N-(alkyl $C_{1-3}$)-(alcan $C_{1-3}$ amido)phényle;

pour donner le composé aminophényle ou (alkyl $C_{1-3}$ amino)phényle correspondant, de Formules (I), (E), ou (G); ou

G. l'oxydation d'un composé de Formule (I), lorsque l'un des groupes $R^1$ et R est un groupe phényle substitué par un ou plusieurs groupes alkyl $C_{1-3}$ thio, alkyl $C_{1-3}$ sulfinyle, alkényl $C_{1-3}$ thio, ou acyloxyalkylthio, avec un agent oxydant, pour donner le composé alkyl $C_{1-3}$ sulfinyle, alkyl $C_{1-3}$ sulfonyle, acyloxyalkylsulfinyle, ou alkényl $C_{1-3}$ sulfinyle correspondant, de Formule (I); ou

H. la réduction d'un composé de Formules (E), ou (I), tel que défini précédemment, dans lequel le groupe X de la Formule (E), ou un des groupes R et $R^1$ de la Formule (I), est un groupe phényle mono- ou di-substitué contenant un groupe nitro, avec l'hydrogène gazeux en présence d'un catalyseur, pour donner le composé aminophényle correspondant, de Formules (I), ou (E); ou

I. la cycloalkylation d'un composé de Formules (I), ou (E), tel que défini précédemment, dans lequel un des groupes $R^1$ et R de la Formule (I), ou le groupe X de la Formule (E), est un groupe aminophényle, avec selon les cas, le dihalobutane ou le dihalopentane, en présence d'une base, pour donner le composé N-pipéridino, ou N-pyrrolidino correspondant, de Formules (I), ou (E); ou

J. lorsque les produits à préparer sont des composés de Formules (I) ou (E), tels que définis précédemment, dans lesquels les groupes $R^1$ et R de la Formule (I), ou le groupe X de la Formule (E), est un groupe (alkyl $C_{1-3}$ amino)phényle, la réduction des composés alcan $C_{1-3}$ amido correspondants, par le borane ou le complexe borane diméthylsulfure dans le tétrahydrofurane, pour donner les composés désirés de Formules (I), ou (E); ou

K. lorsque les produits à préparer sont des composés de Formules (I) ou (E), tels que définis précédemment, dans lesquels l'un des groupes $R^1$ et R de la Formule (I), ou le groupe X de la Formule (E), est un groupe (dialkyl $C_{1-3}$ amino)phényle, la réduction des composés N-(alkyl $C_{1-3}$)-(alcan $C_{1-3}$ amido) correspondants par le borane ou le complexe borane diméthylsulfure dans le tétrahydrofurane, pour donner les composés désirés de Formules (I), ou (E); ou

L.

(i) la réaction d'un composé de Formule (E), ou (G) avec un réactif alkyl $C_{1-5}$ lithium pour donner le réactif lithium correspondant, par métallation ou inter-échange lithium-halogène, respectivement;

(ii) l'addition d'étherate d'halogénure de magnésium en exces, au réactif lithium, pour donner par transmétallation, le réactif de Grignard correspondant;

(iii) l'addition du réactif de Grignard à un sel de N-acylpyridinium, pour donner le composé correspondant de Formule (F) tel que défini plus haut: ou

(iv) la désacylation/oxydation du composé de Formule (F) pour donner le composé correspondant de Formule (I); ou

M. le traitement du dérivé 3-lithio d'un composé de Formules (E), ou (G), chacun tel que défini précédemment, avec un chlorure de trialkyl étain, pour donner un composé de Formule (J) ayant la formule suivante :

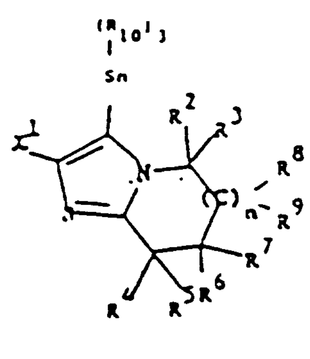

FORMULE (J)

dans lequel :

- n est égal à 0 ou à 1;
- $R^2$, $R^3$, $R^4$, $R^5$, $R^6$, $R^7$, $R^8$, et $R^9$ sont des atomes d'hydrogène, ou un ou deux de $R^2$, $R^3$, $R^4$, $R^5$, $R^6$, $R^7$, $R^8$, et $R^9$ sont choisis indépendamment parmi l'hydrogène ou les groupes alkyle

$C_{1-2}$;

- $R_{10}$ est un groupe alkyle $C_{1-4}$; et
- $X^1$ est choisi parmi

(a) les groupes phényle monosubstitués, ledit substituant étant choisi parmi l'hydrogène, les groupes fluoro, chloro, alcoxy $C_{1-3}$, alkyle $C_{1-4}$, dialkyl $C_{1-3}$ amino, $CF_3$, alkyl $C_{1-3}$ amino, N-pyrrolidino, N-pipéridino, prop-2-èn-1-oxy, ou 2,2,2-trihaloéthoxy; ou

(b) les groupes pyridyle ou pyridyle alkyle-substitués;

et la réaction du composé de Formule (J) avec un mélange d'halogénure ou triflate d'aryle ou d'hétéroaryle, et de tétrakis(triphosphine)palladium dans un mélange de tétrahydrofurane et d'hexaméthylphosphoramide, pour donner un composé de Formule (I); ou

N. lorsque le produit à préparer est un composé de Formule (I) dans lequel un des groupes R ou $R^1$ est un groupe phényle substitué contenant un ou plusieurs groupes alkylthio, le traitement du composé phényle fluoro-substitué correspondant, de Formule (I), avec un sel métallique de l'alkylmercaptan, dans un solvant polaire aprotique, pour donner le composé désiré de Formule (I); ou

O. lorsque le produit à préparer est un composé de Formule (I) dans lequel un des groupes R ou $R^1$ est un groupe phényle substitué par un groupe acyloxyalkylthio dans lequel le groupe alkyle est éventuellement substitué par un groupe alkyle $C_{1-4}$, le traitement d'un composé de Formule (I) dans lequel $R^1$ est un groupe phényle substitué par au moins un groupe alkylsulfinyle, avec un anhydride d'acide alcanoïque, pour donner le composé désiré de Formule (I); ou

P. lorsque le produit à préparer est un composé de Formule (I) dans lequel un des groupes $R^1$ ou R est un groupe phényle substitué par au moins un groupe sulfhydryle, l'hydrolyse du produit issu du procédé O décrit ci-dessus, pour donner les composés désirés de Formule (I); ou

Q. lorsque le produit à préparer est un composé de Formule (I) dans lequel un des groupes $R^1$ ou R est un groupe phényle substitué par au moins un groupe acylthio, dithioacyle, thiocarbamyle, ou dithiocarbamyle, le traitement du produit issu du procédé O décrit ci-dessus, avec un halogénure d'acyle, un anhydride d'acide alcanoïque, un halogénure de thioacyle, un anhydride d'acide dithioalconoïque, un halogénure de dialkylcarbamyle, ou un halogénure de dialkylthiocarbamyle, en présence d'une base telle que la pyridine, pour donner les composés désirés de Formule (I); ou

R. lorsque le produit à préparer est un composé de Formule (I) dans lequel le groupe $R^1$ ou le groupe R est un groupe phényle substitué par un groupe alkénylthio, l'alkylation d'un composé de Formule (I) dans lequel un des groupes $R^1$ ou R est un groupe phényle substitué par au moins un groupe sulfhydryle, avec un halogénure d'alkényle substitué de façon appropriée, tel que le bromure d'allyle, pour donner des composés de Formule (I) dans lesquels $R^1$ ou R est un groupe phényle substitué par au moins un groupe alkénylthio; ou

S. lorsque le produit à préparer est un composé de Formule (I) dans lequel le groupe $R^1$ ou le groupe R est un groupe phényle substitué par un groupe alkénylthio dans lequel le soufre est attaché à l'atome de carbone portant la double liaison,

i) le traitement du produit mercapto issu du procédé P décrit ci-dessus. avec une base forte, pour donner le composé sel de métal du mercaptide correspondant,

ii) le traitement du composé sel de métal du mercaptide avec un trialkylsilylméthylchlorure, pour donner un composé de Formule (I) dans lequel le groupe phényle a au moins un substituant trialkylsilylméthylsulfure,

iii) le traitement du composé trialkylsilylméthylsulfure-substitué, dans un solvant aprotique, avec un réactif lithiant, suivi de l'aldéhyde ou cétone aliphatique approprié, pour donner un composé de Formule (I) dans lequel R ou $R^1$ est un groupe phényle substitué par au moins un groupe alkénylthio; ou

T. lorsque le produit à préparer est un composé de Formule (I) dans lequel le groupe R ou le groupe $R^1$ est un groupe pyridyle substitué par un groupe alkyle, la désacylation et l'aromatisation d'un composé de Formule (L) :

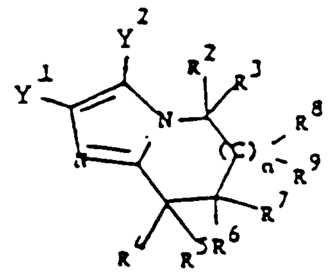

FORMULE (L)

dans lequel

- n est égal à 0 ou à 1;
- $R^2$, $R^3$, $R^4$, $R^5$, $R^6$, $R^7$, $R^8$, et $R^9$ sont tous des atomes d'hydrogène, ou un ou deux de $R^2$, $R^3$, $R^4$, $R^5$, $R^6$, $R^7$, $R^8$, et $R^9$ sont choisis indépendamment parmi l'hydrogène ou les groupes alkyle $C_{1-2}$;
- un des groupes $Y^1$ ou $Y^2$ est choisi indépendamment parmi les groupes 4-[1,2-dihydro-2-(alkyl $C_{1-4}$)]pyridyle substitués par des groupes N-(alcanoyle $C_{1-8}$), N-(alcoxy $C_{1-8}$ carbonyle), N-(benzoyle), N-(phénoxycarbonyle), N-(phénylacétyle), ou N-(benzyloxycarbonyle);
- et l'autre est choisi parmi les groupes phényle monosubstitués, ledit substituant étant choisi parmi l'hydrogène, les groupes halo, alcoxy $C_{1-3}$, $_3$, alkyl $C_{1-3}$ thio, alkyle $C_{1-4}$, N-(alkyl $C_{1-3}$)-N-(alcan $C_{1-3}$ amido), dialkyl $C_{1-3}$ amino, $CF_3$, N-pyrrolidino, N-pipéridino, prop-2-èn-1-oxy, ou 2,2,2-trihaloéthoxy;

  ou d'un sel de celui-ci, pharmaceutiquement acceptable, par le soufre, dans la décaline, la tétraline, le p-cymène, ou le xylène au reflux, avec l'oxygène gazeux pendant 15 minutes, pour donner le composé de Formule (I) correspondant.

**2.** Procédé selon la revendication 1, dans lequel le composé de Formule (I) est le 2-(4-méthylthiophényl)-3-(4-pyridyl)-6,7-dihydro-[5H]-pyrrolo[1,2-a]imidazole, ou un sel de celui-ci, pharmaceutiquement acceptable.

**3.** Procédé selon la revendication 1, dans lequel le composé de Formule (I) est le 2-(4-méthylsulfinylphényl)-3-(4-pyridyl)-6,7-dihydro-[5H]-pyrrolo[1,2-a]imidazole, ou un sel de celui-ci, pharmaceutiquement acceptable.

**4.** Procédé selon la revendication 1, qui comprend la préparation d'un composé de Formule (I) qui est :

- le 2-(4-méthylsulfonylphényl)-3-(4-pyridyl)-6,7-dihydro-[5H]-pyrrolo-[1,2-a]imidazole;
- le 2-(4-méthoxyphényl)-3-(2,6-diméthyl-4-pyridyl)-6,7-dihydro-[5H]-pyrrolo[1,2-a]imidazole;
- le 2-(4-éthylthiophényl)-3-(4-pyridyl)-6,7-dihydro-[5H]-pyrrolo[1,2-a]imidazole;
- le 2-(4-éthylsulfinylphényl)-3-(4-pyridyl)-6,7-dihydro-[5H]-pyrrolo-[1,2-a]imidazole;
- le 2-(4-mercaptophényl)-3-(4-pyridyl)-6,7-dihydro-[5H]-pyrrolo[1,2-a]imidazole;
- le 2-(4-triméthylacétylthiophényl)-3-(4-pyridyl)-6,7-dihydro-[5H]-pyrrolo[1,2-a]imidazole;
- le 2-(4-acétylthiophényl)-3-(4-pyridyl)-6,7-dihydro-[5H]-pyrrolo-[1,2-a]imidazole;
- le 2-(4-éthylsulfonylphényl)-3-(4-pyridyl)-6,7-dihydro-[5H]-pyrrolo-[1,2-a]imidazole,
- le 2-(4-fluorophényl)-3-[4-(2-méthyl)pyridyl]-6,7-dihydro-[5H]-pyrrolo-[1,2-a]imidazole;
- le 2-(4-méthylthiophényl)-3-[4-(2-méthyl)pyridyl]-6,7-dihydro-[5H]-pyrrolo-[1,2-a]imidazole;
- le 2-(4-méthylsulfinylphényl)-3-[4-(2-méthyl)pyridyl]-6,7-dihydro-[5H]-pyrrolo-[1,2-a]imidazole;
- le 2-(4-carbéthoxyméthylthiophényl)-3-(4-pyridyl)-6,7-dihydro-[5H]-pyrrolo[1,2-a]imidazole; ou
- le 2-(4-acétoxyméthylthiophényl)-3-(4-pyridyl)-6,7-dihydro-[5H]-pyrrolo[1,2-a]imidazole.

**5.** Procédé pour la préparation d'un composé de Formule (I) qui est le 2-(4-méthylthiophényl)-3-(4-pyridyl)-6,7-dihydro-[5H]-pyrrolo[1,2-a]imidazole, comprenant le traitement du composé phényle fluorosubstitué correspondant de Formule (I) selon la revendication 1, avec 1,2 équivalent d'un sel métallique de l'alkylmercaptan, dans un solvant polaire arotique.

**6.** Procédé pour la préparation d'un composé de Formule (I) qui est le 2-(4-méthylsulfinylphényl)-3-(4-pyridyl)-6,7-dihydro-[5H]-pyrrolo-[1,2-a]imidazole, comprenant l'oxydation du composé 4-méthylthiophényl correspondant, par un agent oxydant approprié.

**7.** Procédé pour la préparation d'une composition pharmaceutique contenant un composé de Formule (I) selon l'une quelconque des revendications 1 à 4, lequel procédé comprend la mise en association du composé avec un véhicule pharmaceutiquement acceptable.

**8.** Composé de formule :

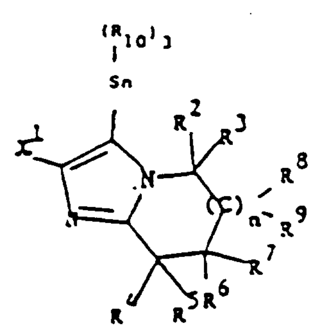

FORMULE (J)

dans lequel
- n est égal à 0 ou à 1;
- $R^2$, $R^3$, $R^4$, $R^5$, $R^6$, $R^7$, $R^8$, et $R^9$ sont des atomes d'hydrogène, ou un ou deux de $R^2$, $R^3$, $R^4$, $R^5$, $R^6$, $R^7$, $R^8$, et $R^9$ sont choisis indépendamment parmi l'hydrogène ou les groupes alkyle $C_{1-2}$;
- $R_{10}$ est un groupe alkyle $C_{1-4}$; et
- $X^1$ est choisi parmi

(a) les groupes phényle monosubstitués, ledit substituant étant choisi parmi l'hydrogène, les groupes fluoro, chloro, alcoxy $C_{1-3}$, alkyle $C_{1-4}$, dialkyl $C_{1-3}$ amino, $CF_3$, alkyl $C_{1-3}$ amino, N-pyrrolidino, N-pipéridino, prop-2-èn-1-oxy, ou 2,2,2-trihaloéthoxy;
(b) les groupes pyridyle ou pyridyle alkyle-substitués;

ou un sel de celui-ci, pharmaceutiquement acceptable.

**9.** Composé selon la revendication 8, qui est le 2-(4-méthoxyphényl)-6,7-dihydro-[5H]-pyrrolo[1,2-a]-imidazol-3-yl-tri-n-butyl étain

**10.** Composé de formule :

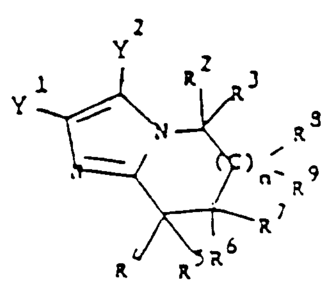

FORMULE (L)

dans lequel
- n est égal à 0 ou à 1;

- $R^2$, $R^3$, $R^4$, $R^5$, $R^6$, $R^7$, $R^8$, et $R^9$ sont tous des atomes d'hydrogène, ou un ou deux de $R^2$, $R^3$, $R^4$, $R^5$, $R^6$, $R^7$, $R^8$, et $R^9$ sont choisis indépendamment parmi l'hydrogène ou les groupes alkyle $C_{1-2}$;
- un des groupes $Y^1$ ou $Y^2$ est choisi indépendamment parmi les groupes 4-[1,2-dihydro-2-(alkyl $C_{1-4}$)]pyridyle substitués par des groupes N-(alcanoyle $C_{1-8}$), N-(alcoxy $C_{1-8}$ carbonyle), N-(benzoyle), N-(phénoxycarbonyle), N-(phénylacétyle), ou N-(benzyloxycarbonyle);
- et l'autre est choisi parmi les groupes phényle monosubstitués, ledit substituant étant choisi parmi l'hydrogène, les groupes halo, alcoxy $C_{1-3}$, alkyl $C_{1-3}$ thio, alkyle $C_{1-4}$, N-(alkyl $C_{1-3}$)-N-(alcan $C_{1-3}$ amido), dialkyl $C_{1-3}$ amino, $CF_3$, N-pyrrolidino, N-pipéridino, prop-2-èn-1-oxy, ou 2,2,2-trihaloéthoxy;

ou un sel de celui-ci, pharmaceutiquement acceptable.